# EUROPEAN PATENT SPECIFICATION

(11) **EP 2 361 976 B1**
(45) Date of publication and mention of the grant of the patent: **25.05.2016**
(21) Application number: 11000670.7
(22) Date of filing: 26.04.2006
(51) Int. Cl.: C12N 9/10, C12P 13/22, C07H 21/04, C12N 9/90, C12P 17/10

(54) **Polypeptides and biosynthetic pathways for the production of stereoisomers of monatin and their precursors**
Polypeptide und Biosynthesewege zur Herstellung von Stereoisomeren von Monatin und ihren Vorläufern
Polypeptides et voies biosynthétiques pour la production de stéréoisomères de monatine et leurs précurseurs

(30) Priority: 26.04.2005 US 674932 P
(43) Date of publication of application: 31.08.2011
(62) Divisional of application: 06751574.2
(73) Proprietor: Cargill, Incorporated, Wayzata MN 55391-5624 (US)
(72) Inventor: Brazeau, Brian, J., Minnesota 55116 (US); Burke, Ellen, San Diego California 92106 (US); De Souza, Mervyn, Plymouth Minnesota 55441 (US); Gort, Steven, J., Apply Valley Minnesota 55124 (US); Hicks, Paula, M., Eden Prairie Minnesota 55347 (US); Kollman, Sherry, R., Rogers Minnesota 55374 (US); Luginbuhl, Peter, San Diego California 92130 (US); McFarlan, Sara, C., Saint Paul Minnesota 55116 (US); Richardson, Toby, San Diego California 92131 (US); Sanchez-Riera, Fernando, A., San Carlos California 94070 (US); Solheid, Christopher, Minneapolis Minnesota 55412 (US); Weiner, David, Del Mar California 92014 (US); Zhao, Lishan, Emeryville California 94608 (US)
(74) Representative: Wibbelmann, Jobst

(56) References cited:
- WO-A1-2005/016022
- WO-A1-2005/020721
- WO-A2-03/091396
- BERGER BRADLEY J ET AL: "Methionine regeneration and aminotransferases in Bacillus subtilis, Bacillus cereus, and Bacillus anthracis", 20030401; 20030400, vol. 185, no. 8, 1 April 2003 (2003-04-01) , pages 2418-2431, XP002345665,

## Description

### BACKGROUND OF THE INVENTION

### Field of the Invention

This disclosure provides polypeptides and biosynthetic pathways that are useful in the production of certain stereoisomers of monatin, such as R,R monatin, and salts thereof.

### Background Art

Monatin is a high-intensity sweetener having the chemical formula: 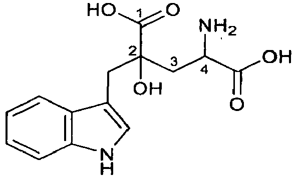 Monatin includes two chiral centers leading to four potential stereoisomeric configurations. The R,R configuration (the "R,R stereoisomer" or "R,R monatin"); the S,S configuration (the "S,S stereoisomer" or "S,S monatin"); the R,S configuration (the "R,S stereoisomer" or "R,S monatin"); and the S,R configuration (the "S,R stereoisomer" or "S,R monatin"). As used herein, unless stated otherwise, the term "monatin" is used to refer to compositions including all four stereoisomers of monatin, compositions including any combination of monatin stereoisomers, (e.g., a composition including only the R,R and S,S, stereoisomers of monatin), as well as a single isomeric form.

For purposes of this disclosure, the monatin carbon backbone will be numbered as illustrated above, with the carbon directly covalently attached to the alcohol group being identified as the 2-position carbon and the carbon directly covalently attached to the amino group being identified as the 4-position carbon. Consequently, references herein to R,R monatin, S,S monatin, R,S monatin, and S,R monatin mean: 2R,4R monatin, 2S,4S monatin, 2R,4S monatin, and 2S,4R monatin, respectively, unless otherwise indicated.

It should be noted that in the literature, the monatin carbon backbone has also been numbered using an alternative convention, with the carbon attached to the alcohol group being the 4-position carbon, and the carbon attached to the amino group being the 2-position carbon. Accordingly, for example, references to 2S,4R monatin in this disclosure would be the same as references to 2R,4S monatin in literature using the alternative numbering convention.

Furthermore, because of various naming conventions, monatin is known by a number of alternative chemical names, including: 2-hydroxy-2-(indol-3-ylmethyl)-4-aminoglutaric acid; 4-amino-2-hydroxy-2-(1H-indol-3-ylmethyl)-pentanedioic acid; 4-hydroxy-4-(3-indolylmethyl)glutamic acid; and, 3-(1-amino-1,3-dicarboxy-3-hydroxy-but-4-yl)indole.

Certain isomeric forms of monatin can be found in the bark of roots of the *Schlerochiton ilicifolius* plant located in the Transvaal Region of South Africa. U.S. Patent Application Nos. 10/422,366 ("the '366 application"), 10/979,821 ("the '821 Application"), and 11/114,922 ("the '922 application), discloses, *inter alia,* polypeptides, pathways, and microorganisms for *in vitro* and *in vivo* production of monatin.

WO 2005/020721 discloses D-aminotransferases from *Bacillus.* WO 2005/016022 describes the biosynthetic pathway of monatin as well as the use of D-aminotransferases in this pathway.

### BRIEF SUMMARY OF THE INVENTION

This disclosure provides, among other things, polypeptides and biosynthetic pathways that are useful in the production of certain stereoisomers of monatin, such as R,R monatin, and salts thereof. The methods include the use of one or more polypeptides, and in particular, enzymes, such as broad specificity D-aminotransferases (also called D-alanine aminotransferases to produce R,R monatin without having to use stoichiometric amounts of D-amino acid substrate as the amino acid donor for MP amination.

In an effort to be concise, where ever intermediates/products are identified in the specification and claims (e.g. monatin or monatin precursor) as being formed, the term "and/or salts thereof" should be understood to be included where applicable. In other words, for example, the phrase "indole-3-pyruvate is converted to MP" should be understood to read "indole-3-pyruvic acid is converted to MP and and/or salts thereof." A person of ordinary skill, in fact, would appreciate that under reaction conditions shown the salts of the intermediates/products are in fact present or also present.

According to some embodiments, the method produces a monatin composition wherein the monatin component of the composition includes only the R,R and S,R form of monatin. The term "only," when used to indicate that only certain isomers are formed, unless otherwise stated means that the pathway would produce only the identified isomers if racemization did not occur. Consequently, the term "only" should not be taken to mean absence of other isomers, but rather a person of ordinary skill would understand that other isomeric forms may be present in a relatively small amount due to racemization which may occur. According to some embodiments, the method produces a monatin composition wherein the monatin component of the composition includes only the R,R form of monatin (thus meaning except to the extent racemization occurs resulting in other isomeric forms).

As used herein, the phrase "monatin composition" means a composition including one or more isomers of monatin; the term can also mean a composition including only a single isomeric form of monatin and nothing else, depending on the context.

A process for producing a monatin composition is provided, which includes producing indole-3-pyruvate from L-tryptophan, producing 2-hydroxy 2-(indol-3ylmethyl)-4-keto glutaric acid ("monatin precursor" or "MP") from indole-3-pyruvate, and producing monatin from MP. The reaction of L-tryptophan to produce indole-3-pyruvate is facilitated by an enzyme having greater specificity, greater activity, or both for L-tryptophan as a substrate than for R-MP, R,R monatin, or both. The reaction of indole-3-pyruvate may be facilitated by an enzyme having R-specific aldolase activity and consequently produces R-MP. A racemase enzyme is provided that can facilitate epimerization of the amino acid that is formed as a byproduct of the L-tryptophan transamination reaction (or that is formed from another amino acid that is a byproduct of the tryptophan reaction) from one isomeric form to another isomeric form.

A process for producing a monatin composition is provided, which includes producing indole-3-pyruvate from L-tryptophan, producing 2-hydroxy 2-(indol-3ylmethyl)-4-keto glutaric acid ("monatin precursor" or "MP") from indole-3-pyruvate, and producing monatin from MP. The reaction of L-tryptophan to produce indole-3-pyruvate is facilitated by an enzyme having greater specificity, greater activity, or both for L-tryptophan as a substrate than for R-MP, R,R monatin, or both, and the reaction of MP to form monatin is facilitated by an enzyme, which is stereoselective for R-MP.

It should be noted that, where references are made to a series of reactions such as in the preceding paragraphs, the invention does not require each step to be explicitly performed; it is sufficient that the steps may be implicitly performed. In other words, for example, the process for producing a monatin composition, which includes producing indole-3-pyruvate from L-tryptophan, producing 2-hydroxy 2-(indol-3ylmethyl)-4-keto glutaric acid ("monatin precursor" or "MP") from indole-3-pyruvate, and producing monatin from MP, wherein each reaction is facilitated by an appropriate enzyme, can be performed by combining L-tryptophan with the enzymes and setting conditions so that the enumerated reactions could occur. In such an instance L-tryptophan could react to produce indole-3-pyruvate, the indole-3-pyruvate produced from the L-tryptophan reaction could react to form MP, and the MP produced from the indole-3-pyruvate reaction could react to form monatin. The process could also be performed, by way of example, by providing a compound that can produce L-tryptophan, under conditions suitable for L-tryptophan production to occur and combining that compound with enzymes capable of facilitating the series of reactions set forth under conditions which would be suitable for those reactions to occur. As yet another example, the process could be performed by providing a microorganism genetically engineered to produce monatin according to the described pathway, and providing appropriate conditions for the fermentation process to occur. For example, a microorganism, which naturally produces large amounts of L-tryptophan (or D-tryptophan) could be genetically engineered to produce or over-produce one or more of the enzymes used to facilitate reactions in the pathway to monatin, and appropriate conditions could be provided so that the microorganism would thereby produce monatin.

A process for producing monatin is provided, in which an α-keto acid substrate forms an L-amino acid when L-tryptophan is converted to indole-3-pyruvate, indole-3-pyruvate reacts to form MP (which can include both R-MP and S-MP though preferably includes only or predominately R-MP), and the L-amino acid reacts to regenerate (also referred to as "recycle") the α-keto acid substrate when R-MP is converted to R,R monatin. The reaction of R-MP to form R,R monatin is facilitated by a stereoinverting aminotransferase such as D-methionine aminotransferase (EC 2.6.1.41) or an enzyme derived from a D-phenylglycine aminotransferase.

A process for producing a monatin composition is provided, which includes producing D-tryptophan from L-tryptophan, producing indole-3-pyruvate from D-tryptophan, producing R-MP from indole-3-pyruvate, and producing R,R monatin from R-MP. The production of the D-tryptophan from the L-tryptophan is facilitated by a tryptophan racemase and functional equivalents thereof. In certain further embodiments, the reactions of D-tryptophan to form indole-3-pyruvate and of MP to form monatin are facilitated by the same enzyme. In yet other further embodiments, the reaction of indole-3-pyruvate is facilitated by an enzyme having R-specific aldolase activity and consequently R-MP is formed, and the reactions of D-tryptophan to form indole-3-pyruvate and of R-MP to form R,R monatin are facilitated by the same enzyme.

A method for producing R,R-monatin, or a salt thereof, comprising, or consisting essentially of, (a) producing D-tryptophan from L-tryptophan utilizing a tryptophan racemase (the racemase should have limited or no activity on monatin), (b) producing indole-3-pyruvate from D-tryptophan, (c) producing R-monatin precursor from indole-3-pyruvate, and (d) producing R,R-monatin from R-monatin precursor is disclosed herein.

More specifically, the present invention provides a method for producing R,R monatin, or a salt thereof, comprising reacting monatin precursor and one or more D-aminotransferases chosen from a *Bacillus halodurans* D-aminotransferase comprising the amino acid sequence listed in accession number NP_243677, a hybrid D-aminotransferase comprising the amino acid sequence of SEQ ID NO: 99, a D-aminotransferase of ATCC 4978 comprising the amino acid sequence of SEQ ID NO: 86, a D-aminotransferase of ATCC 7063 comprising the amino acid sequence of SEQ ID NO: 87, and homologs thereof with at least 90% sequence homology thereto. Likewise, the present invention also provides the use of one or more D-aminotransferases chosen from a *Bacillus halodurans* D-aminotransferase comprising the amino acid sequence listed in accession number NP_243677, a hybrid D-aminotransferase comprising the amino acid sequence of SEQ ID NO: 99, a D-aminotransferase of ATCC 4978 comprising the amino acid sequence of SEQ ID NO: 86, a D-aminotransferase of ATCC 7063 comprising the amino acid sequence of SEQ ID NO: 87, and homologs thereof with at least 90% sequence homology thereto, for producing R,R monatin, or a salt thereof. In some embodiments, said method or use further comprises reacting D-tryptophan and said one or more D-aminotransferases, in particular wherein at least about 75% of monatin produced is R,R monatin. In a particular embodiment of the use, said one or more D-aminotransferases are reacted with monatin precursor and/or D-tryptophan. In one embodiment, the D-aminotransferase is a *Bacillus halodurans* D-aminotransferase comprising the amino acid sequence listed in accession number NP_243677 or an amino acid sequence with at least 90% sequence homology thereto. In another embodiment, the D-aminotransferase is a hybrid D-aminotransferase comprising the amino acid sequence of SEQ ID NO: 99 or an amino acid sequence with at least 90% sequence homology thereto. In still another embodiment, the D-aminotransferase is a D-aminotransferase of ATCC 4978 comprising the amino acid sequence of SEQ ID NO.86 or an amino acid sequence with at least 90% sequence homology thereto. In still another embodiment, the D-aminotransferase is a D-aminotransferase of ATCC 7063 comprising the amino acid sequence of SEQ ID NO.87 or an amino acid sequence with at least 90% sequence homology thereto.

Finally, the present invention also provides a hybrid D-aminotransferase comprising SEQ ID NO:99, or a homolog thereof with at least 90% sequence homology thereto; a D-aminotransferase of ATCC 4978 comprising SEQ ID NO.86, or a homolog thereof with at least 90% sequence homology thereto; and a D-aminotransferase of ATCC 7063 comprising SEQ ID NO:87, or a homolog thereof with at least 90% sequence homology thereto.

While multiple embodiments are disclosed, still other embodiments of the present invention may become apparent to those skilled in the art from the specification. Accordingly, the drawings and detailed description are to be regarded as illustrative in nature and not restrictive.

### BRIEF DESCRIPTION OF THE FIGURES

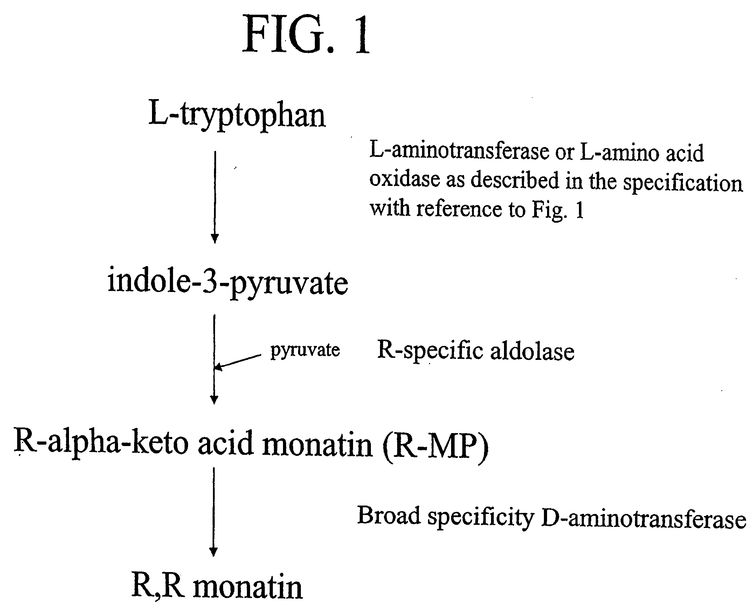

**FIG. 1** is a flow chart that shows an example of an enzymatic process for producing R,R monatin from L-tryptophan in accordance with the invention. In this example, the process includes using an L-aminotransferase (examples of which include an L-tryptophan aminotransferase, an L-aromatic aminotransferase, an L-aspartate aminotransferase, and an L-alanine aminotransferase) in the reaction of L-tryptophan that has greater specificity and/or selectivity for L-tryptophan as a substrate than for R-MP and/or an L-amino acid oxidase with limited activity and/or specificity for R,R monatin as a substrate.

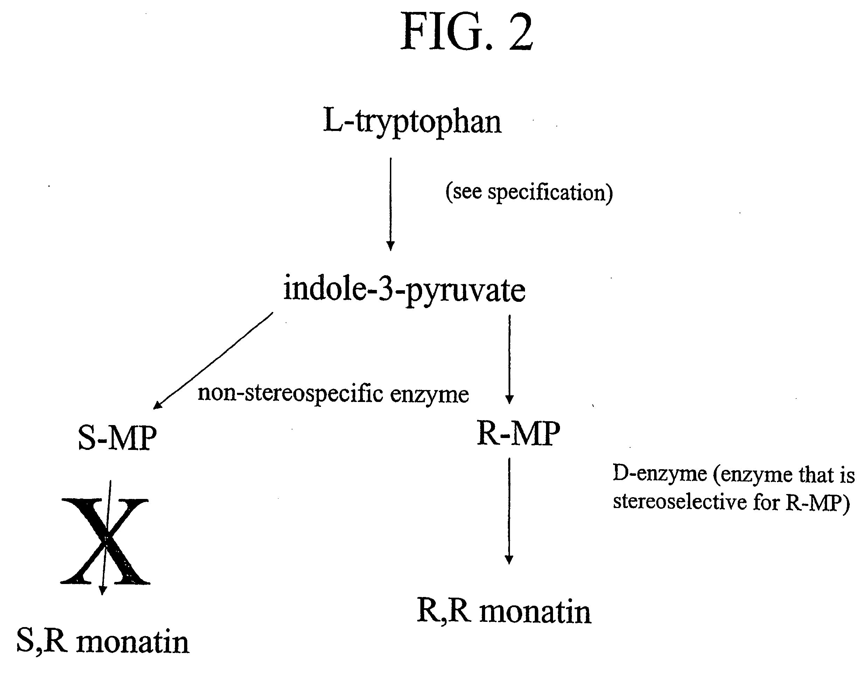

**FIG. 2** is a flow chart that shows an example of another process for producing R,R monatin in accordance with the invention. In this example, the process includes using an enzyme to convert R-MP to monatin which is stereoselective for R-MP.

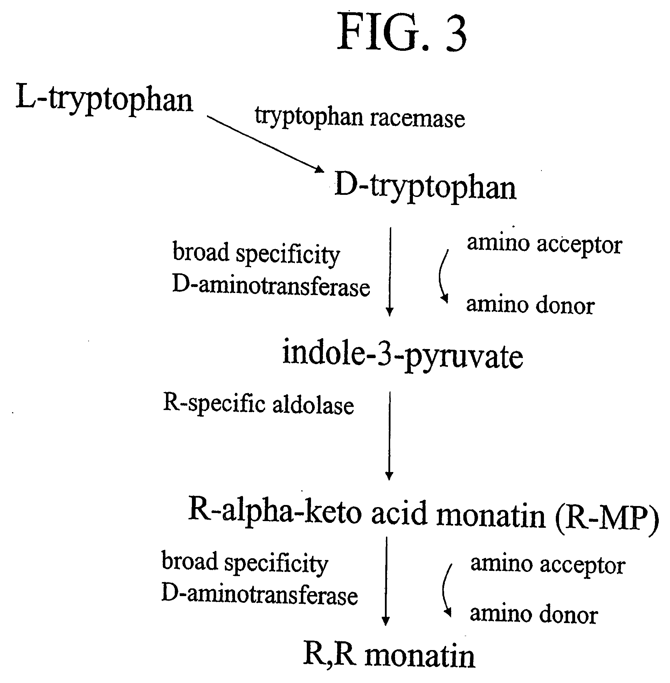

**FIG. 3** is a flow chart that shows an example of yet another process for producing R,R monatin from L-tryptophan. In this example, the process includes converting L-tryptophan to D-tryptophan using a tryptophan racemase and using a D-amino acid product in the reaction coupled to the reaction forming indole-3-pyruvate as a substrate in the reaction coupled to the reaction forming R,R monatin.

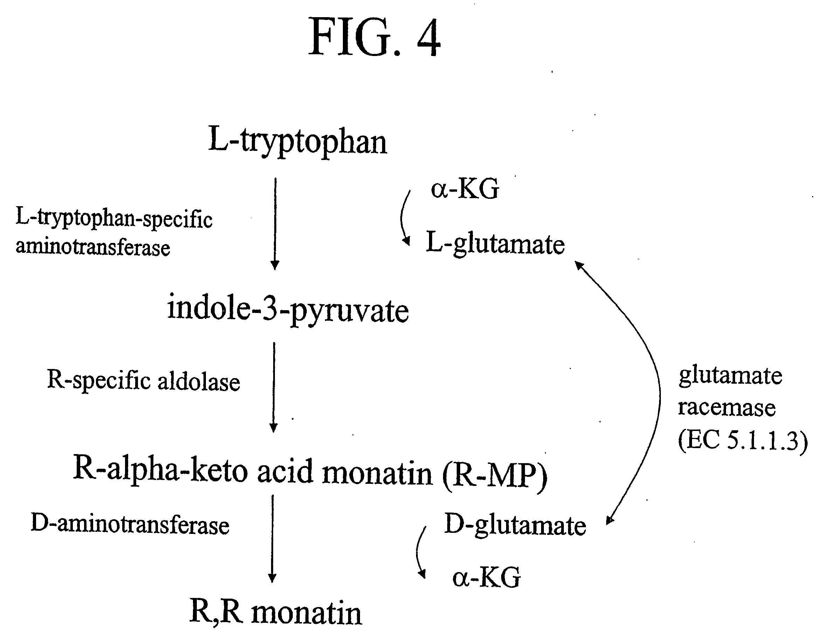

**FIG. 4** is a flow chart that shows an example of yet another process for producing R,R monatin from L-tryptophan. In this example, the process includes converting the L-amino acid formed in the reaction coupled with the L-tryptophan reaction to a D-amino acid; this D-amino acid acts as an amino donor for the reaction in which R-MP is converted to R,R monatin.

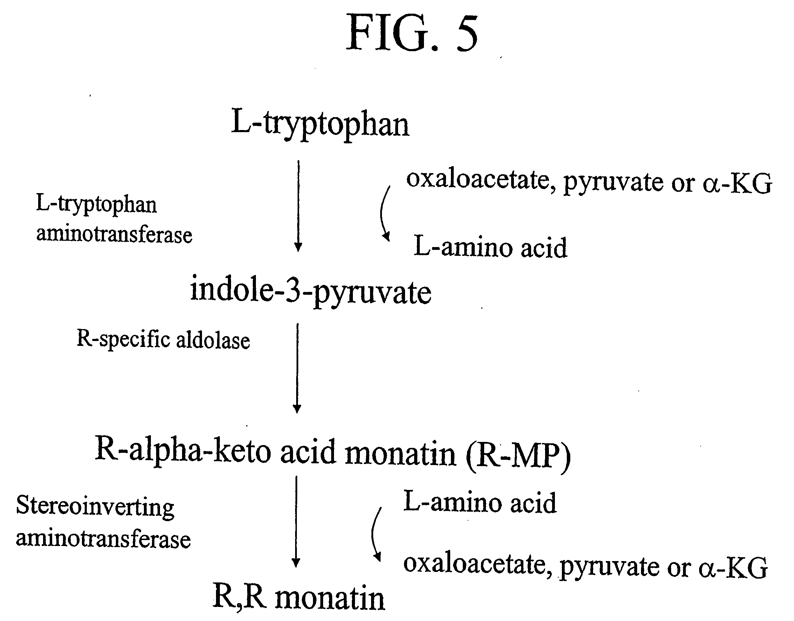

**FIG. 5** is a flow chart that shows an example of yet another process for producing R,R monatin from L-tryptophan. In this example, the process includes enzymatically facilitating the conversion of R-MP to R,R monatin using a stereoinverting enzyme so that the L-amino acid formed by the reaction coupled to the L-tryptophan reaction can be used as a substrate for the reaction coupled to the R-MP to R,R monatin reaction.

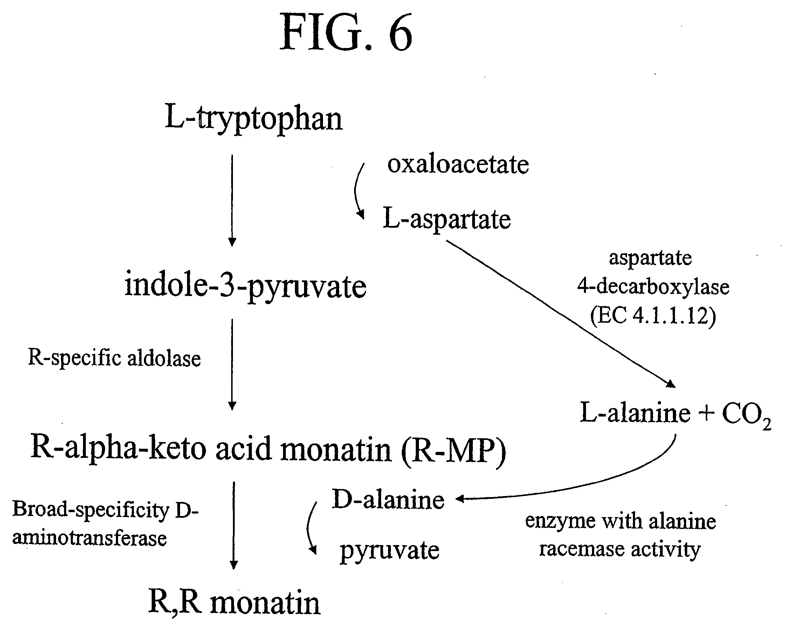

**FIG. 6** is a flow chart that shows an example of yet another process for producing R,R monatin. In this example, the process includes recycling the L-amino acid produced in the reaction forming indole-3-pyruvate with the D-amino acid used as a reactant with R-MP in the reaction forming R,R monatin through a series of conversion reactions.

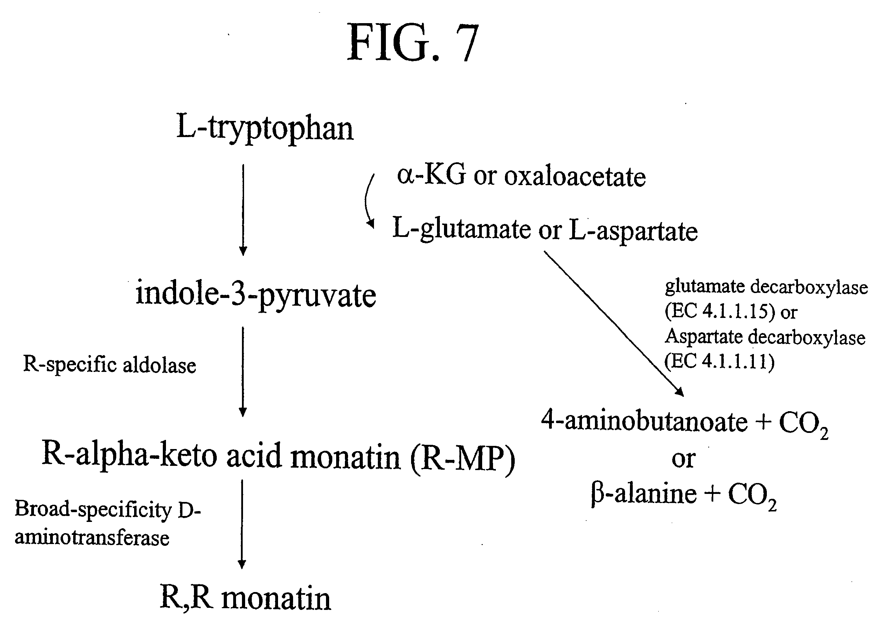

**FIG. 7** is a flow chart that shows an example of yet another process for producing R,R monatin. In this example, the process includes pushing the L-tryptophan reaction forward (i.e., driving the reaction toward the production of indole-3-pyruvate) by converting the L-amino acid byproduct of that reaction into another product. In this example, the L-amino acid L-aspartate byproduct is converted into L-alanine in an irreversible reaction using a decarboxylase.

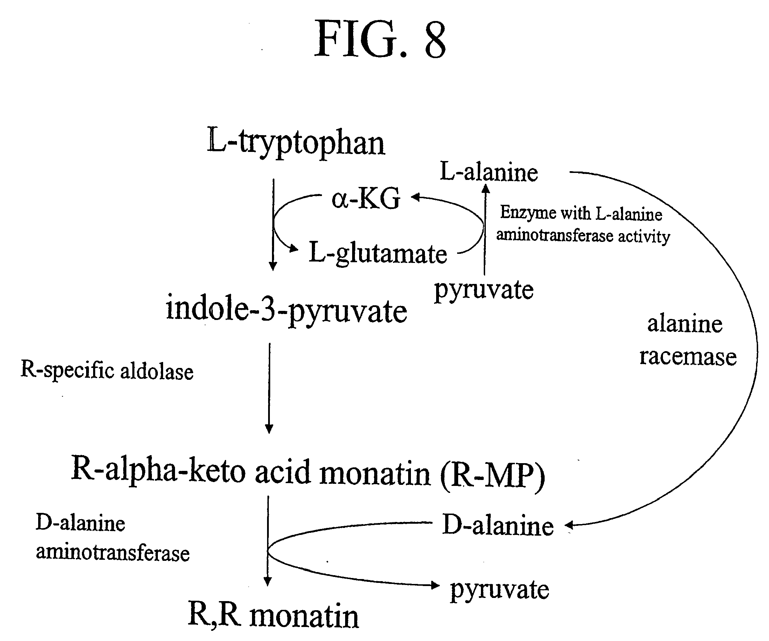

**FIG. 8** is a flow chart that shows an example of yet another process for producing R,R monatin. In this example, the process includes recycling the amino acid byproduct of the L-tryptophan reaction with the amino acid reactant of the R-MP reaction through a series of conversion reactions.
**FIG. 9(A and B)** show the amino acid sequence alignment of various published *Bacillus* D-amino acid aminotransferases ("DAATs"). Underlined amino acids indicate the regions of homology. Five PCR primers were designed based on the conserved regions. The PCR primers are as follows: 5' GAAGACCGTGGTTATCAATTT 3' (SEQ ID NO:65) (forward primer, F1 as indicated in FIG. 9A), 5' GATGGTATTTACGAAGTAATC 3' (SEQ ID NO:66) (forward primer, F2 as indicated in FIG. 9A), 5' AGATTTAATATCACAACGTAAC 3' (SEQ ID NO:67) (reverse primer, R1 as indicated in FIG. 9A), 5' GCCAAGTAAAATTTAAGATTTA 3'(SEQ ID NO:68) (reverse primer, R2 as indicated in FIG. 9A), 5' ATTTGCTGGGTGCGTATAAAG 3' (SEQ ID NO:69) (reverse primer, R3 as indicated in FIG. 9B).
**FIG. 10(A and B)** show the amino acid sequence alignment of the two novel DAATs from ATCC 4978 and ATCC 7063 with the *B. sphaericus* DAAT (cloned in Example 18). Non-homologous amino acids are underlined.

### DETAILED DESCRIPTION OF THE INVENTION

### Abbreviations and Terms

The following explanations of terms and methods are provided to better describe the present disclosure and to guide those of ordinary skill in the art in the practice of the present disclosure. As used herein, "including" means "comprising." Wherever the term "includes" is used, it should be understood that "includes but is not limited to" is meant, whether or not "is limited to" is explicitly stated. In addition, the singular forms "a" or "an" or "the" include plural references unless the context clearly dictates otherwise. For example, reference to "comprising a protein" includes one or a plurality of such proteins, and reference to "comprising the cell" includes reference to one or more cells and equivalents thereof known to those skilled in the art, and so forth. The term "about" encompasses the range of experimental error that occurs in any measurement. Unless otherwise stated, all measurement numbers are presumed to have the word "about" in front of them even if the word "about" is not expressly used.

**Conservative substitution:** a substitution of one amino acid for another amino acid in a polypeptide, which substitution has little to no impact on the activity of the polypeptide. The substitution is considered conservative independent of whether the exchanged amino acids appear structurally or functionally similar. For example, ideally, a tryptophan aminotransferase polypeptide including one or more conservative substitutions retains tryptophan aminotransferase activity. A polypeptide can be produced to contain one or more conservative substitutions by manipulating the nucleotide sequence that encodes that polypeptide using, for example, standard procedures such as site-directed mutagenesis or PCR or other methods known to those in the art.

Non-limiting examples of amino acids that may be substituted for an original amino acid in a protein and that may be regarded as conservative substitutions if there is little to no impact on the activity of the polypeptide include: Ala substituted with ser or thr; arg substituted with gln, his, or lys; asn substituted with glu, gln, lys, his, asp; asp substituted with asn, glu, or gln; cys substituted with ser or ala; gln substituted with asn, glu, lys, his, asp, or arg; glu substituted with asn, gln lys, or asp; gly substituted with pro; his substituted with asn, lys, gln, arg, tyr; ile substituted with leu, met, val, phe; leu substituted with ile, met, val, phe; lys substituted with asn, glu, gln, his, arg; met substituted with ile, leu, val, phe; phe substituted with trp, tyr, met, ile, or leu; ser substituted with thr, ala; thr substituted with ser or ala; trp substituted with phe, tyr; tyr substituted with his, phe, or trp; and val substituted with met, ile, leu.

Further information about conservative substitutions can be found in, among other locations, Ben-Bassat et al., J. Bacteriol. 169:751-757, (1987); O'Regan et al., Gene 77:237-251, (1989); Sahin-Toth et al., Protein Sci. 3:240-247, (1994); Hochuli et al., Bio/Technology 6:1321-1325, (1988); WO 00/67796 (Curd et al.) and in standard textbooks of genetics and molecular biology.

**Derived:** For purposes of the specification and claims, a substance is "derived" from organism or source if any one or more of the following are true: 1) the substance is present in the organism/source; 2) the substance is removed from the native host; or, 3) the substance is removed from the native host and is evolved, for example, by mutagenesis.

**Isolated:** The term "isolated" as used herein refers to any substance removed from its native host; the substance need not exhibit any specific degree of purity. For example "isolated nucleic acid" refers to a naturally-occurring nucleic acid that is not immediately contiguous with both of the sequences with which it is immediately contiguous (one on the 5' end and one on the 3' end) in the naturally-occurring genome of the organism from which it is derived. For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e.g., a cDNA or a genomic DNA fragment produced by PCR or restriction endonuclease treatment) independent of other sequences as well as recombinant DNA that is incorporated into a vector, an autonomously replicating plasmid, a virus (e.g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" as used herein with reference to nucleic acid also includes any non-naturally-occurring nucleic acid because non-naturally-occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acid can be independent of other sequences, or incorporated into a vector, an autonomously replicating plasmid, a virus (e.g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

A nucleic acid existing among hundreds to millions of other nucleic acid molecules within, for example, cDNA or genomic libraries, or gel slices containing a genomic DNA restriction digest is not to be considered an isolated nucleic acid.

**Purified:** The term "purified" as used herein indicates that contaminants have been removed from the sample of interest. The term "purified" does not require absolute purity, but rather is intended as a relative term, unless otherwise indicated by the context. Thus, for example, a purified polypeptide or nucleic acid preparation can be one in which the subject polypeptide or nucleic acid is at a higher concentration than the polypeptide or nucleic acid would be in its natural environment within an organism or at a higher concentration than in the environment from which it was removed.

**Stereoinverting aminotransferase:** A "stereoinverting aminotransferase" is a polypeptide capable of preferentially or selectively producing a chiral amino acid product (such as monatin) while using an opposite chirality substrate as the amino donor. For example, a stereoinverting aminotransferase may be a D-phenylglycine aminotransferase (also called D-4-hydroxyphenylglycine aminotransferase) that preferentially or selectively uses L-glutamate as a substrate to produce R,R monatin. Non-limiting examples of stereoinverting aminotransferases include D-methionine aminotransferase (EC 2.6.1.41) and enzymes having D-phenylglycine aminotransferase activity or D-4-hydroxyphenylglycine aminotransferase activity.

**Complementing Gene:** A "complementing gene" is a gene that, when expressed, nullifies a mutation in an organism. For example, if an organism has a null mutation in one of the genes required for synthesis of tryptophan by the cell, a complementing gene could be one that, when expressed, allows the strain to grow on minimal medium (i.e., without tryptophan).

**Stereoselective Enzyme:** A "stereoselective enzyme" is an enzyme that has greater specificity and/or greater activity for one isomer, as compared to the specificity and/or activity for another isomer. For example, a stereoselective enzyme is one that has greater specificity and/or activity for R-MP than for S-MP. In preferred embodiments, a stereoselective enzyme has limited activity for one isomer as compared to another. "Limited" activity means activity that is minimally or not perceptible, for example as determined according to experiments provided herein. Example 6, for example, identifies HEXAspCP9T/R122G as an enzyme with limited activity on S,S monatin. Example 8 identifies the *S. meliloti* TatA as another enzyme with limited activity for S-MP. In Example 18, the *B. halodurans* D-aminotransferase had higher selectivity for R-MP as compared with S-MP, resulting in higher stereopurity of R,R monatin. Also, Example 19 indicates that the hybrid DAT has limited activity on S-MP compared to R-MP.

**Homologous:** The term "homologous" as used herein indicates that a protein or a nucleic acid exhibits a relatively high degree of sequence identity to a sequence of another protein or nucleic acid when the two sequences are aligned using standard methods. For example, an R-specific aldolase is homologous to the aldolase of SEQ ID NO:22 if the R-specific aldolase contains at least about 50% sequence identity to the aldolase of SEQ ID NO:22 when the two sequences are aligned using standard methods.

**EC number:** The enzyme classification number as assigned by the International Union of Biochemistry and Molecular Biology.

### Biosynthetic Pathways to Produce R,R and Other Stereoisomers of Monatin

As described, *inter alia,* in WO 03/091396 A2 (see, *e.g.,* Figures 1-3 and 11-13), monatin can be produced from tryptophan through a multi-step pathway involving biological conversions (i.e. facilitating the reaction of a substrate to a product with a polypeptide). A pathway described involves biologically converting tryptophan to indole-3-pyruvate, biologically converting indole-3-pyruvate to 2-hydroxy 2-(indol-3-ylmethyl)-4-keto glutaric acid ("MP"), and biologically converting MP to monatin. The biosynthesis pathway that is used to produce monatin may comprise, or consist essentially of, one or more of the following steps, mechanisms and/or pathways. The steps, mechanisms, and/or pathways described below are simply intended to be exemplary.

One method of producing monatin, or a salt thereof, comprises (a) producing indole-3-pyruvate from L-tryptophan, (b) producing monatin precursor from the indole-3-pyruvate, and (c) producing monatin from the monatin precursor.

Enzymes useful for converting tryptophan to indole-3-pyruvate include members of the enzyme classifications ("EC") 2.6.1.27, 1.4.1.19, 1.4.99.1, 2.6.1.28, 1.4.3.2, 1.4.3.3, 2.6.1.5, 2.6.1.-, 2.6.1.1, 2.6.1.21 and 3.5.1.-. These classes include polypeptides such as: tryptophan aminotransferase, which converts L-tryptophan and α-KG (i.e., α-ketoglutarate, also called 2-oxoglutarate) to indole-3-pyruvate and L-glutamate; D-tryptophan aminotransferase, which converts D-tryptophan and a 2-oxo acid to indole-3-pyruvate and an amino acid; tryptophan dehydrogenase, which converts L-tryptophan and NAD(P) to indole-3-pyruvate and NH₃ and NAD(P)H; D-amino acid dehydrogenase, which converts D-amino acids and FAD to indole-3-pyruvate and NH₃ and FADH₂; tryptophan-phenylpyruvate transaminase, which converts L-tryptophan and phenylpyruvate to indole-3-pyruvate and L-phenylalanine; L-amino acid oxidase, which converts an L-amino acid and H₂O and O₂ to a 2-oxo acid and NH₃ and H₂O₂; D-amino acid oxidase, which converts a D-amino acid and H₂O and O₂ to a 2-oxo acid and NH₃ and H₂O₂; and tryptophan oxidase, which converts L-tryptophan and H₂O and O₂ to indole-3-pyruvate and NH₃ and H₂O₂. These classes also contain tyrosine (aromatic) aminotransferase, aspartate aminotransferase, D-amino acid (or D-alanine) aminotransferase, and broad (multiple substrate) aminotransferase which have multiple aminotransferase activities, some of which can convert tryptophan and a 2-oxo acid to indole-3-pyruvate and an amino acid. In addition, these classes include phenylalanine deaminases, which can convert tryptophan to indole-3-pyruvate and ammonium in the presence of water.

The production of indole-3-pyruvate from L-tryptophan can also be facilitated by one or more enzymes having greater activity, greater specificity, or both, for L-tryptophan as a substrate than for either MP or monatin. Examples of enzymes having greater activity and/or greater specificity for L-tryptophan as a substrate than for either MP or monatin include, but is not limited to L-tryptophan aminotransferases, L-aromatic aminotransferases, L-aspartate aminotransferases, and L-amino acid oxidases.

Enzymes useful for converting indole-3-pyruvate to MP include members of enzyme classes EC 4.1.3.-, 4.1.3.16, 4.1.3.17, and 4.1.2.-. These classes include carbon-carbon synthases/lyases, such as aldolases that catalyze the condensation of two carboxylic acid substrates. Enzyme class EC 4.1.3.- are those synthases/lyases that form carbon-carbon bonds utilizing oxo-acid substrates (such as indole-3-pyruvate) as the electrophile, while EC 4.1.2.- are synthases/lyases that form carbon-carbon bonds utilizing aldehyde substrates (such as benzaldehyde) as the electrophile. For example, KHG aldolase (EC 4.1.3.16) and ProA aldolase (EC 4.1.3.17), are known to convert indole-3-pyruvate and pyruvate to MP. Although ProA aldolase can be thought to identify only the 4-hydroxy-4-methyl-2-oxoglutarate aldolase derived from *Comamonas testosteroni,* herein the term ProA aldolase is used to mean any polypeptide with 4-hydroxy-4-methyl-2-oxoglutarate aldolase activity unless otherwise stated. Suitable examples of Pro aldolases include *Comamonas testosteroni* ProA (SEQ ID NO:1 (nucleic acid sequence), SEQ ID NO:2 (amino acid sequence)) and *Sinorhizobium meliloti* ProA (NCBI Accession No.: CAC46344), or enzymes that display homology to *Comamonas testosteroni* ProA (SEQ ID NO:1 (nucleic acid sequence), SEQ ID NO:2 (amino acid sequence)) and/or *Sinorhizobium meliloti* ProA (NCBI Accession No.: CAC46344). For example, suitable enzymes may have at least about 40%, 50%, 60%, 70%, 80%, 90%, 95%, and/or 99% amino acid sequence identity with *Comamonas testosteroni* ProA (SEQ ID NO: 2) and/or *Sinorhizobium meliloti* ProA (NCBI Accession No.: CAC46344). MP can also be generated using chemical reactions, such as the aldol condensations.

Enzymes useful for the conversion of MP to monatin include members of the enzyme classes (EC): tryptophan aminotransferases (2.6.1.27), tryptophan dehydrogenases (1.4.1.19), D-amino acid dehydrogenases (1.4.99.1), glutamate dehydrogenases (1.4.1.2-4), phenylalanine dehydrogenase (1.4.1.20), tryptophan-phenylpyruvate transaminases (2.6.1.28), or more generally members of the aminotransferase family (2.6.1.-) such as aspartate aminotransferase (EC 2.6.1.1), tyrosine (aromatic) aminotransferase (2.6.1.5), D-tryptophan aminotransferase, or D-alanine (2.6.1.21) aminotransferase (*see* Figure 2 of WO 03/091396 A2). This reaction can also be performed using chemical reactions. Amination of the keto acid (MP) is performed by reductive amination using ammonia and sodium cyanoborohydride. Figures 11-13 of WO 03/091396 A2 show additional polypeptides that can be used to convert MP to monatin, as well as providing increased yields of monatin from indole-3-pyruvate or tryptophan.

The taste profile of a monatin composition can be altered by controlling the relative amount of the various stereoisomers of monatin in the composition. The present disclosure provides pathways and substances for producing monatin compositions with a desired percentage of R,R monatin and/or S,R monatin.

The chirality of the monatin compounds that is produced by pathways such as those exemplified herein can be altered both by pH and by the polypeptides used for the biological conversions. When monatin is formed using a biosynthetic pathway, the following can be considered. In a biocatalytic reaction, the chirality of the monatin carbon-2 (see chemical structure above) is determined by the enzyme that converts indole-3-pyruvate to MP. Multiple enzymes (e.g., from EC 4.1.2.-, 4.1.3.-) can convert indole-3-pyruvate to MP. Thus, one can choose the enzyme that forms the desired isomer. Alternatively, the enantiospecificity of the enzyme that converts indole-3-pyruvate to MP can be modified through the use of directed evolution or catalytic antibodies can be engineered to catalyze the desired reaction. Once MP is produced (either enzymatically or by chemical condensation), the amino group can be added stereospecifically. Either the R or S configuration of carbon-4 (*see* previous chemical structure) can be generated depending on whether a D- or L- aromatic acid aminotransferase is used. Many aminotransferases are specific for the L-isomer, however, D-tryptophan aminotransferases exist in certain plants (Kohiba and Mito, Proceedings of the 8th International Symposium on Vitamin B6 and Carbonyl Catalysis, Osaka, Japan 1990). Moreover, D-alanine aminotransferases (EC 2.6.1.21), D-methionine-pyruvate aminotransferases (EC 2.6.1.41) and both (R)-3-amino-2-methylpropanoate aminotransferase (EC 2.6.1.61), (S)-3-amino-2-methylpropanoate aminotransferase (EC 2.6.1.22), and D-phenylglycine aminotransferase have been identified. Certain aminotransferases may only accept the substrate for this reaction with a particular configuration at the C2 carbon. Therefore, even if the conversion to MP is not stereospecific, the stereochemistry of the final product can be controlled through the appropriate selection of an aminotransferase. Because the reaction is reversible, the unreacted MP (undesired isomer) can be recycled back to its constituents and a racemic mixture of MP can be reformed.

Referring now to the figures, the following should be noted. The flow charts identify examples of pathways for producing monatin, but the pathways shown on the figures, and the methods of the invention as defined in the claims, are not limited to any particular method for practicing the pathways, unless otherwise stated. For example, the pathways may be practiced *in vivo, in vitro,* or a combination thereof.

Furthermore, practice of a method of the disclosure utilizing one or more of the pathways disclosed herein does not require that each of the identified components (e.g. reactants and enzymes) is explicitly provided by the practitioner; rather, it is sufficient that the components, (or sources of components), and reaction conditions are present in the composition (or host cell) or otherwise available so that the pathway can potentially proceed. In other words, for example, if a figure depicts a process for producing a monatin composition, which includes producing indole-3-pyruvate from L-tryptophan, producing 2-hydroxy 2-(indol-3ylmethyl)-4-keto glutaric acid ("monatin precursor" or "MP") from indole-3-pyruvate, and producing monatin from MP, wherein each reaction is facilitated by an appropriate enzyme, it is contemplated that practice of that pathway includes combining L-tryptophan with α-ketoglutarate and enzymes contemplated for facilitating the identified reactions, and under conditions suitable for each of the reactions to occur without also explicitly providing indole-3-pyruvate or MP. In such an instance L-tryptophan could react with α-ketoglutarate to produce indole-3-pyruvate. Depending upon the conditions and the provided enzyme, the indole-3-pyruvate produced from the L-tryptophan reaction can react to form MP, and then depending upon the conditions and the provided enzyme, the MP produced from the indole-3-pyruvate reaction can react to form monatin.

It should also be noted that practice of a method of the disclosure utilizing one or more of the pathways disclosed herein does not require the practitioner to explicitly provide the identified starting materials or enzymes, if such materials or enzymes are otherwise already present or available, or capable of being synthesized from a substance that is already present or available in the reaction milieu. In other words, it is contemplated that practice of any pathways that identify L-tryptophan as a starting material would include providing a compound that can produce L-tryptophan, under conditions suitable for L-tryptophan production to occur and combining that compound with enzymes capable of facilitating the series of reactions set forth under conditions that would be suitable for those reactions to occur. As another example, it is also contemplated that practicing the identified pathway includes providing a microorganism genetically engineered to produce monatin according to the described pathway, and providing appropriate conditions for the fermentation process to occur. For example, a microorganism, which naturally produces large amounts of L-tryptophan or D-tryptophan (see U.S. Patent No. 5,728,555) be can be genetically engineered to produce or over-produce one or more of the enzymes used to facilitate (catalyze) reactions in the pathway to monatin, and appropriate conditions can be provided so that the microorganism would thereby produce monatin.

Turning now to FIG. 1, the flow chart shown schematically depicts a process for making a monatin composition including R,R monatin. As shown in FIG.1, the overall pathway involves a reaction of tryptophan to form indole-3-pyruvate, a reaction of indole-3-pyruvate to produce MP, and a reaction of MP to produce monatin, including R,R monatin.

FIG. 1 further illustrates specific permutations of this overall pathway, designed to increase the production of the R,R form of monatin at the expense of the S,S, R,S and S,R forms of monatin. In particular, FIG.1 illustrates the embodiment wherein: the aminotransferase enzyme utilized in the L-tryptophan reaction has greater activity and/or specificity for that reaction versus the reactions of MP and 4S monatin or the oxidase has greater activity and/or specificity for L-tryptophan than for 4R monatin; the enzyme which facilitates the reaction of indole-3-pyruvate is an R-specific aldolase; and, the enzyme which facilitates the reaction of MP is a broad specificity D-enzyme, preferably evolved to work more efficiently with the R isomer of MP.

FIG. 1 also illustrates particular permutations designed to make the production of R,R monatin more economical. For example, in FIG. 1 L-tryptophan-as opposed to D-tryptophan or combinations of L- and D-tryptophan-is identified as the starting material. While the choice of the specific form of tryptophan does not impact the chirality of the ultimate monatin compounds in the monatin composition (because the tryptophan reaction forms indole-3-pyruvate, which has no chirality), some may prefer utilizing L-tryptophan as a starting material at least because L-tryptophan is currently less expensive and more easily obtainable than D-tryptophan.

Focusing now on the first reaction shown in FIG. 1, when tryptophan is converted to indole-3-pyruvate any one or more of alpha-ketoglutarate, oxaloacetate, and/or pyruvate reacts with the tryptophan to form an amino acid (glutamate, aspartate, and alanine respectively) and indole-3-pyruvate. FIG. 1 depicts the embodiment wherein the tryptophan starting material is L-tryptophan, and the alpha-ketoglutarate, oxaloacetate, and/or pyruvate produce the L-isomer form of the amino acid (e.g. L-glutamate, L-aspartate, and/or L-alanine, respectively).

As shown in FIG.1, an approach to enhancing the production of R,R monatin involves facilitating the reaction of L-tryptophan with an enzyme having greater specificity, greater activity, or both for tryptophan as opposed to MP or monatin, and facilitating the reaction of MP with a D-specific enzyme. As is disclosed in WO 03/091396 A2, certain enzymes can facilitate the reaction of tryptophan to produce indole-3-pyruvate, as well as the amination reaction of MP to produce monatin. Use of an L-aminotransferase in the amination step creates an S chiral center at the monatin C-4 position, whereas use of a D-enzyme creates a D chiral center at the monatin C-4 position. Thus, in the instance where an L-aminotransferase, which facilitates the tryptophan reaction, is also active in the MP reaction, R,S and S,S monatin can be formed, depending on the form of MP present. In addition, certain other enzymes-the L-amino acid oxidases-can not only facilitate the reaction of tryptophan to indole-3-pyruvate, but may have a side activity for the degradation of R,R monatin. According to some embodiments, this 4R side activity is minimized or eliminated. An oxidase side activity on 4S forms of monatin would decrease or minimize them from the final product and could be desirable depending on the final composition desired. Consequently, the greater the specificity and/or activity of the L-enzyme chosen for tryptophan versus the MP or monatin, the greater the amount of R,R and S,R produced versus S,S and R,S monatin.

Suitable enzymes for the tryptophan reaction, in accordance with the embodiment illustrated in FIG. 1, include: L-aminotransferases capable of facilitating a reaction of L-tryptophan to form indole-3-pyruvate, and which have greater specificity for that reaction over the reaction of R-MP to form 4S isomers of monatin; and, L-amino acid oxidases capable of facilitating a reaction of L-tryptophan to form indole-3-pyruvate, and which have greater specificity and/or activity for that reaction versus the reaction of 4R isomers of monatin to form MP, and functional equivalents of any of the foregoing. More specifically, non-limiting examples of suitable enzymes can be chosen from L-tryptophan aminotransferases (EC 2.6.1.27) and tyrosine (aromatic) aminotransferases (EC 2.6.1.5) and L-amino acid oxidases (EC 1.4.3.2), and mutants derived from enzymes having aspartate aminotransferase activity.

Example 6 identifies a specific enzyme, a mutant HEXaspC polypeptide which includes a Pro 9 to Tyr substitution and an Arg 122 to Gly substitution useful for facilitating the reactions of L-tryptophan and α-KG, oxaloacetate, pyruvate, or combinations thereof to form indole-3-pyruvate and L-glutamate, L-aspartate, and L-alanine, respectively. Another specific enzyme having "limited" activity is TatA, the L-tryptophan aminotransferase from *S. meliloti.* Other enzymes suitable for the tryptophan reaction in accordance with preferred embodiments of the pathway shown in Fig. 1 include those with the following characteristics: an enzyme that transaminates MP at 1/10 the rate or less than the rate of L-tryptophan as in Example 6 or an enzyme when used with a racemase, as in Example 9, that produces greater than 90% of the 4R isomers of monatin.

Examples of enzymes not having a high degree of specificity for the L-tryptophan to indole-3-pyruvate conversion compared to the MP to monatin conversion include: HEXAspC (Example 6), *Leishmania major* broad specificity aminotransferase (WO 03/091396 A2), the Porcine aminotransferase (WO 03/091396 A2) and *Rhodobacter sphaeroides* TatA (Example 9). These enzymes may, however, be evolved, for example through mutagenesis to have limited activity for R-MP and/or R,R monatin versus tryptophan.

Focusing now on the second reaction identified in FIG. 1, the choice of enzyme for facilitating (or catalyzing) the reaction of indole-3-pyruvate to MP influences the relative amount of R,R monatin versus S,R monatin produced. In general, the greater the relative amount of R-MP versus S-MP produced, the greater the relative amount of R,R monatin versus S,R monatin produced (when a D-enzyme facilitates the reaction of MP to monatin). Enzymes useful in this regard include any enzymes that produce a higher R-MP:S-MP ratio than that produced by the reaction of indole-3-pyruvate and pyruvate when facilitated by any one of the *E. coli* KHG aldolase (Genbank Accession No. AAC74920.1), the *Bacillus* KHG aldolase (Genbank Accession No. CAB14127.1) or the *Comamonas testosteroni* ProA aldolase (SEQ ID NO:1 (nucleic acid sequence), SEQ ID NO:2 (amino acid sequence)). Thus, if it is desired to preferentially produce R-MP, one or more enzymes capable of producing greater amounts of R-MP relative to S-MP can be used. When a monatin composition having the R,R form of monatin as its only monatin component is desired, an enzyme that selectively produces R-MP as opposed to S-MP (an "R-specific enzyme") should be used. Examples of R-specific enzymes that may be used to selectively produce R-MP as opposed to S-MP are the aldolase of SEQ ID NO:22 and *Sinorhizobium meliloti* HMG aldolase, as shown in Example 3.

FIG.1 identifies the particular embodiment wherein an R-specific aldolase facilitates the reaction of indole-3-pyruvate and pyruvate to form R-MP. Also contemplated, however, is the use of aldolases for the indole-3-pyruvate and pyruvate reaction which preferentially produce R-MP, as well as aldolases that produce a higher R-MP:S-MP ratio than is produced by any one of the *E. coli* KHG aldolase (Genbank Accession No. AAC74920.1), the *Bacillus* KHG aldolase (Genbank Accession No. CAB14127.1) or the *Comamonas testosteroni* ProA aldolase (SEQ ID NO:1 (nucleic acid sequence), SEQ ID NO:2 (amino acid sequence)). In addition, it is also contemplated that indole-3-pyruvate may react with a different C3 source (for example serine or cysteine) to form R-MP and consequently other enzymes (for example other lyases or synthases) may facilitate such a reaction. Other substrates that are readily converted to pyruvate (such as oxaloacetate) may also be used. Example 3 provides sources of aldolase enzymes that may preferentially or selectively produce R-MP or produce a higher R-MP:S-MP ratio than is produced by the reaction of indole-3-pyruvate and pyruvate when facilitated by any one of the *E. coli* KHG aldolase (Genbank Accession No. AAC74920.1), the *Bacillus* KHG aldolase (Genbank Accession No. CAB14127.1) or the *Comamonas testosteroni* ProA aldolase (SEQ ID NO:1 (nucleic acid sequence), SEQ ID NO:2 (amino acid sequence)), such as the aldolase of SEQ ID NO:22. Example 5 also provides screening methods for identifying such enzymes. It is also contemplated that enzymes, which preferentially or selectively produce R-MP or produce more R-MP than any one of the *E. coli* KHG aldolase (Genbank Accession No. AAC74920.1), the *Bacillus* KHG aldolase (Genbank Accession No. CAB14127.1) or the *Comamonas testosteroni* ProA aldolase (SEQ ID NO:1 (nucleic acid sequence), SEQ ID NO:2 (amino acid sequence)) may be evolved from aldolases known or found in nature. Any techniques known in the art for evolving enzymes, for example to improve a desired characteristic-such as to increase the activity of an enzyme for a substrate-as compared to the wild type enzyme can be used. Examples 4, 5, 6, 7, 9, 10, and 11 provide some techniques for evolving enzymes.

Focusing now on the last step of the pathway identified in FIG. 1, the reaction of R-MP to form R,R monatin is shown to be facilitated by a broad specificity D-aminotransferase, for example D-alanine aminotransferase (EC 2.6.1.21, also known as D-amino acid aminotransferase or D-aspartate aminotransferase) or a D-amino acid dehydrogenase. As discussed above, the conversion of MP to monatin is an amination reaction, which creates a chiral center at the monatin C-4 carbon. Where the R-chiral form is desired at the C-4 position, enzymes should be used which produce "R" chiral centers in amino acids. Non-limiting exemplary enzymes include: a D-alanine-aminotransferase derived from *Bacillus* (Examples 15-18), including the D-alanine-aminotransferase derived from *Bacillus halodurans* (Example 18) and a mutated branched chain aminotransferase that has modified stereospecificity (Example 7).

Another exemplary enzyme includes a hybrid D-aminotransferase. The hybrid D-aminotransferase can contain structural elements from two differing amino acid aminotransferases. The hybrid D-aminotransferase can then be further evolved (e.g. via mutagenesis or recombinant engineering) for improved performance in converting MP to monatin. An example of such a hybrid D-aminotransferase is shown in Example 19. The hybrid D-aminotransferase illustrated in Example 19 included elements from a D-aminotransferase from *B. spaericus* and a D-aminotransferase from G. *stearothermophilus.* R,R-monatin was produced utilizing this D-aminotransferase (Example 19).

Example 2 also illustrates the production of R,R monatin utilizing various D-aminotransferases.

According to some embodiments, the D-aminotransferase has greater specificity, greater activity, or both for the R-MP as a substrate than for indole-3-pyruvate. In certain other embodiments, the D-aminotransferase has limited activity for the indole-3-pyruvate as a substrate. Enzymes with such characteristics may be evolved or mutated from existing enzymes, for example as shown in Example 6.

Also, the reaction of R-MP to form R,R monatin can be facilitated by a D-amino acid dehydrogenase. Example 20 illustrates the production of R,R monatin from R-MP utilizing a D-amino acid dehydrogenase (D-AADH-101 through 108, BioCatalytics). These D-amino acid dehydrogenases may be further evolved (e.g. via mutagenesis or recombinant engineering) for improved performance.

FIG. 2 depicts another strategy for targeting production of R,R monatin. Whereas in the embodiment of FIG. 1, the aldolase used in the reaction of indole-3-pyruvate to form R-MP influences the ratio of R,R:S,R formed, in the embodiment of FIG. 2, the D-enzyme that facilitates the conversion of MP to monatin influences the ratio of R,R:S,R formed. According to the embodiment of FIG. 2, a non-stereospecific enzyme may be used to facilitate the conversion of indole-3-pyruvate to MP, and consequently both S-MP and R-MP can be formed. To obtain a desired ratio of R,R monatin to S,R monatin, a D-enzyme is chosen (or evolved) with appropriate stereoselectivity for R-MP versus S-MP. Where a monatin composition having the R,R form of monatin as its only monatin component is desired, an enzyme that selectively facilitates the reaction of R-MP to monatin as opposed to S-MP to monatin would be preferred. For example, the *Bacillus halodurans* D-aminotransferase (Example 18) and the hybrid D-aminotransferase containing structural elements from both *Bacillus sphaericus* and *Geobacillus stearothermophilus* (Example 19) may be utilized as the enzyme that selectively facilitates the reaction of R-MP to monatin.

FIG.3 illustrates another alternative pathway for production of compositions enriched in R,R monatin. The pathway of FIG. 3 is a modification of the pathway of FIG. 1. In the pathway shown in FIG. 3, indole-3-pyruvate is produced indirectly, rather than directly, from L-tryptophan. More specifically, L-tryptophan is converted to D-tryptophan, and D-tryptophan is then converted to indole-3-pyruvate. Example 4 illustrates the production of R,R monatin from L-tryptophan using a tryptophan racemase.

The conversion of L-tryptophan to D-tryptophan can be facilitated by a tryptophan racemase or functional equivalent thereof. Example 4 provides potential sources of tryptophan racemases and screening methods for identifying such enzymes. Example 4 describes examples of tryptophan racemases that are capable of converting L-tryptophan into D-tryptophan. These tryptophan racemases can be further evolved (e.g. via mutagenesis or recombinant engineering) for improved performance.

Non-limiting examples of tryptophan racemases include homologs or mutants of amino acid racemases (EC 5.1.1.-), for example serine racemase, in which the homologs or mutants are capable of converting L-tryptophan to D-tryptophan. Non-limiting examples of sources from which the amino acid racemase may be derived include microorganisms such as *Salmonella typhimurium, Escherichia coli, Bacillus subtilis, Bacillus sphaericus, Bacillus halodurans, Geobacillus stearothermophilus, Bacillus licheniformis, Pseudomonas aeruginosa, Vibrio cholerae, Schizosaccaroyces pombe, Bacillus cereus, Enterococcus gallinarum, Pediococcus pentosaceus, Bacillus pumilus, Lactobacillus fermenti, Lactobacillus brevis, Aquifex pyrophilus, Lactobacilli, Streptococcus, Anabaena* sp., *Pseudomonas striata, Lentinus edodes, Scapharca brouhtonii Desulfurococcus* sp., *Thermococcus* sp., and *Pseudomonas striata.* Additional non-limiting examples of sources from which the amino acid racemase may be derived include silkworm, rat brain, or mouse brain. These amino acid racemases may be evolved (e.g. via mutagenesis or recombinant engineering) for improved performance in converting L-tryptophan to D-tryptophan.

Non-limiting examples of potential sources from which suitable tryptophan racemases may be derived include: microorganisms such as *Pseudomonas,* for example *Pseudomonas chlororaphis* (*Pseudomonas aurereofaciens*) (ATCC15926), and *Burkholderia pyrrocina* (ATCC15958). Additional non-limiting examples of potential sources from which suitable tryptophan racemases may be derived include plants, for example tobacco plants, such as *Nicotiana tabacum,* wheat plants, such as *Triticum aestivum,* beets, tomatoes, and *Sclerochiton ilicifolius.*

The pathway shown in FIG. 3 has certain benefits, including that even when R,R monatin is the desired product, the same enzyme can be used for the reaction that produces indole-3-pyruvate as for the reaction that produces monatin as a product. That is, in the pathway illustrated in FIG. 1, an L-aminotransferase (or suitable L-enzyme) facilitates the reaction producing indole-3-pyruvate, but a D-aminotransferase facilitates the reaction producing monatin. By contrast in the pathway of FIG. 3, a certain D-aminotransferase that facilitates the reaction producing indole-3-pyruvate, can also facilitate the reaction producing monatin. Consequently, in pathways according to Fig. 3, broad specificity D-aminotransferases may be preferred when there is a desire to use the same enzyme for the reaction forming indole-3-pyruvate as for the reaction forming monatin. By contrast, in pathways according to Figs. 1, 2, 4, 6, 7, and 8 production of monatin may be more efficient when a D-aminotransferase is chosen that has limited activity and/or specificity for indole-3-pyruvate as compared to R-MP.

Another benefit of the pathway schematically represented in FIG. 3 is that the amino acid product of the reaction coupled to the reaction producing indole-3-pyruvate can now be used as a substrate in the reaction coupled to the reaction producing monatin. That is, in the pathway illustrated in FIG. 1, L-tryptophan reacts to produce indole-3-pyruvate and at the same time oxaloacetate, alpha-ketoglutarate and/or pyruvate react to produce an L-amino acid. Because the reaction of R-MP to form monatin is coupled with a reaction utilizing a D-amino acid as a substrate, the L-amino acid of the reaction forming indole-3-pyruvate is not, under the conditions shown, recycled for use in the reaction coupled to the R-MP reaction. By contrast, in the pathway illustrated in FIG. 3, the reaction of D-tryptophan to form indole-3-pyruvate is coupled to a reaction forming a D-amino acid product, which D-amino acid can be recycled for use in the reaction coupled to the R-MP reaction. This allows one to use non-stoichiometric amounts of amino acceptor in step one and the amino donor for step 3 is produced in step 1.

FIGS. 4 and 5 illustrate additional modifications of the pathway shown in FIG. 1. These modifications are directed to recycling the amino acid product formed by the reaction coupled with the L-tryptophan transamination reaction with the amino acid reactant of the reaction coupled to the MP to monatin reaction.

Turning to FIG. 4, the recycling is accomplished by providing an enzyme that can facilitate the conversion of an L-amino acid to a D-amino acid and vice versa. More specifically, where, as is shown in FIG. 4, α-KG reacts to form L-glutamate when L-tryptophan reacts to form indole-3-pyruvate, a glutamate racemase (EC 5.1.1.3) or functional equivalent can be provided that can facilitate the conversion of L-glutamate to D-glutamate and vice versa. In such an instance, the L-glutamate formed as a product along with the production of indole-3-pyruvate is partially removed by virtue of its conversion to D-glutamate, and the D-glutamate formed from the conversion of L-glutamate is then available as a substrate for the reaction coupled with the MP to monatin reaction. Similarly, the α-KG formed in the reaction of D-glutamate is available as a substrate for the reaction coupled to the L-tryptophan to indole-3-pyruvate reaction.

Non-limited examples of potential sources from which a glutamate racemase may be derived include *Pediococcus pentosaceus, Bacillus pumilus, Lactobacillus fermenti, Lactobacillus brevis, E. coli, Aquifex pyrophilus,* and *Bacillus subtilis.* More specifically (also non-limiting), the glutamate racemase may be expressed from a nucleic acid such as *Pediococcus pentaosaceus murI* gene (Genbank Accession No. L22789), or *Lactobacillus brevis* glutamate racemase.

Where oxaloacetate reacts to form L-aspartate when L-tryptophan reacts to form indole-3-pyruvate, an aspartate racemase (EC 5.1.1.13) or functional equivalent can be provided to convert L-aspartate to D-aspartate. In such an instance, the L-aspartate that is formed in the same reaction that produces indole-3-pyruvate is partially removed by virtue of its conversion to D-aspartate, and the D-aspartate is then available to as a substrate for the reaction coupled to the MP to monatin reaction. Similarly, the oxaloacetate formed in the reaction of D-aspartate is available to act as a substrate for the reaction coupled to the L-tryptophan to indole-3-pyruvate reaction.

Non-limiting examples of suitable enzymes having aspartate racemase activity include ASPR-101 (BioCatalytics, Inc., 129 N. Hill Ave, Suite 103, Pasadena, CA 91106-1955) and homologs or mutants of an amino acid racemase (EC 5.1.1.-) which are capable of facilitating the conversion of L-aspartate to D-aspartate.

Non-limiting examples of potential sources from which aspartate racemases may be derived include: *Desulfur-ococcus, Thermococcus, bivalve mollusk Scapharca brouhtonii, Acinetobacter, Agrobacterium, Archaeoglobus, Bacillus, Bordetella, Bradyrhizobium, Brevibacterium, Burkholderia, Campylobacter, Candida, Caulobacter, Clostridium, Desulfitobacterium, Desulfotalea, Enterococcus, Erwinia, Escherichia, Ferroplasma, Helicobacter, Klebsiella, Lactobacillus, Mannheimia, Medicago, Mesorhizobium, Methanococcus, Methanosarcina, Oceanobacillus, Oenococcus, Pediococcus, Polaribacter, Pseudomonas, Pyrococcus, Ralsonia, Shigella, Sinorhizobium, Salmonella, Sphingomonas, Streptococcus, Thermoanaerobacter, Vibrio, Wolinella, Xanthomonas, Xanthobacter, Yersinia and Zymomonas.*

Where pyruvate reacts to form L-alanine when L-tryptophan reacts to form indole-3-pyruvate, an alanine racemase or functional equivalent can be provided to convert L-alanine to D-alanine. In such an instance, the L-alanine that is formed in the same reaction that produces indole-3-pyruvate is removed by virtue of its conversion to D-alanine, and the D-alanine formed from the conversion of L-alanine is then available to act as a substrate for the reaction coupled to the MP to monatin reaction. Similarly, the pyruvate formed in the reaction of D-alanine is available to act as a substrate for the reaction couple with the L-tryptophan to indole-3-pyruvate reaction.

Non-limiting examples of suitable alanine racemases include A8936 (Sigma, PO Box 14508, St. Louis, MO, 63178) and the *Geobacillus stearothermophilus* alanine racemase as described in Example 4.

Non-limiting examples of potential sources from which the alanine racemase may be derived include: *Brucella abortus, Streptococcus faecalis Salmonella typhimurium, Escherichia coli, Bacillus subtilis, Pseudomonas aeruginosa, Vibrio cholerae, Schizosaccaroyces pombe, Bacillus cereus and Lentinus edodes.*

Examples 9 and 12 illustrate the use of the above racemases, their impact on increasing the ratio of the desired monatin product, and provide potential sources for the racemase enzymes.

Turning to FIG. 5, a stereoinverting aminotransferase is used to facilitate the reaction of R-MP to monatin. Although typically the R-MP (or S-MP) reaction to form R,R monatin (or S,R monatin) is coupled with the reaction of a D-amino acid, a stereoinverting aminotransferase may facilitate the coupled reactions of R-MP (or S-MP) to form R,R monatin (or S,R monatin) using an L-amino acid. In this way, the L-amino acid product of the L-tryptophan aminotransferase reaction may be used as a substrate for the transamination of MP to monatin, and the product (i.e. oxaloacetate, pyruvate, and/or α-KG) of the reaction coupled to the MP to monatin reaction can be used as a starting material for the reaction coupled to the L-tryptophan to indole-3-pyruvate reaction. Non-limiting examples of stereoinverting aminotransferases that may be used include mutants derived from D-phenylglycine aminotransferase (EC 2.6.1.72, also known as D-4-hydroxyphenylglycine aminotransferase), D-methionine aminotransferase (EC 2.6.1.41, also known as D-met-aminotransferase and D-methionine-pyruvate aminotransferase), and homologs thereof. Non-limiting examples of potential sources from which the mutants of D-phenylglycine aminotransferase may be derived include *Pseudomonas,* such as *Pseudomonas putida* LW-4 and *Pseudomonas stutzeri* ST-201. Non-limiting examples of potential sources from which the D-methionine aminotransferase may be derived include cauliflower and peanut.

Examples 10 and 11 together provide potential sources of stereoinverting enzymes, and methods of making such enzymes. The examples also provide screening methods for identifying such enzymes. It is also contemplated that such enzymes may be evolved from stereoinverting enzymes known or found in nature. As a non-limiting example, the stereoinverting aminotransferase can be a homolog or mutant of a D-amino acid aminotransferase or a homolog or mutant of an amino acid racemase (EC 5.1.1.-).

FIGS. 6 and 7 also illustrate modifications to the pathway of FIG. 1. The pathways illustrated in FIGS. 6 and 7 provide methods to push equilibrium reactions forward (i.e., toward the direction of monatin production) by removing the byproduct of the tryptophan reaction with an irreversible reaction and in some cases providing substrate for the MP reaction.

Turning to FIG. 6, the pathway shown removes the L-amino acid product of the reaction coupled to the tryptophan reaction by converting it to a different L-amino acid and CO₂, and then provides a substrate for the reaction coupled to the MP reaction by converting the newly formed L-amino acid to a D-amino acid. Specifically, L-tryptophan is shown to react alongside oxaloacetate to form indole-3-pyruvate and L-aspartate. An aspartate 4-decarboxylase (EC 4.1.1.12) or functional equivalent is used to facilitate the conversion of L-aspartate to L-alanine and carbon dioxide, and an enzyme with alanine racemase activity is used to facilitate the conversion of L-alanine to D-alanine, which D-alanine can serve as an amino donor for the conversion of R-MP to monatin.

Turning to FIG. 7, the pathway shown illustrates additional methods for removing the L-amino acid product of the reaction coupled to the tryptophan reaction. Embodiments as presented in the figure produce a byproduct(s) that is unavailable to react in the reverse direction, for example due to volatility (e.g., carbon dioxide) or by spontaneous conversion to an unreactive endproduct. An example of such an approach includes embodiments in which α-KG reacts alongside L-tryptophan to produce L-glutamate, and, if desired, a glutamate decarboxylase (EC 4.1.1.15) or functional equivalent can be provided to facilitate the conversion of L-glutamate to 4-aminobutanoate (with carbon dioxide as a byproduct). Non-limiting examples of potential sources from which the L-glutamate decarboxylase can be derived include: *Clostridium perfringens, C. welchii, or E. coli.*

Another example of such an approach for driving the tryptophan reaction forward (in the direction of monatin production) includes reactions in which oxaloacetate is utilized as a co-substrate in the reaction that utilizes L-tryptophan and in which the oxaloacetate is converted to L-aspartate; if desired, an aspartate decarboxylase (EC 4.1.1.11) or functional equivalent can be provided to facilitate the conversion of L-aspartate to β-alanine (with carbon dioxide as a byproduct).

Turning to FIG. 8, the pathway shown illustrates yet additional methods for converting the L-amino acid product of the reaction coupled to the tryptophan reaction to a substrate for the reaction coupled to the MP reaction. Specifically, where α-KG is utilized in the same reaction as L-tryptophan, and in which the α-KG forms L-glutamate, an enzyme with L-alanine aminotransferase activity and pyruvate can be provided, wherein the L-alanine aminotransferase enzyme facilitates the reaction of pyruvate and L-glutamate to form L-alanine. An alanine racemase or functional equivalent can also be provided in order to facilitate the conversion of the L-alanine to D-alanine, which D-alanine can be used as a substrate along with MP to form monatin and pyruvate. See Example 12.

Implicitly described in the biosynthesis pathways above, and in the reactions described in the Examples below, are mixtures containing one or more compounds and/or enzymes required in the biosynthesis pathways for producing monatin, including R,R monatin, or monatin precursor, including R monatin precursor.

For production *in vitro,* any or all of the biosynthetic pathways described herein or individual steps in the pathways described herein can be conducted in *in vitro* solution or *in vivo,* in a host cell, in series or in parallel. When the method of the invention utilizes one or more reactions that are performed *in vitro,* the biosynthetic reaction that is performed *in vitro* can be performed by combining the desired ingredients for the reaction(s) by admixture in an aqueous reaction medium or solution. The reaction mixture so formed is maintained for a period of time sufficient for the desired product(s) to be synthesized.

Additionally, the activity of one or more enzymes can be enhanced through the continuous use of cofactors during purification of the one or more enzymes. For example, including pyridoxal-5'-phosphate when purifying *B. sphaericus* D-alanine aminotransferase results in increased activity (Example 14).

When one or more of the reactions in the pathways of the invention are to be performed *in vitro,* any or all of the enzymes utilized in the biosynthesis pathways described herein can optionally be immobilized onto a solid support. Examples of such solid supports include those that contain epoxy, aldehyde, chelators, or primary amine groups. Specific examples of suitable solid supports include, but are not limited to, Eupergit^{®} C (Rohm and Haas Company, Philadelphia, PA) resin beads and SEPABEADS^{®} EC-EP (Resindion). Example 21 illustrates the immobilization of the *B. sphaericus* D-alanine aminotransferase onto Eupergit^{®} C resin beads. Example 22 illustrates the immobilization of the *Sinorhizobium meliloti* ProA aldolase onto Eupergit^{®} C resin beads. Production of R,R monatin utilizing these immobilized enzymes is shown in Example 23.

The individual reactions shown in the biosynthetic pathways described herein can be facilitated (catalyzed) by a single enzyme or by a mixture of multiple enzymes acting concurrently.

The methods of the invention can be used to make a monatin composition that contains a desired percentage of R,R-monatin. In addition to the reaction steps described above, a specific reaction step can be catalyzed by more than one enzyme, for example, a mixture of enzymes, so that the resulting composition or preparation contains a desired percentage of R,R-monatin, including a maximum desired percentage of R,R-monatin. Alternatively, the monatin made by two separate engineered pathways according to the methods of the invention be combined to produce a composition or preparation containing such desired percentage of R,R-monatin.

When an enzyme of a designated class of enzymes is utilized as an example, it is expected that an enzyme with at least about 50%, 55%, 60%, 65%, 70%, 75%, 80%, 82%, 85%, 87%, 90%, 91%, 92%, 93%, 94%, 95%, 96%, 97%, 98%, and 99% homology could also be utilized in that reaction. For example, an R-specific aldolase with at least about 50%, 55%, 60%, 65%, 70%, 75%, 80%, 82%, 85%, 87%, 90%, 91%, 92%, 93%, 94%, 95%, 96%, 97%, 98%, and 99% homology to the aldolase of SEQ ID NO:22 could be utilized in any of the above described pathways to yield R,R monatin.

Additionally, when an enzyme of a designated class of enzymes is utilized as an example, it is expected that a fragment of that enzyme that has the same activity could also be utilized in that reaction. For example, a fragment of the aldolase of SEQ ID NO:22 that also functions as an aldolase could be utilized in any of the above described pathways to yield R,R monatin.

Monatin that is produced utilizing one or more of the polypeptides or biosynthetic pathways disclosed herein, is generally at least about 0.5-30% R,R-monatin, by weight of the total monatin produced. In other embodiments, the monatin produced utilizing one or more of the polypeptides or biosynthetic pathways disclosed herein, is greater than 30% R,R-monatin, by weight of the total monatin produced; for example, the R,R-monatin is 40%, 50%, 60%, 65%, 70%, 75%, 80%, 85%, 90%, 95% or 99% of the total monatin produced Alternatively, various amounts of two or more preparations of monatin can be combined so as to result in a preparation that is a desired percentage of R,R-monatin. For example, a monatin preparation that is 30% R,R-monatin can be combined with a monatin preparation that is 90% R,R-monatin; if equal amounts of 30% and 90% R,R-monatin preparations are combined, the resulting monatin preparation would be 60% R,R-monatin.

The monatin, or an intermediate (including monatin precursor), produced utilizing one or more of the polypeptides or biosynthetic pathways disclosed herein, may be purified from the components of the reaction. In one embodiment, the monatin or intermediate, such as monatin precursor, may be purified simply by removing the substance that is to be purified from the enzyme preparation in which it was synthesized.

In other embodiments, the intermediate, monatin precursor or monatin is purified from a preparation in which it was synthesized so that the resulting "purified" composition or preparation is at least about 5-60% monatin by weight of total organic compounds. In another embodiment, the monatin or intermediate, such as monatin precursor, may be purified to a degree of purity of at least about 70%, 80%, 90%, 95% or 99% by weight of total organic compounds.

The monatin, or the intermediate (including monatin precursor), produced utilizing one or more of the polypeptides or biosynthetic pathways disclosed herein, may be purified from the components of the reaction by any method known to a person of ordinary skill in the art. In one embodiment, the monatin or intermediate may be purified as described in Example 13. Optimally, the purified monatin or intermediate may be repeatedly recrystallized until the desired degree of purity is achieved.

### EXAMPLES

### Example 1

### Detection of Monatin, Tryptophan, Alanine, and Glutamate

This example describes methods used to detect the presence of monatin, tryptophan and glutamate. It also describes a method for the separation and detection of the four stereoisomers of monatin.

### LC/MS/MS Multiple reaction Monitoring ("MRM") Analysis of Monatin and Tryptophan

Analyses of mixtures for monatin and tryptophan derived from *in vitro* or *in vivo* biochemical reactions were performed using a Waters/Micromass liquid chromatography-tandem mass spectrometry (LC/MS/MS) instrument including a Waters 2795 liquid chromatograph with a Waters 996 Photo-Diode Array (PDA) absorbance monitor placed in series between the chromatograph and a Micromass Quattro Ultima triple quadrupole mass spectrometer. LC separations were made using an Xterra MS C₈ reversed-phase chromatography column, 2.1mm x 250 mm at 40 °C. The LC mobile phase consisted of A) water containing 0.05% (v/v) trifluoroacetic acid and B) methanol containing 0.05% (v/v) trifluoroacetic acid.

The gradient elution was linear from 5% B to 35% B, 0-4 min, linear from 35% B to 60% B, 4-6.5 min, linear from 60% B to 90% B, 6.5-7 min, isocratic at 90% B 7-11 min, linear from 90% B to 95% B, 11-12 min, linear from 95% B to 5% B, 12-13 min, with a 2 min re-equilibration period between runs. The flow rate was 0.25 mL/min, and PDA absorbance was monitored from 200 nm to 400 nm. All parameters of the ESI-MS were optimized and selected based on generation of protonated molecular ions ([M + H]⁺) of the analytes of interest, and production of characteristic fragment ions. The following instrumental parameters were used for LC/MS/MS Multiple Reaction Monitoring (MRM) analysis of monatin and tryptophan: Capillary: 3.5 kV; Cone: 40 V; Hex 1: 20 V; Aperture: 0 V; Hex 2: 0 V; Source temperature: 100 °C; Desolvation temperature: 350 °C; Desolvation gas: 500 L/h; Cone gas: 50 L/h; Low mass resolution (Q1): 12.0; High mass resolution (Q1): 12.0; Ion energy: 0.2; Entrance: -5 V; Collision Energy: 8; Exit: IV; Low mass resolution (Q2): 15; High mass resolution (Q2): 15; Ion energy (Q2): 3.5; Multiplier: 650. Five monatin-specific parent-to daughter MRM transitions are used to specifically detect monatin in *in vitro* and *in vivo* reactions. The transitions monitored are 293.1 to 158.3, 293.1 to 168.2, 293.1 to 211.2, 293.1 to 230.2, and 293.1 to 257.2. Tryptophan is monitored with the MRM transition 204.7 to 146.4. For internal standard quantification of monatin and tryptophan, four calibration standards containing four different ratios of each analyte to d5-tryptophan and d5-monatin, are analyzed. These data are subjected to a linear least squares analysis to form a calibration curve for monatin and tryptophan. To each sample is added a fixed amount of d5-tryptophan and d5-monatin (d5-monatin was synthesized from d5-tryptophan according to the methods from WO03/091396 A2), and the response ratios (monatin/d5-monatin; tryptophan/d5-tryptophan) used in conjunction with the calibration curves described above to calculate the amount of each analyte in the mixtures.

### Accurate Mass Measurement of monatin.

High resolution MS analysis was carried out using an Applied Biosystems-Perkin Elmer Q-Star hybrid quadrupole/time-of-flight mass spectrometer. The measured mass for protonated monatin used tryptophan as an internal mass calibration standard. The calculated mass of protonated monatin, based on the elemental composition C₁₄H₁₇N₂O₅ is 293.1137. Monatin produced using the biocatalytic process described in Examples 2 and 3 showed a measured mass of 293.1144. This is a mass measurement error of less than 2 parts per million (ppm"), providing conclusive evidence of the elemental composition of monatin produced enzymatically.

### Chiral LC/MS/MS ("MRM") Measurement of Monatin

Determination of the stereoisomer distribution of monatin in *in vitro* and *in vivo* reactions was accomplished by derivitization with 1-fluoro-2-4-dinitrophenyl-5-L-alanine amide ("FDAA"), followed by reversed-phase LC/MS/MS MRM measurement.

### Derivitization of Monatin with FDAA

To 50 µL of sample or standard was added 200 µL of a 1% solution of FDAA in acetone. Forty µL of 1.0 M sodium bicarbonate was added, and the mixture incubated for 1 h at 40 °C with occasional mixing. The sample was removed and cooled, and neutralized with 20 µL of 2.0 M HCl (more HCl may be required to effect neutralization of a buffered biological mixture). After degassing is complete, samples were ready for analysis by LC/MS/MS.

### LC/MS/MS Multiple Reaction Monitoring for the Determination of the Stereoisomer Distribution of Monatin in in vitro and in vivo Reactions.

Analyses were performed using the LC/MS/MS instrumentation described above. LC separations capable of separating all four stereoisomers of monatin (specifically FDAA-monatin) were performed on a Phenomenex Luna 2.0 x 250 mm (3 µm) C18 reversed phase chromatography column at 40 °C. The LC mobile phase consisted of A) water containing 0.05% (mass/volume) ammonium acetate and B) acetonitrile. The elution was isocratic at 13% B, 0-2 min, linear from 13% B to 30% B, 2-15 min, linear from 30% B to 80% B, 15-16 min, isocratic at 80% B 16-21 min, and linear from 80% B to 13% B, 21-22 min, with a 8 min re-equilibration period between runs. The flow rate was 0.23 mL/min, and PDA absorbance was monitored from 200 nm to 400 nm. All parameters of the ESI-MS were optimized and selected based on generation of deprotonated molecular ions ([M - H]⁻) of FDAA-monatin, and production of characteristic fragment ions.

The following instrumental parameters were used for LC/MS analysis of monatin in the negative ion ESI/MS mode: Capillary: 2.0 kV; Cone: 25 V; Hex 1: 10 V; Aperture: 0 V; Hex 2: 0 V; Source temperature: 100°C; Desolvation temperature: 350 °C; Desolvation gas: 500 L/h; Cone gas: 50 L/h; Low mass resolution (Q1): 12.0; High mass resolution (Q1): 12.0; Ion energy: 0.2; Entrance: -5V; Collision Energy: 20; Exit: 1V; Low mass resolution (Q2): 12; High mass resolution (Q2): 12; Ion energy (Q2): 3.0; Multiplier: 650. Three FDAA-monatin-specific parent-to daughter transitions are used to specifically detect FDAA-monatin in *in vitro* and *in vivo* reactions. The transitions are 543.6 to 268.2, 543.6 to 499.2, and 543.6 to 525.2. Identification of FDAA-monatin stereoisomers is based on chromatographic retention time as compared to purified synthetic monatin stereoisomers, and mass spectral data.

### Liquid Chromatography-Post Column Fluorescence Detection of Amino Acids Including Glutamate and Alanine

Liquid chromatography with post-column fluorescence detection for the determination of glutamate in *in vitro* and *in vivo* reactions was performed on a Waters 2690 LC system or equivalent combined with a Waters 474 scanning fluorescence detector, and a Waters post-column reaction module. LC separations were performed on an Interaction-Sodium loaded ion exchange column at 60 °C. Mobile phase A was Pickering Na 328 buffer (Pickering Laboratories, Inc.; Mountain View, CA). Mobile phase B was Pickering Na 740 buffer. The gradient elution was from 0% B to 100% B, 0-20 min, isocratic at 100% B, 20-36 min, and linear from 100% B to 0% B, 36-37 min, with at least an 8 min re-equilibration period between runs, depending on sample matrix. The flow rate for the mobile phase was 0.5 mL/min. The flow rate for the OPA post-column derivitization solution was 0.5 mL/min. The fluorescence detector settings were EX 338 nm and Em 425 nm. Norleucine was employed as an internal standard for the analysis. Identification of amino acids was based on chromatographic retention time data for purified standards.

### Detection of L- and D-Amino Acids by LC/MS/MS

Samples containing a mixture of L- and D-amino acids such as tryptophan, glutamate, and aspartate from biochemical reaction experiments were first treated with formic acid to denature protein. The sample was then centrifuged and filtered through a 0.45 µm nylon syringe filter prior to LC/MS/MS analysis. Identification of L- and D-amino acids was based on retention time and mass selective detection. LC separation was accomplished by using Waters 2690 liquid chromatography system and an ASTEC 2.1mm x 250 mm Chirobiotic TAG chromatography column with column temperature set at 45° C. LC mobile phase A and B were 0.25% acetic acid and 0.25% acetic acid in methanol respectively. Isocratic elution of 60% mobile phase A and 40% B with flow rate of 0.25 ml/min. was set up for glutamate; whereas 30% mobile phase A and 70% B with flow rate of 0.3 ml/min was set up for aspartate and tryptophan.

The detection system for analysis of L- and D-amino acids included a Waters 996 Photo-Diode Array (PDA) detector and a Micromass Quattro Ultima triple quadrupole mass spectrometer. The PDA, scanning from 195 to 350 nm, was placed in series between the chromatography system and the mass spectrometer. Parameters for the Micromass Quattro Ultima triple quadrupole mass spectrometer operating in positive electrospray ionization mode (+ESI) were set as the following: Capillary: 3.0 kV; Cone: 20 V; Hex 1: 15 V; Aperture: 1 V; Hex 2: 0 V; Source temperature: 100°C; Desolvation temperature: 350 °C; Desolvation gas: 530 L/h; Cone gas: 30 L/h; Low mass Q1 resolution: 12.5; High mass Q1 resolution: 12.5; Ion energy 1: 0.2; Entrance: -5; Collision: 8; Exit 1: 10; Low mass Q2 resolution: 12.5; High mass Q2 resolution: 12.5; Ion energy 2: 0.5; Multiplier: 650 V. MS/MS experiments with Multiple Reaction Monitoring (MRM) mode were set up to selectively monitor reaction transitions of 204.70 to 146.50, 147.8 to 84.2, 147.8 to 102.1, 134.00 to 74.30, and 134.00 to 88.2. Quantification of tryptophan, glutamate, and aspartate acids were based off signal responses of m/z= 146.5, m/z= 102.1, and m/z= 88.2 respectively.

### Production of Monatin and Monatin Precursor ("MP") for Standards and for Assays Production of Monatin

A racemic mixture of R,R and S,S monatin was synthetically produced as described in U.S. Patent No. 5,128,482.

The R,R and S,S monatin were separated by a derivatization and hydrolysis step. Briefly, the monatin racemic mixture was esterified, the free amino group was blocked with Cbz, a lactone was formed, and the S,S lactone was selectively hydrolyzed using an immobilized protease enzyme. The monatin can also be separated as described in Bassoli, A. et al., Eur. J. Org. Chem., 8:1652-1658, (2005).

### MP Production

R-MP was produced by the transamination of R,R monatin using AT-103 broad range D-aminotransferase (BioCatalytics) in 0.1 M potassium phosphate buffer, using sodium pyruvate as the amino acceptor. S-MP was produced by the transamination of S,S monatin using AT-102 L-aminotransferase (BioCatalytics) in 0.1 M potassium phosphate buffer, using sodium pyruvate as the amino acceptor. Both reactions were carried out at 30 °C and at a pH of approximately 8.0-8.3, for approximately 20 hours. Both compounds were purified using preparative scale HPLC with a Rohm and Haas (Philadelphia, PA) hydrophobic resin (XAD™1600), eluting in water. Samples containing greater than 90% purity monatin precursor were collected and freeze-dried.

### Example 2

### Production of Monatin from Indole-3-Pyruvate

AT-103 transaminase was part of a transaminase library purchased from BioCatalytics (Pasadena, CA) and the enzyme was tested for production of monatin in coupled reactions using the ProA aldolase from *C*. *testosteroni.* The aldolase was prepared as described in WO 03/091396 A2. AT-103 is a broad specificity D-transaminase (EC 2.6.1.21) from a *Bacillus* species that requires a D-amino acid (such as D-glutamate, D-aspartate, or D-alanine) as the amino acid donor. Enzymes and additional components/substrates were added directly to the reaction buffer provided in the kit, which contained 100 mM potassium phosphate buffer pH 7.5, 100 mM amino donor, and 0.1 mM pyridoxal-5'-phosphate ("PLP"). To one mL of reaction buffer were added: 4 mg indole-3-pyruvate, 20 mg pyruvate, approximately 50 µg ProA provided in a cellular extract, 1 µL 2 M MgCl₂, and 2 mg of aminotransferase enzyme. Reactions were performed in duplicate. The reactions were incubated overnight at 30 °C with gentle shaking (100 rpm). The samples were filtered and submitted for reversed-phase LC/MS/MS analysis as described in Example 1. The results indicated that approximately 370 µg/mL monatin were produced using AT-103 enzyme. The results were further analyzed to determine ratios of S,R/R,S versus R,R/S,S monatin, on the basis of the peak areas of the two stereoisomer pools that resolve during the chromatographic separation. Of the total monatin produced by AT-103, 69% was R,R/S,S monatin in comparison to the mixed isomers. This enzyme is homologous to the *Bacillus subtilis* DAT enzyme described in WO 03/091396 A2, which is known to have a broad specificity for D-amino acids. Chiral analysis was performed using the FDAA methodology described in Example 1, which verified that the D-aminotransferase was making predominantly R,R monatin, and some S,R monatin as expected. Further transamination experiments with S,S monatin or R,R monatin and α-ketoglutarate as substrates verified that the BioCatalytics enzyme was highly selective for the D-configuration at carbon 4, as expected. In these experiments, no glutamate was detected in the reaction with S,S monatin and α-ketoglutarate as substrates.

To decrease the amount of S,S monatin or R,S monatin produced as byproducts in coupled reactions with AT-103 (the broad range D-transaminase) and the ProA aldolase, the aldolase was purified using His-Bind cartridges, following manufacturer's protocols (Novagen, Madison, WI). The purified enzyme preferably should not contain wildtype L-aminotransferase activities that can be present in cellular extracts (such as the native *E. coli* AspC or TyrB activities). The His-Bind eluent was desalted to remove imidazole using PD-10 columns (G25 Sephadex, Amersham-Pharmacia) and was eluted in 50 mM Tris-Cl, pH 7. Experiments were carried out in duplicate in a volume of 1 mL and contained 100 mM Tris-Cl buffer, pH 7.8, 50 µg ProA aldolase, 4 mg indole-3-pyruvate, 1 or 2 mg D-aminotransferase, 200 mM sodium pyruvate, 2 mM MgCl₂, 3 mM potassium phosphate, 0.1 mM PLP, and 14.7 mg of D-glutamate. The tubes were incubated at 30 °C with gentle shaking. Two-hour time points were taken and frozen immediately at -20 °C. The pH was adjusted at two hours from 5 to between 7-8 using NaOH, and the assays were incubated overnight. Samples were filtered and analyzed for monatin as described in Example 1. The two-hour samples did not have detectable amounts of monatin, probably due to the low pH. The overnight samples contained approximately 190 ng/mL monatin when 1 mg of D-aminotransferase was used, and approximately 84% was R,R monatin and 16% was S,R monatin. When 2 mg of D-aminotransferase were used, 540 ng/mL monatin was produced, approximately 71% was R,R monatin.

Similar experiments were conducted using Biocatalytics Aminotransferase buffer, which contained 100 mM potassium phosphate pH 7.5, 0.1 mM PLP, and 100 mM D-glutamate. Solid indole-3-pyruvate and D-aminotransferase were added as above. ProA aldolase (50 µg), MgCl₂, and 50 mM pyruvate were added from stock solutions. The assays were treated as above, although no pH adjustment was required in this case. A negative control was done with just the BioCatalytics supplied enzyme and buffer, which did not contain monatin. The experimental results are shown in Table 1.

**Table 1: Production of Monatin from Indole-3-Pyruvate in Phosphate Buffer**

| Mg D-Aminotransferase | Time (hours) | Total Monatin (ng/mL) | %R,R |
|---|---|---|---|
| 0 | 2 | 0 | n/a |
| 1 | 2 | 6780 | not determined |
| 2 | 2 | 13170 | 55% |
| 0 | 16 | 0 | n/a |
| 1 | 16 | 15000 | not determined |
| 2 | 16 | 28930 | 51% |

The production of monatin in phosphate buffer is clearly higher than that in Tris buffered systems.

To compare activities of the cloned *B. subtilis* DAT from WO 03/091396 A2 with the BioCatalytics enzyme (AT-103) additional assays were done. The *B. subtilis dat* gene was also subcloned into pET30a to remove the His-6 tag. Untagged and tagged enzyme were produced in BL21(DE3), as described in WO 03/091396 A2. Cellular extracts were made and total protein assays were done to estimate protein concentration as described previously. Duplicate one mL reactions were done which contained: 500 µg D-aminotransferase, 50 µg ProA aldolase, 100 mM potassium phosphate pH 7.5, 3 mM MgCl₂, 4 mg indole-3-pyruvate, 200 mM sodium pyruvate, 7.35 mg (50 mM) D-glutamate, and 0.1 mM PLP. Samples were incubated at 30 °C for 1 hour, 2 hours, and overnight, and were filtered for LC/MS/MS analysis. The samples contained only the S,R and R,R stereoisomers of monatin, as determined by the FDAA derivitization protocol described in Example 1. The results are summarized in Table 2 below. The % RR was determined by peak areas that were separated by reversed phase chromatography.

**Table 2: Comparison of D-Aminotransferase Enzymes**

| Enzyme | Time (Hours) | Monatin (ppb) | % RR Monatin |
|---|---|---|---|
| *B. sub* DAT-HIS | 1 | 512 | not determined |
| *B. sub* DAT untagged | 1 | 1056 | not determined |
| BioCatalytics AT-103 | 1 | 2353 | not determined |
| *B. sub* DAT-HIS | 2 | 894 | ∼80-90% |
| *B. sub* DAT untagged | 2 | 1913 | ∼80% |
| BioCatalytics AT-103 | 2 | 6887 | 92.5% |
| *B. sub* DAT-HIS | 16 | 3014 | 31 |
| *B. sub* DAT untagged | 16 | 5612 | 33 |
| BioCatalytics AT-103 | 16 | 16131 | 66 |

The removal of the HIS-6 tag appears to have improved the activity of the *B. subtilis* D-aminotransferase; however, the BioCatalytics D-aminotransferase homolog clearly had the highest activity. It also showed greater substrate preference for the R-monatin precursor. Increased incubation times appear to reduce the enantiomeric excess of R,R monatin that is produced.

Because the *Bacillus* D-aminotransferase enzymes have a preference for pyruvate as an amino acceptor, and D-alanine as an amino donor, it was expected that D-alanine could be utilized as the amino donor for conversion of MP to monatin with similar or better results. Duplicate one mL reactions were done which contained: 500 µg D-aminotransferase, 50 µg purified ProA aldolase, 100 mM potassium phosphate pH 7.5, 3 mM MgCl₂, 4 mg indole-3-pyruvate, 100 mM sodium pyruvate, 25 mM D-glutamate or D-alanine, and 0.1 mM PLP. Samples were incubated for 2 hours, and treated as above prior to analysis. When D-alanine was used as the amino donor, slightly higher levels of monatin were produced (23 versus 21 ppm) as expected. Additionally, it is expected that high concentrations of pyruvate may inhibit the transamination step, thus dosing in smaller amounts of pyruvate over time may improve the overall rate of monatin production. One can see from the above data that even though one-half of the pyruvate was used in this case compared to the above table, significantly more monatin was produced. Even though ProA aldolases in the literature were reported to produce primarily S-enantiomers of aldol condensation products, the ProA aldolase used in this study clearly makes a high percentage of R-MP and in coupled assays produces up to 92% R,R monatin. The high percentage of R,R monatin is not due to D-aminotransferase selectivity, as was shown in Example 19.

### Example 3

### Production of R,R Monatin from D-Tryptophan

The following were added per 1 mL of reaction mixture: approximately 60 µg *C*. *testosteroni* ProA aldolase (supplied in cellular extracts, as described in WO 03/091396 A2), 4 mM MgCl₂, 50 mM D-tryptophan, 0.5 mg BioCatalytics D-aminotransferase (AT-103), 100 mM sodium pyruvate, 100 mM potassium phosphate buffer pH 7.5 or 100 mM sodium acetate buffer pH 8, 0.05 mM PLP, 3 mM potassium phosphate (only to the acetate reactions), and 10 mM α-ketoglutarate. Experiments were run in duplicate, with negative controls in which no aldolase was added. Samples were incubated overnight (20 hours) at 30 °C with gentle shaking. The actual pH of the sodium acetate samples was approximately 5, while the final pH for the phosphate buffered samples was approximately 7. None of the aldolases appeared to have significant activity at pH 5; the sample containing ProA aldolase was slightly above the negative control but probably not above experimental error. In potassium phosphate, the ProA aldolase produced 73.4 ppm monatin with a ratio of R,R:S,R of 1.7:1 (∼63% R,R from D-tryptophan).

Because the *Bacillus* D-aminotransferase enzymes have a preference for pyruvate as an amino acceptor, and D-alanine as an amino donor, it was expected that the addition of alpha-ketoglutarate is unnecessary when producing R,R or S,R monatin from D-tryptophan. The above experiment was repeated (in 100 mM potassium phosphate buffer) using purified ProA aldolase (50-60 µg), and an incubation time of 2.5 hours. Duplicate experiments were run, with and without alpha-ketoglutarate. When 10 mM alpha-ketoglutarate was added, 56.1 ppm monatin was formed using D-tryptophan as the substrate (79.5 % R,R, 20.5% S,R). When alpha-ketoglutarate was omitted, 102.5 ppm monatin was formed (79% R,R, 21% S,R).

### Comparison of total monatin production and isomeric distribution for HMG aldolases from Sinorhizobium meliloti, C. testosteroni, and the aldolase of SEQ ID NO:22.

AT-103 transaminase (a broad specificity D-aminotransferase) was purchased from BioCatalytics (Pasadena, CA) and either this enzyme or the *B. sphaericus* recombinant enzyme produced in Example 18 was used in coupled reactions with HMG aldolases to produce monatin from D-tryptophan and pyruvate as described in U.S. Published Application No. 2005282260.

The HMG aldolases from *C*. *testosteroni* (ProA) and *S. meliloti* were prepared and purified as described in U.S. Publication No. 20040063175 and WO 03091396 A2. To produce test quantities of the aldolase of SEQ ID NO:22, a 50 mL culture was grown in Luria-Bertani ("LB") medium containing ampicillin (100 µg/mL), to an OD₆₀₀ of approximately 0.5. The strain containing the SEQ ID NO:21 construct was induced with 200 µg/L anhydrotetracycline. The cells were grown 5 hours post-induction, and cellular extracts were prepared according to manufacturer's protocols (Novagen, Bugbuster reagent). Benzonuclease and protease inhibitor were also added. The soluble proteins in the cellular extracts were separated on a BioRad Laboratories Experion Automated Electrophoresis Station and analyzed for concentration and percent expression using the Experion Software version 1.1.98.0. The aldolase of SEQ ID NO:22 was used as a crude (unpurified) enzyme for the reactions below.

The following were added per 1 mL of reaction mixture: approximately 50 µg aldolase, 4 mM MgCl₂, 50 mM D-tryptophan, 0.5 mg purified *B. sphaericus* D-aminotransferase, 200 mM sodium pyruvate, 100 mM potassium phosphate buffer pH 7.5, and 0.05 mM PLP. Experiments were run in duplicate, with negative controls in which no aldolase was added. Samples were incubated 1 hour and overnight (18 hours) at 30 °C with gentle shaking. Small amounts of monatin (< 0.5 ppm) are produced without aldolase in overnight reactions, due to non-enzymatic reactions catalyzed by magnesium and phosphate. Those values were subtracted from the numbers shown below, and averaged results are shown. The only stereoisomers detected when producing monatin using these methods are R,R and S,R. The percent R,R is listed below, and was determined by reversed-phase LC peak area.

**Table 3: Total Monatin Produced from D-Tryptophan and % R,R**

| Enzyme (timepoint) | Total Monatin (ppm) | % R,R Monatin |
|---|---|---|
| *C. testosteroni* ProA (1 hour) | 16.63 | 86.45 |
| *C. testosteroni* ProA (18 hours) | 86.86 | 63.1 |
| *S. meliloti* HMG (1 hour) | 20.5 | 96.7 |
| *S. meliloti* HMG (18 hours) | 88.3 | 89.9 |
| SEQ ID NO:22 (1 hour) | 14.70 | 100 |
| SEQ ID NO:22 (18 hours) | 95.14 | 97.35 |

The 18 hour sample for the aldolase of SEQ ID NO:22 was also analyzed for stereoisomeric distribution by the FDAA derivatization method listed in Example 1, which yielded a result of 94.9% R,R and 5.1% S,R monatin. The aldolase of SEQ ID NO:22 has a higher enantiospecificity for production of R-MP as compared to *C*. *testosteroni* and *S. meliloti* HMG aldolases.

The same experiments were done, side by side, using L-tryptophan as the starting substrate and coupling the aldolases with HexAspC broad specificity L-aminotransferase produced and purified as described in U.S. Published Application No. 2005282260. These reactions should yield primarily S,S monatin and R,S monatin. The reactions were also supplemented with 10 mM alpha-ketoglutarate as the amino acceptor for L-tryptophan transamination. Again, duplicate results are averaged below for total monatin (subtracting background levels without aldolase present), and percent S,S monatin is shown based on reversed phase LC peak area. In some cases, because the aldolases are quite R-specific and produce little total monatin, the reversed phase estimates of stereoisomeric distribution are less accurate due to some tailing of the tryptophan peak that can co-elute with the S,S/R,R monatin peak. The trends are still informative in comparing R-specificity of the aldolases. Results from further analysis using the FDAA derivatization method are shown in parentheses for several samples, and are more accurate. Total monatin numbers above approximately 400 ppm are higher than the linear range of the scale of the standards used to quantitate the results, so are qualitative results. The *C*. *testosteroni* ProA aldolase typically produces 95-100% S,S monatin, as shown in U.S. Published Application No. 2005282260.

**Table 4: Total Monatin Produced from L-Tryptophan and % S,S**

| Enzyme (timepoint) | Total Monatin (ppm) | % S,S Monatin |
|---|---|---|
| *C*. *testosteroni* ProA (1 hour) | 440.35 | 92.5 |
| *C. testosteroni* ProA (18 hour) | 958.3 | 92.2 |
| *S. meliloti* HMG (1 hour) | 45.9 | 66.3 |
| *S. meliloti* HMG (18 hour) | 108.1 | 61.4 |
| SEQ ID NO:22 (1 hour) | 17.85 | 55.1 (18.9) |
| SEQ ID NO:22 (18 hour) | 135.5 | 27.3 (19.1) |

One can see that the R-specificity of the aldolase of SEQ ID NO:22 is quite high compared to the benchmark ProA enzyme. This R-specificity is also reflected in the low % S,S monatin produced, despite the high degree of specificity of the HexAspC aminotransferase for S-MP in these reactions. Again the *S. meliloti* HMG aldolase falls between the *C*. *testosteroni* ProA aldolase and the aldolase of SEQ ID NO:22 in terms of R-specificity, based on the levels of S,S monatin produced. The total monatin numbers, when comparing S,S monatin production versus R,R monatin production, are not indicative of the aldolase activity. The D-aminotransferase is less active than HexAspC for MP transamination reactions, particularly at the concentrations of MP that are present in these reactions.

For further comparison of the aldolase of SEQ ID NO:22 to the ProA enzyme from *C*. *testosteroni,* varying ratios of D-aminotransferase to aldolase were utilized in reactions starting with D-tryptophan (no duplicate samples for these experiments). The reactions were carried out as described above. For the reactions in which the aldolase concentration was kept constant, approximately 50 µg aldolase was used. For reactions in which the amount of D-aminotransferase was kept constant, 0.5 mg was used. For the 2 and 10 mg/mL concentration of D-aminotransferase, lyophilized enzyme was used. For the 2 highest D-aminotransferase concentrations, duplicates were run.

**Table 5. Effect of D-Aminotransferase Concentration on R,R Monatin Production**

| Aldolase | Concentration of D-Aminotransferase | Time | Total Monatin (approximate ppm) | % R,R Monatin |
|---|---|---|---|---|
| SEQ ID NO:22 | 0.25 mg/mL | 1 hour | 2 | 100 |
| SEQ ID NO:22 | 0.25 mg/mL | overnight | 141 | 97.1 |
| SEQ ID NO:22 | 0.5 mg/mL | 1 hour | 8 | 100 |
| SEQ ID NO:22 | 0.5 mg/mL | overnight | 273 | 96.5 |
| SEQ ID NO:22 | 1 mg/mL | 1 hour | 34 | 100 |
| SEQ ID NO:22 | 1 mg/mL | overnight | 638 | 96.5 |
| SEQ ID NO:22 | 2 mg/mL | 1 hour | 979 | 100 |
| SEQ ID NO:22 | 2 mg/mL | overnight | 1910 | 97.3 |
| SEQ ID NO:22 | 10 mg/mL | 1 hour | 2930 | 99.1 |
| SEQ ID NO:22 | 10 mg/mL | overnight | 2950 | 96.5 |
| *C. testosteroni* ProA | 0.25 mg/mL | 1 hour | 4 | 78.7 |
| *C. testosteroni* ProA | 0.25 mg/mL | overnight | 257 | 61.1 |
| *C. testosteroni* ProA | 0.5 mg/mL | 1 hour | 25 | 79.0 |
| *C. testosteroni* ProA | 0.5 mg/mL | overnight | 480 | 62.5 |
| *C. testosteroni* ProA | 1 mg/mL | 1 hour | 74 | 73.8 |
| *C. testosteroni* ProA | 1 mg/mL | overnight | 810 | 68.1 |
| *C. testosteroni* ProA | 2 mg/mL | 1 hour | 325 | 73.1 |
| *C. testosteroni* ProA | 2 mg/mL | overnight | 2220 | 71.9 |
| *C. testosteroni* ProA | 10 mg/mL | 1 hour | 2910 | 59.7 |
| *C. testosteroni* ProA | 10 mg/mL | overnight | 2450 | 67.5 |

For monatin levels above 400 ppm, the results are not in the linear range of the standard curve and are approximate values only. The maximum amount of R,R monatin produced, when diluted appropriately, was approximately 1100 ppm. FDAA stereoisomeric analysis was done for the aldolase of SEQ ID NO:22 with 10 mg/mL D-aminotransferase samples. At two hours, the sample contained 98.5% R,R monatin. At 17 hours, the sample contained 95.9% R,R monatin. The aldolase of SEQ ID NO:22 produced high percentages of R,R monatin, even after long incubation times and using large amounts of aminotransferase. If adequate D-aminotransferase is supplied, the aldolase of SEQ ID NO:22 produces as much total monatin as *C*. *testosteroni* ProA aldolase, indicating a similar specific activity.

**Table 6. Effect of Aldolase Concentration on R,R Monatin Production**

| Aldolase | Concentration of Aldolase | Time | Total Monatin (ppm) | % R,R Monatin |
|---|---|---|---|---|
| SEQ ID NO:22 | 25 µg/mL | 1 hour | 7.0 | 100 |
| SEQ ID NO:22 | 25 µg/mL | overnight | 275 | 97.4 |
| SEQ ID NO:22 | 50 µg/mL | 1 hour | 9.0 | 97.3 |
| SEQ ID NO:22 | 50 µg/mL | overnight | 334 | 95.7 |
| SEQ ID NO:22 | 100 µg/mL | overnight | 297 | 93.3 |
| *C. testosteroni* ProA | 25 µg/mL | 1 hour | 16 | 78.2 |
| *C*. *testosteroni* ProA | 25 µg/mL | overnight | 491 | 73.2 |
| *C. testosteroni* ProA | 50 µg/mL | 1 hour | 18 | 64.1 |
| *C. testosteroni* ProA | 50 µg/mL | overnight | 437 | 63.0 |
| *C. testosteroni* ProA | 100 µg/mL | 1 hour | 26 | 62.5 |
| *C. testosteroni* ProA | 100 µg/mL | overnight | 513 | 61.5 |

When the aldolase concentration is varied, there is not much of an increase in total monatin. The percent R,R decreases with time and also with aldolase concentration, particularly when the D-aminotransferase is limiting.

To further examine the R-specificity of the aldolases tested, experiments were done starting with L-tryptophan and HexAspC aminotransferase, which was produced and purified as described in U.S. Published Application No. 2005282260. The HexAspC shows a strong selectivity for transamination of S-MP versus R-MP, thus percentages above 50% R,S monatin indicate a highly stereospecific aldolase. Ten mM alpha-ketoglutarate was supplied as an amino acceptor; however, at high concentrations, pyruvate is also utilized by the L-aminotransferase. In these reactions, typically only S,S and R,S monatin are produced within the limits of detection of the FDAA derivatization protocol.

**Table 7. Effect of L-Aminotransferase Concentration on S,S Monatin Production**

| Aldolase | Concentration of L-Aminotransferase | Time | Total Monatin (approximate ppm) | % S,S Monatin |
|---|---|---|---|---|
| SEQ ID NO:22 | 0.25 mg/mL | 1 hour | 13 | 33.8 |
| SEQ ID NO:22 | 0.25 mg/mL | overnight | 127 | 34.2 |
| SEQ ID NO:22 | 0.5 mg/mL | 1 hour | 31 | 30.9 |
| SEQ ID NO:22 | 0.5 mg/mL | overnight | 272 | 26.8 |
| SEQ ID NO:22 | 1 mg/mL | 1 hour | 34 | 20.3 |
| SEQ ID NO:22 | 1 mg/mL | overnight | 385 | 23.5 |
| *C. testosteroni* ProA | 0.25 mg/mL | 1 hour | 523 | 94.2 |
| *C. testosteroni* ProA | 0.25 mg/mL | overnight | 1817 | 93.7 |
| *C. testosteroni* ProA | 0.5 mg/mL | 1 hour | 602 | 91.8 |
| *C. testosteroni* ProA | 0.5 mg/mL | overnight | 2122 | 89.9 |
| *C. testosteroni* ProA | 1 mg/mL | 1 hour | 873 | 90.2 |
| *C. testosteroni* ProA | 1 mg/mL | overnight | 1237 | 82.6 |

**Table 8. Effect of Aldolase Concentration on S,S Monatin Production**

| Aldolase | Concentration of Aldolase | Time | Total Monatin (ppm) | % S,S Monatin |
|---|---|---|---|---|
| SEQ ID NO:22 | 25 µg/mL | 1 hour | 11 | 25.1 |
| SEQ ID NO:22 | 25 µg/mL | overnight | 112 | 20.0 |
| SEQ ID NO:22 | 50 µg/mL | 1 hour | 18 | 31.8 |
| SEQ ID NO:22 | 50 µg/mL | overnight | 160 | 27.0 |
| SEQ ID NO:22 | 100 µg/mL | 1 hour | 33 | 33.2 |
| SEQ ID NO:22 | 100 µg/mL | overnight | 238 | 41.4 |
| *C*. *testosteroni* ProA | 25 µg/mL | 1 hour | 305 | 86.4 |
| *C. testosteroni* ProA | 25 µg/mL | overnight | 1094 | 87.5 |
| *C. testosteroni* ProA | 50 µg/mL | 1 hour | 575 | 90.9 |
| *C. testosteroni* ProA | 50 µg/mL | overnight | 1449 | 89.5 |
| *C. testosteroni* ProA | 100 µg/mL | 1 hour | 817 | 93.6 |
| *C. testosteroni* ProA | 100 µg/mL | overnight | 1360 | 89.7 |

For aldolases that are highly R-specific, such as SEQ ID NO:22, less total monatin is produced and increasing the amount of aldolase does increase total monatin (as well as % S,S). These aldolases produce less S-MP substrate, the preferred substrate for the L-aminotransferase used. For enzymes that are less R-specific, such as ProA, increasing aldolase does not significantly improve total monatin production or % S,S monatin. Increasing the amount of L-aminotransferase added decreases the percentage of S,S monatin produced.

The activity and specificity of the aldolase of SEQ ID NO:22 was further studied in two buffer systems-100 mM potassium phosphate, as above, and 100 mM 3-(N-morpholino)propanesulfonic acid ("MOPS") (with 3 mM potassium phosphate). The assays were performed as above, using 1 mg/ml AT-103 D-aminotransferase and 50 mM D-tryptophan. Experiments were run in duplicate for 4.5 hours. The aldolase of SEQ ID NO:22 produced 116 ppm monatin and 99.1 % R,R monatin in potassium phosphate (FDAA derivatization method). In MOPS, the aldolase of SEQ ID NO:22 produced 75.5 ppm monatin, and 96.2% was R,R monatin. The background levels of monatin produced in MOPS, without the aldolase of SEQ ID NO:22, were significantly higher, and the percent R,R was lower with MOPS, even in the controls. It is possible that the D-aminotransferase selectivity and activity are affected by the presence of the MOPS.

### Subcloning of SEQ ID NO:21

The aldolase gene of SEQ ID NO:21 was received from Diversa Corp. SEQ ID NO:21 was part of an environmental library which was screened by Diversa Corp. for aldolase genes. However, the aldolase gene of SEQ ID NO:21 may be reconstructed by any method known to a person of ordinary skill in the art. For example, the aldolase gene of SEQ ID NO:21 may be reconstructed utilizing assembly PCR methods, as described in Examples 10, 18 and 19.

The following primers were used to PCR amplify the aldolase gene (SEQ ID NO:21): 5'-gaggagctcgagtcagacgtatttcagtcctttttc-3' (SEQ ID NO:23) and 5'-agaagacatatgatttatcagccggggac-3' (SEQ ID NO:24). The resulting PCR product was digested with *Xho*I and *Nde*I to cut at the sites that had been engineered into the primers. The fragment was gel purified (QIAquick Gel extraction Kit (Qiagen, Velencia, CA)) and ligated (using T4 DNA ligase) with pET28b that had been digested with *Xho*I and *Nde*I and gel purified. The ligation was transformed into TOP10F' chemically competent cells. Colonies growing on the plates were screened for inserts and several isolates with inserts were submitted for DNA sequence analysis (Agencourt, Beverly, MA).

### Purification of the Aldolase of SEQ ID NO:22

Confirmed aldolase clones were transformed into either BL21 (DE3) or BL21 (DE3) pLysS. Overnight cultures grown with the appropriate antibiotic were diluted into fresh media (typically 1:100) and grown to an OD₆₀₀ ∼0.6 with aeration at 37 °C. Cultures were then induced with 1 mM isopropyl thiogalacatoside ("IPTG") and shifted to 30 °C (with aeration) and incubation was continued overnight. Cells were harvested by centrifugation. The cell pellet was typically subjected to one freeze thaw cycle to assist with cell lysis. The cell pellet was lysed in BugBuster and Benzoase (Novagen, Madison, WI) (according to the manufacturer's protocol). Cell debris was removed by centrifugation. The crude protein extract was applied to a HisBind column (Novagen, Madison, WI) that had been prepared according to the manufacturer's protocol. The column was washed and protein was eluted according to the manufacturer's protocol. The purified protein was desalted with PD-10 columns (GE Healthcare, Piscataway, NJ). The buffer used for the exchange was 50 mM potassium phosphate pH 7.5, 100 mM NaCl, 4 mM MgCl₂. Purified protein was concentrated with Amicon centrifugal concentrators (Millipore, Billerica, MA).

### Example 4

### (1) Tryptophan Racemase

R,R-monatin has been produced using D-aminotransferase and an aldolase when D-tryptophan was used as the starting material (Example 3). That notwithstanding, L-tryptophan may be a preferred starting material for several reasons. For example, L-tryptophan may be less expensive and more readily available than D-tryptophan. This disclosure describes several methods for obtaining an active tryptophan racemase. Yields of R,R monatin are improved by using an R-specific aldolase, i.e., an aldolase that preferentially or selectively produces R-MP. FIGS. 1 and 2 illustrate methods for producing stereoisomerically-enriched R,R monatin from L-tryptophan using a tryptophan racemase, a D-aminotransferase and an R-specific aldolase.

A selection for a tryptophan racemase was created by constructing a strain that requires an active racemase for growth. A tryptophan auxotroph needs a source of L-tryptophan when grown on minimal medium. Supplementing the medium with D-tryptophan is one way to select for a racemase that converts D-tryptophan to L-tryptophan. The tryptophan auxotrophs were tested for growth on minimal medium supplemented with D-tryptophan. The strains, CAG18455 and CAG18579 from the Coli Genetic Stock Center and NRRL12264 (also *lipA⁻,* λDE3lysogenized, and cured of its plasmid), did not grow when supplemented with D-tryptophan but grew when supplemented with L-tryptophan. *E. coli* may be used as a host organism but other host organisms also may used, such as yeast, other bacteria, or other eukaryotic organisms. A tryptophan auxotroph (specifically NRRL12264 (also *lipA⁻,* λDE3 lysogenized and cured of its plasmid)) will grow on D-tryptophan when it has been transformed with a D-aminotransferase. This confirms the ability of *E. coli* to transport D-tryptophan into the cell.

Salcher and Lingens described the presence of a tryptophan racemase in *Pseudomonas aurereofaciens* (ATCC15926). Salcher, O., and Lingens, F., J Gen. Microbiol. 121:465-471 (1980). Tryptophan racemase has also been described in several plants including tobacco, beets, tomato, and wheat and the enzyme appears to be induced by conditions of osmotic stress or drought. Tryptophan racemase may play a role in *Sclerochiton ilicifolius* in the native monatin production pathway. To isolate this racemase activity, an expression library is constructed from ATCC 15926 (or another organism with tryptophan racemase activity) and the library is transformed into the tryptophan auxotroph. A strain is selected that will grow using D-tryptophan as the tryptophan source. A similar method is also used to screen many strains with known racemases to look for a racemase with activity on D-tryptophan. Examples of racemases that may have activity on D-tryptophan include alanine, serine, and glutamate racemases. Yoshimura T., and Esaki, N., "Amino Acid Racemases: Functions and Mechanisms," Journal of Bioscience and Bioengineering 96, 103-109, (2003).

Alanine racemase is PLP dependent and has been cloned from Salmonella typhimurium (dadB gene). Other sources of alanine racemases are Escherichia coli, Bacillus subtilis, Pseudomonas aeruginosa, Vibrio cholerae, Schizosaccaroyces pombe, and Bacillus cereus. A basidiomycetous mushroom, Lentinus edodes, also contains a broad activity alanine racemase.

Serine racemase is also PLP dependent and is found in Eukaryotes (e.g. silkworm, rat brain, mouse brain cDNA), as well as in bacteria (*Enterococcus gallinarum*).

Glutamate racemase is PLP-independent and has been cloned from *Pediococcus pentosaceus, Bacillus pumilus, Lactobacillus fermenti, Lactobacillus brevis, E. coli, Aquifex pyrophilus,* and *Bacillus subtilis.* Some glutamate racemases are very specific and, consequently, even structurally similar amino acids aspartate, asparagine, and glutamine may not be substrates for the enzyme.

Aspartate racemases also exist and are PLP independent. Aspartate racemases are found in *Lactobacilli, Streptococcus* strains, and some archaea such as *Desulfurococcus* and *Thermococcus* strains. The bivalve mollusk *Scapharca brouhtonii* also contains an aspartate racemase.

Other racemases found in the literature include amino acid racemase (EC 5.1.1.10) from *Anabaena* sp. and *Pseudomonas striata,* proline racemase, and multifunctional phenylalanine racemase. Related epimerases or racemases are also being tested. Potential racemases are tested to make sure they are not D-tryptophan aminotransferases. The screening of potential racemases is done by sequence analysis and/or an enzyme assay. This screening method for selection of a tryptophan racemase is also used for other bacteria or archaea for which tryptophan racemase has been described, as well as for eukaryotic cDNA libraries that have been constructed in such a manor as to allow expression.

Enzymes that pass the test as a tryptophan racemase are screened for activity on monatin as described in Example 8. Ideally, one obtains an enzyme that is very specific for tryptophan and has little or no racemase activity on monatin.

A tryptophan racemase also may be evolved and/or improved (via mutagenesis or recombinant engineering) from an existing racemase, transaminase, or epimerase. Additionally, because crystal structures for alanine aminotransferases (and other aminotransferases) are known, these may be used as a basis for rational, structure based mutagenesis. The process described above is used as an initial selection for tryptophan racemase activity and as a screen for improved activity.

### (2) Tryptophan Racemase Libraries

### Construction of Libraries:

*Burkholderia pyrrocina* (ATCC15958) and *Pseudomonas chlororaphis* (ATCC15926) were obtained from the American Type Culture Collection. They were grown as recommended by ATCC and genomic DNA was prepared according to the method described in Mekalanos, J.J., "Duplication and amplification of toxin genes in Vibrio cholerae," Cell 35:253-263, (1983). The genomic DNA was partially digested with the *Sau*3AI restriction enzyme. 1-3 Kbp fragments were gel purified using a Qiagen QIAquick Gel Extraction Kit (Valencia, CA). The purified DNA was ligated into pTrc99a (Amersham, Piscataway, NJ) that had been digested with *BamHI* and purified as above. The ligation was done at room temperature with overnight incubation using a 3:1 molar ratio of insert to vector. The ligated library was transformed into TOP10F' chemically competent cells (Invitrogen, Carlsbad, CA) and plated on LB medium with 100µg/ml ampicillin. After overnight incubation of the transformation plates, colonies were scraped off of the plates, washed with liquid LB medium and an appropriate size cell pellet was mini-prepped using a Qiagen QIAquick mini-prep kit (Valencia, CA). Approximately 30,000 colonies were pooled and mini-prepped.

The pooled plasmid was transformed into CAG18455 (*trpC83*::Tn*10, rph-1*) or CAG18579 (*trpC*::Tn*10kan, rph-1*). Both strains are tryptophan auxotrophs so they will not grow on M9 minimal medium (Difco) unless the medium is supplemented with tryptophan. The transformants were plated on M9 minimal medium supplemented with D-tryptophan. This selects for a strain that can convert D-tryptophan to L-tryptophan.

Prior to transformation of the library, the strains were tested for growth on minimal medium with L- or D-tryptophan. The strains were tested for growth on minimal medium supplemented with D-tryptophan and no growth was observed. Both strains grew on identical medium supplemented with L-tryptophan instead of D-tryptophan. Additionally, a derivative of NRRL12264 (the strain used had been cured of the tryptophan operon plasmid, lysogenized with λDE3, and deleted for *lipA,* in addition to the other chromosomally encoded mutations (*serb,* Δ*trpED, tnaA2, aroP*)) was transformed with a D-specific aminotransferase from *Bacillus subtilis* (WO 03/091396). The NRRL12264 strain could not grow on minimal medium supplemented with D-tryptophan, but grew on identical medium supplemented with L-tryptophan instead of D-tryptophan. Expression of the D-aminotransferase was driven by the T7 promoter. The transformed strain was able to grow on M9 minimal medium supplemented with D-tryptophan.

The colonies that grow on the D-tryptophan medium are screened. The plasmid is isolated and retransformed into the parent strain (CAG18455 or CAG18579) to confirm that growth on D-tryptophan medium is dependent on the plasmid and not on a host mutation. The nucleotide sequence of the plasmids that complement the tryptophan auxotrophy are analyzed. Clones that are determined to contain a tryptophan racemase gene are further analyzed.

The tryptophan racemase from other tissue sources is isolated in a similar fashion. There are literature reports of tryptophan racemase activity in both tobacco tissue culture cells (*Nicotiana tabacum* L. var. Wisconsin 38) (Miura, G.A., and Mills, S.E., "The conversion of D-tryptophan to L-tryptophan in cell cultures of tobacco," Plant Physiol. 47:483-487, (1974)) and in crude protein extracts of wheat (*Triticum aestivum*) (Rekoslavskaya, N.I., et al., "Synthesis and physiological function of D-tryptophan during wheat germination," Russian J. Plant Physiol. 44:196-203, (1997)). A cDNA expression library is made from tissue, as described in the literature, and the expression library is used to transform a tryptophan auxotroph as described above.

It would be expected that if the same strains are used and the same growth conditions are reproduced as described in the literature, the enzyme with tryptophan racemase activity could be isolated or the mRNA could be isolated and a cDNA expression library could be prepared that would contain a coding sequence for an enzyme with tryptophan racemase activity. For instance, certain growth stages or certain medium components may be required to induce cellular production of an enzyme with tryptophan racemase activity.

### (3) Tryptophan Racemase Assay

Clones that are identified as potentially having a tryptophan racemase are transformed into a strain of *E. coli* commonly used for expression of recombinant proteins, such as BL21. The cells are grown in LB broth to an optical density at 600 nm of 0.4-0.6. The promoter driving expression of the racemase is induced with IPTG (0.1 mM final concentration). After induction, the cells are allowed to express the protein for 1-3 hours at 37 °C (with aeration). The cells are harvested and lysed by French press, sonication, or by chemical means (such as BugBuster (Novagen)). The lysed cells are centrifuged to remove the cell debris. The clarified extract is used directly in assays.

Varying amounts of extract is added to a solution such that the final concentration is 50 mM potassium phosphate (pH 7.0) and 2 mM L-tryptophan. Pyridoxal-5'-phosphate is added at a final concentration of 10 µM. The samples are incubated and then analyzed by LC/MS. The presence of a D-tryptophan peak when only L-tryptophan is used as a substrate indicates a positive result. D-tryptophan concentration should increase with increasing time until equilibrium is reached, and the rate should also increase with protein concentration until the concentration of enzyme is high enough that it is no longer saturated with substrate. D-tryptophan may also be converted to L-tryptophan as above.

A complementing gene may code for a D-aminotransferase. This transamination reaction requires an alpha-keto acid such as α-ketoglutarate, oxaloacetate, or pyruvate as an amino acceptor. These compounds will likely be present in a cell extract, usually in small amounts. These compounds may be removed using a PD-10 desalting column and the assay may still be performed in a crude extract. Likewise, a complementing gene may also code for a D-amino acid oxidase or D-amino acid dehydrogenase. These enzymes also require cofactors and cosubstrates that can be removed by a PD-10 desalting column. The tryptophan racemase activity is purified using conventional column chromatography. Finally, the open reading frame identified as a potential tryptophan racemase is cloned into an expression vector with an affinity tag. The potential tryptophan racemase is then purified by affinity chromatography. In either case the purified protein is used in enzyme assays essentially as described above.

### (4) Reverse Genetic Engineering of Tryptophan Racemase

The tryptophan racemase may be purified from either plant or microbial sources by conventional protein purification techniques, including ammonium sulfate fractionation and conventional column chromatography. Once the protein has been purified such that a spot can be isolated on a 2-D gel, peptide microsequencing techniques or conventional Edman type amino acid sequencing are utilized (on the internet, *see* "golgi.harvard.edu/microchem/" for descriptions of the protocols and equipment typically used for this type of work). In some cases, however, the genome sequence of the organism cannot be used as a source of the protein for the protein purification because such sequence has not been determined yet. In that situation, the first set of degenerate primers may be designed based on sequence available from the closest known relative of the protein source. Degenerate PCR and genome walking is then be performed according to established protocols to isolate the tryptophan racemase coding sequence.

### (5) Cloning of Alanine Racemase from Geobacillus stearothermophillus

The alanine racemase (SEQ ID NO:41) from *Geobacillus stearothermophillus* was cloned. Genomic DNA from *G*. *stearothermophilus* (ATCC12980D) was purchased from ATCC (Manassas, VA). The following primers were used to amplify the alanine racemase gene from *G*. *stearothermophilus:* 5'-atggacgagtttcaccgcga-3' (SEQ ID NO:25) and 5'-ttatgcatcgcttcatccgc-3' (SEQ ID NO:26). The PCR product was ligated to pCR-Blunt-TOPO using the Zero Blunt TOPO PCR cloning kit (Invitrogen, Carlsbad, CA). Correct clones were confirmed by sequencing (Agencourt, Beverly, MA). A correct clone was used as template in a subsequent PCR reaction.

The following primers were used to amplify the alanine racemase: 5'-ataataggatcctcatccgcggccaacggcg-3' (SEQ ID NO:27) and 5'-gggaaaggtaccgaggaataataaatggacgagtttcaccgcg-3' (SEQ ID NO:28). The PCR product was digested with the restriction enzymes *Kpn*I and *Bam*HI*.* These enzymes cut at sites that had been engineered into the primers. The digested PCR product was gel purified and ligated to pTrc99a that had been digested with *Kpn*I and *Bam*HI and subsequently gel purified. The ligation was transformed into TOP10F' chemically competent cells and plated on LB plated supplemented with 50 µg/ml kanamycin. Isolates were screened for inserts and several isolates with an insert were confirmed to have the correct sequence (SEQ ID NO:40) by sequence analysis (Agencourt, Beverly, MA).

The pTrc99a/alanine racemase construct was subjected to Site-Directed Mutagenesis ("SDM") using the Stratagene (La Jolla, CA) Quick-Change Multi Site-Directed Mutagenesis kit. The mutagenic primers were as follows:
5'-gccggacgacacgcacattnnkgcggtcgtgaaggcgaacgcc-3' (SEQ ID NO:29),
5'-gtgaaggcgaacgcctatggannkggggatgtgcaggtggcaagg-3' (SEQ ID NO:30),
5'-cctcccgcctggcggttgccnnkttggatgaggcgctcgctttaa-3' (SEQ ID NO:31),
5'-caaccaggcgaaaaggtgagcnnkggtgcgacgtacactgcgcag-3' (SEQ ID NO:32),
5'-gatcgggacgattccgatcggcnnkgcggacggctggctccgccg-3' (SEQ ID NO:33),
5'-gccatttggaaacgatcaacnnkgaagtgccttgcacgatcag-3' (SEQ ID NO:34)
(n = any nucleotide and k = g or t).

Residues for mutagenesis were selected by analysis of the existing crystal structure of G. *stearothermophilus* alanine racemase. Large amino acid residues located between 5 and 10 Å from the active site were chosen.

All six primers were used in the SDM reaction as directed in the manufacturer's protocol. The SDM reaction was transformed into XL-10 Gold according to the manufacturer's protocol. The transformation reaction was plated on LB medium supplemented with 100 µg/ml ampicillin. LB broth was added to the plates and the colonies were scraped off the plates. The resuspended cells were allowed to grow at 37 °C for several hours and the plasmids were mini-prepped using the QIAquick mini-prep kit. The resulting mutagenized library was then used to transform the tryptophan auxotroph CAG18455. The transformation was plated on M9 minimal medium that had been supplemented with glucose, trace elements, vitamins, 100 µg/ml ampicillin, 100 µM IPTG, and 3 mM D-tryptophan. After several days of incubation at 37 °C, colonies grew. These colonies were streaked on LB (100 µg/ml ampicillin). The plasmids were isolated from these isolates and were retransformed into CAG18455. The retransformed cells were plated on LB containing 100 µg/ml ampicillin. After isolated colonies formed, they were streaked on M9 D-tryptophan medium as described above. The colonies all seemed to re-grow, indicating that the growth was because of the mutagenized version of the racemase. No growth of the control cells was observed.

Several of the isolates were assayed for *in vitro* activity. Cells were grown to an OD₆₀₀ approximately 0.6 and induced with 100 µM IPTG. Cells were incubated at 37 °C for an additional two hours and were harvested by centrifugation. Cell pellets were stored at -80 °C until use the next day. Cell pellets were thawed on ice. Cells were disrupted with BugBuster (primary amine free) cell lysis reagent and Benzoase (Novagen). Cell debris was removed by centrifugation (∼10,000 x g for 30 minutes at 4 °C). The supernatant was saved as the crude cell extract.

Assay buffer contained 50 mM potassium phosphate (pH 8.0), 10 µM pyridoxal phosphate, 0.01% β-mercaptoethanol, and 50 mM D- or L-tryptophan. 200 µL extract was added per mL of assay. Samples were frozen representing a time 0 timepoint, as well as, 30 minute and overnight timepoints. The samples were spun, filtered, and transferred to SRC for analysis.

**Table 9: Results of Assay Starting from L-Tryptophan**

| Time (Minutes) | L-Tryptophan (ppm) | D-Tryptophan (ppm) |
|---|---|---|
| 0 | 1240 | 3.6 |
| 30 | 1193 | 24.5 |
| overnight | 1192 | 583.2 |

**Table 10: Results of Assay Starting from D-Tryptophan**

| Time (minutes) | L-Tryptophan (ppm) | D-Tryptophan (ppm) |
|---|---|---|
| 0 | 0.5 | 7506 |
| 30 | 0.5 | 7519 |
| overnight | 14.9 | 7463 |

The DNA sequence of the racemase gene in this isolate was determined (SEQ ID NO:42) and the isolate was found to have three mutations. The mutations in the corresponding protein isolate are as follows: M35C, F66E, and Y354A (SEQ ID NO:43). An additional mutation (P197L) was found in this mutant. This is a spontaneous mutation and was not part of the site-directed mutagenesis.

The mutagenized racemase was cloned into pET30 for expression and purification. The following primers were used to PCR amplify the racemase gene from the pTrc99a construct: 5'-gggaaaggtaccgaggaataataaatggacgagtttcaccgcg-3 (SEQ ID NO:35) and 5'-gcggcgccatggacgagtttcaccgcg-3' (SEQ ID NO:36). The PCR product was digested with *Nco*I and *Bam*HI*,* gel purified, and ligated to pET30 that had been digested with *Nco*I and *Bam*HI and subsequently gel purified. The ligation was transformed into TOP 10 chemically competent cells (Invitrogen, Carlsbad, CA). Isolates from the transformation were screened for inserts. Plasmids with an insert were submitted for sequencing (Agencourt, Beverly, MA). Isolates with the correct sequence were transformed into BL21 λDE3 or BL21 λDE3 pLysS for expression and purification. The new construct is designated pET30Trp racemase.

### (6) Purification of Tryptophan Racemase

An overnight culture with the pET30Trp racemase construct was subcultured into fresh LB medium with the appropriate antibiotics (50 µg/ml kanamycin and 20 µg/ml chloramphenicol) and grown to an OD₆₀₀ ∼0.6 (37 °C with aeration). Expression was induced with 100 µM IPTG and incubation was continued at 37 °C with aeration for 2 hours. The cells were harvested by centrifugation and stored at -80 °C until use. The cell pellet was thawed on ice and cells were lysed using BugBuster Primary Amine Free Cell Lysis Reagent and Benzoase Nuclease (Novagen, Madison, WI). Cell debris was removed by centrifugation and the supernatant was used as the crude protein extract. The crude protein extract was filtered using a 0.45 µm syringe filter and applied to a HisBind column (Novagen, Madison, WI) that had been pre-equilibrated according to the manufacturer's instructions. The column was washed and the protein was eluted as directed in the manufacturer's protocol. The purified protein was desalted with a PD-10 column (GE Healthcare, Piscataway, NJ) using 50 mM potassium phosphate pH 8.0, 10 µM pyridoxal-5'-phosphate ("PLP") as the eluent. The desalted protein was concentrated using Amicon centrifugal concentrators (Millipore, Billerica, MA). Wild-type alanine racemase was purified as described above.

### (7) Assay of Tryptophan Racemase

The purified racemase was tested in several assays. In one assay, the production of hydrogen peroxide by a D-amino acid oxidase was used as a detection system. D-tryptophan substrate for the oxidase was produced from L-tryptophan via the racemase enzyme isolated as described in this Example. The assay included 0, 1, 10, 25, 50, 100, 200 µg of enzyme per assay, 50 mM potassium phosphate pH 8.0, 10 µM PLP, 50 mM L-tryptophan. The assays were incubated 1 hour at 37 °C. After incubation, 100 mg/ml D-amino acid oxidase (AOD-101 BioCatalytics, Pasadena, CA) and 0.5 mM FAD was added to the reaction mix. The generation of hydrogen peroxide was measured using the Amplex Red reagent kit (Molecular Probes, Eugene, OR) and a Perkin Elmer HTS 7000 Plus BioAssay Reader Fluorometer (Wellesley, MA). The assay data is summarized in Tables 11 and 12 below:

**Table 11: Standard Curve**

| H₂O₂ Concentration (µM) | Fluorometer Reading |
|---|---|
| 0 | 485 |
| 1 | 8691 |
| 2 | 16958 |
| 3 | 24719 |
| 4 | 31692 |
| 5 | 38083 |

**Table 12: Results of Assay**

| Protein Concentration (µg/assay) | Wild-type Racemase (Fluorometer Reading) | Mutant (Trp) Racemase (Fluorometer Reading) |
|---|---|---|
| 0 | 5226 | 5192 |
| 1 | 4272 | 6215 |
| 10 | 4149 | 10543 |
| 25 | 4239 | 21177 |
| 50 | 3141 | 30465 |
| 100 | 3160 | 39068 |
| 200 | 2370 | 35163 |

The results of the assay indicate that the mutant racemase is required for production of hydrogen peroxide. The amount of hydrogen peroxide produced increased when the amount of the mutant racemase added was increased.

The activity of the racemase (wild-type and mutant) on alanine was analyzed. The reaction buffer contained: 100 mM potassium phosphate pH 8.0, 10 µM PLP, 50 mM L-alanine, 12 µg/mL wild-type racemase or 94 µg/ml mutant racemase. The reactions were stopped with 1 volume of 0.5 M formic acid and analyzed by LC/MS/MS using a Chirobiotic column as described in Example 1.

The assay data is summarized in Table 13 below.

**Table 13**

| Time (minutes) | Wild-type Racemase (ppm D-Alanine Produced) | Mutant Racemase (ppm D-Alanine Produced) |
|---|---|---|
| 0 | 65 | 87 |
| 5 | 334 | 2430 |
| 10 | 1161 | 3257 |
| 20 | 1670 | 4003 |
| 30 | 3075 | 4621 |
| 40 | 3177 | 4931 |
| 60 | 3986 | 5328 |

The mutated racemase appears to retain activity on the original substrate, alanine.

The activity of the mutated racemase was tested using one of L-tryptophan, D-tryptophan, L-alanine, and D-alanine as the substrate. The reaction buffer contained: 100 mM potassium phosphate pH 8.0, 10 µM PLP, 50 mM substrate, 94 µg/ml mutant racemase. The reactions were stopped with 1 volume of 0.5 M formic acid and analyzed as described in Example 1. The assays with alanine as the substrate were incubated at room temperature (~22 °C) and assays with tryptophan as the substrate were incubated at 37°C. The results are summarized in Table 14 below.

**Table 14**

| Time (minutes) | ppm D-trp Produced from L-trp | ppm L-trp Produced from D-trp | ppm D-ala Produced from L-ala | ppm L-ala Produced from D-ala |
|---|---|---|---|---|
| 0 | None detected | 0.8 | 420.5 | 565.9 |
| 5 | None detected | 1 | 1268 | 1874 |
| 10 | None detected | 1.4 | 1448 | 1968 |
| 20 | None detected | 2.2 | 1590 | 1505 |
| 30 | 0.3 | 2.8 | 1840 | 1923 |
| 40 | 3.1 | 2.8 | 1779 | 1960 |
| 60 | 9 | 3.7 | 1295 | 1070 |
| 1080 | 57.4 | 66.7 | 1611 | 2932 |

The racemase enzyme works in both directions and retains wild-type activity.

The mutant racemase was tested on several substrates. The enzyme used in the assay was purified as previously discussed. The assay conditions are as follows:
50 mM potassium phosphate pH 8.0, 10 µM PLP, 25 mM substrate, 40 µg/ml mutant racemase. The reactions were stopped with 1 volume of 2 M formic acid and analyzed as described in Example 1. The assays were incubated at 37 °C. The results (in ppm D-isomer produced from the L-isomer) are summarized in Table 15 below (nd = none detected).

**Table 15**

| Time (Minutes) | Lys | Ala | Glu | Met | Tyr | Leu | Trp | Phe |
|---|---|---|---|---|---|---|---|---|
| 0 | 12 | 156 | 86 | 104 | nd | nd | nd | nd |
| 3 | 2310 | 2180 | 607 | 1200 | nd | 37 | nd | nd |
| 5 | 2450 | 1310 | 1110 | 1290 | nd | 80 | nd | 14 |
| 10 | 6630 | 2850 | 1950 | 2260 | 11 | 139 | nd | 14 |
| 20 | 9550 | 1970 | 4660 | 2090 | 30 | 280 | nd | 47 |
| 30 | 15500 | 2090 | 4860 | 1750 | 63 | 320 | nd | 22 |
| 60 | 10200 | 2540 | 4490 | 2150 | 136 | 710 | nd | 54 |
| 120 | 18000 | 2430 | 6340 | 1940 | 224 | 1050 | nd | 188 |
| 240 | 13200 | 1830 | 6560 | 1990 | 515 | 1170 | 15 | 490 |

It is likely that this racemase will racemize other amino acids in addition to the ones tested here.

Although the mutated racemase appears to have activity on a wide variety of amino acids, there does not appear to be any racemase activity on monatin. The enzyme used in the assay was purified as previously discussed. The assay conditions are as follows: 100 mM potassium phosphate pH 8.0, 10 µM PLP, 50 mM monatin, 1 mg/ml mutant racemase. The assays were incubated at 37 °C. The assays were analyzed by FDAA derivitization as described in Example 1. The results of the assay are shown in Table 16 below.

**Table 16**

| Time (Hours) | S,S Monatin Starting Substrate | R,R Monatin Starting Substrate |
|---|---|---|
| 0 | 100% SS | 100% RR |
| 1 | 100% SS | 100% RR |
| 18 | 100% SS | 100% RR |

Even after 18 hours there was no apparent conversion of S,S monatin to S,R monatin or of R,R monatin to R,S monatin using the mutant racemase.

The ideal enzyme has activity on tryptophan, but little or no activity on other amino acids or amino acid like compounds, particularly monatin. If the enzyme has significant activity on monatin, the enzyme may be mutagenized to decrease the activity on monatin and/or glutamate, while keeping the tryptophan activity unchanged or at a level high enough for the enzyme to be useful in monatin production. Techniques that may be used for mutagenesis include, but are not limited to, error prone PCR, site-directed mutagenesis, modeling to identify site-directed mutagenesis targets (sites that may be involved in substrate binding), passage through mutagenic strains, and DNA shuffling.

### (8) Tryptophan Racemase Monatin Production

The following were added per 1 mL of reaction mixture: approximately 50 µg aldolase of SEQ ID NO:22, 16 mg/mL purified tryptophan racemase, 4 mM MgCl₂, 50 mM L-tryptophan, 0.5 mg D-aminotransferase (purified from *Bacillus sphaericus* as described in Example 14), 100 mM sodium pyruvate, 100 mM potassium phosphate buffer pH 7.5, and 0.05 mM PLP. Because pyruvate is an acceptable amino acceptor for the broad specificity D-aminotransferase, α-ketoglutarate was not used. A control was included in which D-tryptophan was the starting substrate and no racemase was included. The samples were incubated 2 hours or overnight (20 hours) at 30 °C with gentle shaking. Samples were analyzed as described in Example 1. The results of the assay are shown below in Table 17 (nd = none detected).

**Table 17**

| Time (Hours) | Starting Substrate | ppm Total Monatin | RR/SS% RPLC | RS/SR% RPLC | % RR FDAA | %SR FDAA |
|---|---|---|---|---|---|---|
| 2 | L-trp | nd | 0 | 0 | | |
| 18 | L-trp | 7.4 | 100 | 0 | 96.5 | 3.5 |
| 2 | D-trp | 12 | 99.17 | 0.83 | | |
| 18 | D-trp | 170 | 98.65 | 1.35 | 97.5 | 2.5 |

Table 17 shows production of R,R monatin using a tryptophan racemase to convert the L-tryptophan substrate to D-tryptophan. The production of R,R monatin from D-tryptophan, without using the tryptophan racemase, was utilized as a control. The percent R,R monatin produced is nearly the same with either L- or D-tryptophan as the starting material. This result indicates the racemase does not have detectable activity in catalyzing the racemization of R,R monatin.

### (9) Isolation of the Key Amino Acid Changes

Several revertants of the mutagenized alanine racemase were created. The revertants were made by site-directed mutagenesis using the QuikChange Multi Site-Directed Mutagenesis Kit (Stratagene, La Jolla, CA) as previously described using the following primers:
5'-gccatttggaaacgatcaactatgaagtgccttgcacgatcag-3' (SEQ ID NO:37)
5'-ctcccgcctggcggttgccttcttggatgaggcgctcgctttaag-3' (SEQ ID NO:38)
5'gccggacgacacgcacattatggcggtcgtgaaggcgaacgcc-3' (SEQ ID NO:39)

The primers were used individually, and in combination, in an attempt to make the six possible combinations of the three mutations in positions 35, 66, and 354 (numbering based on the ATCC 12980 derived amino acid sequence). Several combinations of the mutations were created and tested for tryptophan racemase activity. The assay conditions were as follows: 50 mM potassium phosphate pH 8.0, 10 µM PLP, 30 mM L-Tryptophan, 100 µg/ml enzyme. The assays were incubated at 37 °C for the specified timeperiod. The samples were analyzed as described in Example 1.

The results of the assays are summarized in Table 18 below (nd = none detected).

**Table 18**

| Time (Minutes) | MF1 | MF2 | MY1 | Mutated Racemase |
|---|---|---|---|---|
| 0 | nd | nd | nd | nd |
| 5 | nd | nd | nd | nd |
| 10 | nd | nd | nd | nd |
| 20 | nd | nd | nd | nd |
| 30 | nd | nd | nd | nd |
| 40 | nd | nd | nd | nd |
| 60 | 9.8 | nd | nd | 12.5 |
| 1080 | 54.8 | 90.8 | nd | 92.4 |

Mutation list:
MF1: N41S (spontaneous mutation), P197L, Y354A
MF2: F66E, P197L, Y354A
MY1: M35C, F66E, P197L
Mutagenized racemase: M35C, F66E, P197L, Y354A

The results indicate that the Y354A mutation is required for activity on tryptophan. When this mutation was absent there was no detectable activity on tryptophan.

An alanine racemase may be further converted to a broader specificity racemase by random methods such as mutagenic PCR, passage through mutagenic strains, or other methods to those known in the art. A more focused evolution of the alanine racemase may be focused on active site residues, including Lys129, Met134, and the residues including and between Gly283 and Trp288 (numbering from *Geobacillus stearothermophilus).*

### Example 5

### Selection Method for Screening of Pyruvate Aldolases in Recombinant E. coli

Many of the processes described in Examples 4*(5)*, 9 and 10*(3)*, and shown in FIGS. 1-9, will work optimally with an aldolase that preferentially produces R-MP from indole-3-pyruvate and pyruvate. Therefore, methods are described to isolate and test clones containing nucleic acid encoding an aldolase that preferentially produces R-MP. Strains of *Escherichia coli* that require pyruvate supplementation when grown on M9 minimal medium with ribose as the carbon source have been described previously. Ponce, E., et al., "Cloning of the two pyruvate kinase isoenzymes structural genes from Escherichia coli.: The relative roles of these enzymes in pyruvate biosynthesis," J. Bacteriol. 177:5719-5722, (1995). The relevant genotype of the strain is: Δ*pykA* Δ*pykF.* The double knockout was generated by the method of Datsenko and Wanner, Proceed. Natl. Acad. Sci. USA 97:6640-6645, (2000). These strains can form a basis for a pyruvate-generating aldolase screen and to screen for aldolases that are more active on a specific stereoisomer of monatin, a particular stereoisomer of monatin precursor, or an analog of monatin or monatin precursor. An analog of monatin precursor includes compounds that have been identified as substrates for ProA aldolases or KHG aldolases, such as 4-hydroxy-4-methyl-2-oxoglutarate, 4-carboxy-4-hydroxy-2-oxoadipate, 4-hydroxy-4-methyl-2-oxoadipate, or other carboxyl rich compounds that are converted to pyruvate in an aldol reaction. An example of an analog of monatin that can be used is 4-hydroxy-4-methyl glutamic acid, which can be easily transaminated to 4-hydroxy-4-methyl-2-oxoglutarate (a substrate of ProA) by native aminotransferases in a test cell.

### Cloning

The following primers were used to generate the *pykA* knockout:

The following primers were used to generate the *pykF* knockout:

A PCR reaction was performed with either pKD3 or pKD4 as template using standard protocols. The PCR product was electroporated into a strain of *E. coli* that expresses the lambda red homologous recombination system. The PCR product had homology to *pykA* or *pykF* and recombined into the chromosome at those sites. When the double crossover occurred, the resulting progeny carried a deleted *pykA* or *pykF* gene and an antibiotic resistance marker. The deleted genes with the antibiotic resistance markers were transduced into an *E. coli* strain (MG1655) using standard P1 transduction techniques.

### Strain Analyses

The double knockout was tested for growth on minimal medium (M9 salts) (Difco) supplemented with Balch's vitamin solution, Balch's modified trace element solution (Balch, W.E., et al., "Methanogens: reevaluation of a unique biological group," Microbiol. Rev. 43:260-296, (1979)), and 0.4% D-ribose. No growth was seen for the double mutant unless 5 mM pyruvate was also included in the media. Wild-type MG1655 grew on the above media both in the presence and absence of pyruvate. The double knockout was tested for growth on the minimal medium described above supplemented with 0.4% glucose rather than ribose. Growth on this medium was similar to that seen with the wild-type strain. With this medium, pyruvate can be generated from glucose *via* the *ptsI* gene product (the enzyme of the phosphotransferase system that makes pyruvate from phosphoenolpyruvate and transfers the phosphate to glucose). The double knockout strain was also tested for growth using the medium as described above supplemented with 0.4% L-arabinose or 0.4% D-xylose rather than ribose. Pyruvate is not generated from growth on these 5-carbon containing (non-PTS) substrates. The double knockout did not grow under these conditions unless it was supplemented with 5 mM pyruvate, while the wild-type strain grew normally both in the presence and absence of pyruvate.

The *proA* aldolase gene from *Comamonas testosteroni* described in Example 2 of WO 03/091396 A2 (cloned in pET30 Xa/LIC) and the *aspC*/*proA* gene operon described in Example 3 of WO 03/091396 A2 (cloned in pET30 Xa/LIC and pET32) were sub-cloned into pBAD-TOPO using the pBAD TOPO TA expression kit (Invitrogen).

Expression of the gene(s), in these constructs, is regulated by the inducible *araBAD* promoter. In the presence of arabinose (for example 0.4%) and IPTG, the gene(s) are expressed. Unless supplemented with pyruvate or a source of pyruvate, the strain will not grow on minimal medium. The medium can be supplemented with monatin, monatin precursor, or an analog of monatin or monatin precursor. Typical ranges of substrate used in literature are 0.5-5 mM. The ProA aldolase can, for example, convert the monatin precursor into pyruvate and indole-3-pyruvate providing the strain a source of pyruvate and allowing growth on minimal medium with 0.4% arabinose. The construct expressing both the *proA* and the *aspC* genes can convert monatin into the monatin precursor and the monatin precursor into pyruvate and indole-3-pyruvate. Additionally, the aminotransferase can convert indole-3-pyruvate to L-tryptophan and complement a tryptophan auxotrophy. This system is used to screen for aldolases and to screen for aldolases that are more active on a specific stereoisomer of monatin, a specific stereoisomer of monatin precursor, or an analog of monatin or monatin precursor. For example, if directed evolution is performed on any of the aldolases disclosed in Example 2 of WO 03/091396 A2, a plate assay utilizing media containing either R or S monatin precursor is used to compare the enantiospecificity of the resulting mutant enzyme. If growth occurs on the plates containing R-monatin precursor and little or no growth occurs on the plate containing S-monatin precursor, the aldolase has specificity for substrates containing the R-chirality at the reaction site.

M9 minimal medium plates were made containing 1x Balch's vitamin solution and Balch's modified trace element solution. Balch, W.E., et al., "Methanogens: reevaluation of a unique biological group." Microbiol. Rev. 43:260-296, (1979). Glucose or arabinose was included as the carbon source (0.4% w/v) and plates were supplemented with either 5 mM monatin (R,R; S,S racemic mixture) that had been dissolved in 20 mM potassium phosphate buffer (pH 8.0) or an equal volume of potassium phosphate buffer without monatin. Growth is summarized in Table 20 below.

**TABLE 20**

| | **Glucose** | **Glucose monatin** | **Arabinose** | **Arabinose monatin** |
|---|---|---|---|---|
| MG1655 | ++++ | ++++ | ++++ | ++++ |
| MG1655 Δ*pykA* Δ*pykF* | ++++ | ++++ | + | + |
| MG1655 Δ*pykA* Δ*pykF*+ *aspCproA*/pBAD-TOPO | ++++ | ++++ | + | ++ |

It is expected that the screen could be optimized by controlling the levels of ProA and AspC, increasing uptake of monatin, using monatin precursor in the place of monatin (in this case the aminotransferase would not need to be present), or using a less hydrophobic analog of monatin such as those described above. Methods for increasing the uptake of monatin include addition of amino acid mixtures, addition of specific amino acids, and the use of detergents, antibiotics, antibiotic analogs, or enzymes that help to permeabilize the cell wall, and addition of a small amount of pyruvate to allow for growth in case the aldolase cannot provide enough pyruvate to support growth. Polymyxin B nonapeptide (Dixon and Chopra, Antimicrobial Agents and Chemotherapy 29:781-788 (1986)) and microcystin RR (Dixon, et al., FEMS Microbiology Letters 230:167-170 (2004)) have been described as agents that permeabilize the outer membrane of *E. coli.*

It is expected that other promoter systems/plasmids can be used in this screening system with equivalent results. Examples include T7 promoter systems, and IPTG inducible promoters such as *tac* and *lac.*

The *aspC* and the *proA* genes were cloned into the pTrc99a expression vector (Amersham, Piscataway, NJ). The resulting vector was transformed into the tryptophan auxotrophs CAG18455 or CAG18579 *(see* Example 4 for strain descriptions). The transformants were plated on M9 minimal medium with 0.1 mM IPTG and 5 mM monatin. After 3 days at 37 °C, the strains with the operon plasmids formed colonies, while the parent strains did not appear to grow. Additionally, the growth was dependent on the presence of IPTG indicating that expression of the operon was required for growth. In this complementation study, the *aspC*/*proA* operon formed MP from monatin and indole-3-pyruvate from MP. The indole-3-pyruvate could then be converted to L-tryptophan allowing the tryptophan auxotrophs to grow on M9 minimal medium.

Several potential organisms may have the R-specific aldolase and can be tested as described above. The presence of R,R-monatin has been detected in culture supernatants of *Corynebacterium glutamicum.* This suggests the presence of an enzyme that is capable of making the R-monatin precursor. Additionally, the presence of multiple isomers of monatin has been detected in cell free extracts of *Sinorhizobium meloti* using reversed phase chromatography, again indicating the possible presence of an aldolase or aminotransferase capable of making an R stereoisomer of monatin precursor.

*Pseudomonas straminea (Pseudomonas ochraceae* NGJI), *Sinorhizobium meliloti, Sphingomonas sp.* LB126, *Arthrobacter keyseri* 12B, *Yersinia pestis* strain CO92, *Bradyrhizobium japonicum* str. USDA 110, *Sphingomonas (Pseudomonas) paucimobilis, Yersinia pestis* KIM, *Ralstonia metallidurans* CH34, *Yersinia pseudotuberculosis* IP 32953, *Rhizobium leguminosarum* biovar viciae rhiz23g02-p1k_1009_341 (Sanger Institute), *Novosphingobium aromaticivorans* DSM 12444, *Pseudomonas putida* KT2440, *Magnetospirillum magnetotacticum* MS-1, *Rhodopseudomonas palustris* CGA009, *Xanthomonas campestris* ATCC-33913, *Xanthomonas axonopodis* citri 306, and *Streptomyces avermitilis* MA-4680 have homologs that have been discovered by BLAST analysis using *proA (Comamonas testosteroni)* as the template. *See* U.S. Application No. 20050282260 These organisms can be used as a source of DNA and tested in the screen mentioned above.

Organisms capable of growth on gallic acid, syringic acid, protocatechuate, phtalate, parahydroxybenzoate, and fluorene may have an aldolase that may make monatin and have potential for the screen mentioned above. The following organisms metabolize protocatechuate via the 4,5-dioxygenase pathway and may have an aldolase that may be of utility: *Bordetella bronchiseptica* RB50, *Bordetella parapertussis* 12822, *Klebsiella pneumoniae* MGH78578, *Magnetospirillum magnetotacticum* MS-1, *Rhodopseudomonas palustris* CGA009, *Sphingomonas aromaticivorans* F199, *Xanthomonas axonopodis* citri 306, *Xanthomonas campestris* ATCC 33913.

And the following organisms degrade protocatechuate via the 3,4 dioxygenase pathway and have an aldolase that may be of utility: *Acinetobacter calcoaceticus* ADP1, *Acinetobacter* species ATCC 33305, ADP1, *Agrobacterium tumefaciens* C58, *Azotobacter vinelandii* AvOP, *Bradyrhizobium japonicum* str. USDA 110, *Bradyrhizobium japonicum* tr. USDA 438, *Brucella abortus, Brucella melitensis* 16M, *Brucella melitensis* suis 1330, *Burkholderia cepacia* J2315, *Burkholderia fungorum* LB400, *Burkholderia pseudomallei* K96243, *Corynebacterium efficiens* YS-314, *Corynebacterium glutamicum* ATCC-13032, *Mesorhizobium loti* MAFF303099, *Mycobacterium avium* subsp. paratuberculosis str. k10, *Pseudomonas aeruginosa* PAO1, *Pseudomonas fluorescens* Pf0-1, *Pseudomonas fluorescens* SBW25, *Pseudomonas putida* KT2440, *Pseudomonas syringae* pv. tomato str. DC3000, *Ralstonia solanacearum, Rhodococcus sp.* strain I24 (IG-15), *Sinorhizobium meliloti* 1021, *Streptomyces avermitilis* MA-4680, *Streptomyces coelicolor* A3 (2), *Xanthomonas axonopodis* citri 306, *Xanthomonas campestris* ATCC-33913.

### Example 6

### Site Directed Mutagenesis of HEXAspC

### Experimental Overview

A hexamutant of *E. coli* AspC *(HEXaspC)* was found to have better activity as compared to AspC for the production of S,S monatin, as described in Example 6 of WO 03/091396 A2. HEX (accession number:/AHFA gi:127190) contains the following mutations from AspC (*E. coli* numbering): V35L, K37Y, T43I, N64L, T104S, and N285S. Based on structural analysis and literature reports (Rothman, S., and Kirsch, J., J. Mol. Biol. 327:593-608, (2003); Rothman, S., et al., Protein Science 13:763-772, (2004)), 5 more mutants were created that were expected to increase the kinetic activity toward substrates utilized in the monatin production pathway: L-tryptophan, S-MP, or both. Two of the mutants increased transamination rates for both tryptophan and S,S monatin. Two of the mutants showed an increased stereoselectivity for the formation of S,S monatin while one was less stereoselective. Based on this, it is expected that a broad specificity D-aminotransferase from *Bacillus sp.* with similar mutations would be useful as the D-aminotransferase in the R,R monatin pathways shown in FIG. 3, and described in Example 4*(4)*. One of the mutants (HEXaspCP9T/R122G) had increased activity for L-tryptophan transamination, but activity in S,S monatin production or S,S monatin transamination was decreased significantly. Thus, it is expected that this enzyme is useful in the first step of the R,R monatin production pathways shown in FIGS. 1, 2, 4, 5, 6, 7, and 8 and described in Examples 9 and 10*(3)* In general, an aminotransferase that has activity similar to that of AspC on L-tryptophan, and limited activity on R-MP and S-MP, would be useful for the processes depicted in FIGS. 1, 2, 4, 5, 6, 7, and 8.

### Methods and Materials

The HEX gene cloned in pUC19 was provided by Professor J. F. Kirsch (Department of Molecular and Cell Biology, University of California, Berkeley, Berkeley, CA 94720-3206) and used as the template for the cloning of the gene into pET23a. *See* Onuffer, J.J., and Kirsch, J.F., "Redesign of the substrate specificity of Escherichia coli aspartate aminotransferase to that of Escherichia coli tyrosine aminotransferase by homology modeling and site-directed mutagenesis," Protein Science 4:1750-1757 (1995). *See also* NCBI accession number 1AHF_A GI:1127190 (HEX amino acid sequence). The following primers were designed for cloning the HEX gene into the pET23a vector (Novagen, Madison, WI): *HEXaspC* primers:
N term: 5'-GCGGAACATATGTTTGAGAACATTACCGCC-3'(SEQ ID NO:7);
C term: 5'-ATAACCGGATCCTTACAGCACTGCCACAATCG-3' (SEQ ID NO:8).

The following PCR protocol was used for gene amplification: In a 100 µL reaction, 50 ng DNA template, 1.0 µM of each primer, 0.2 mM each dNTP, 1 U *Pfu* Turbo Polymerase (Stratagene; LaJolla, CA), and 1X Cloned *Pfu* buffer were added. The thermocycler program utilized a hot start of 94 °C for 5 minutes; followed by 25 cycles of a denaturing step at 94 °C (30 sec), an annealing step at 55 °C (1 min), an extension step at 72 °C (2 min), and finally a finishing step at 72 °C (7 min). The purified PCR product was digested with *Bam*HI and *Nde*I (New England Biolabs) restriction enzymes. The PCR product was ligated into pET23a that was also digested with *Nde*I and *Bam*HI*,* using the Roche Rapid DNA Ligation kit. The desalted ligations were electroporated into *E. coli* DH10B cells using a Bio-Rad Gene Pulser II system, according to manufacturer's protocols. Miniprep DNA was prepared using a Qiagen Spin Miniprep kit and was used as a template for mutagenesis reactions. The plasmid was transformed into *E coli* BL21 (DE3) cells according to the manufacturer's protocols (Novagen).

The tryptophan residue at position 130 is thought to be important for stacking interactions with the pyridoxyl ring, but also appears to be a source of steric hindrance with the S-monatin precursor ("S-MP") substrate, based on protein modeling observations. Therefore, an amino acid with a smaller hydrophobic side chain (phenylalanine) was used to replace the tryptophan. The rest of the mutations were based on kinetics data in literature, although new combinations of desirable mutations were created. All mutations to HEXaspC, with the exception of W130F, were made using the Stratagene Multi-Change kit by following the manufacturer's instructions. The W130F mutation was made using the Stratagene QuikChange kit according to the manufacturer's instructions with the only exception being that the extension temperature for the PCR reaction was decreased to 66 °C. The primers for the multi-change kit were designed using the QuikChange multi-kit primer design tool on <www.stratagene.com>, except for the W130F single mutation primers.

The primer sequences are listed in Table 21 below:

**Table 21**

| Primer | Sequence (5' to 3') |
|---|---|
| aspCW130F_backward | CGCTCTTATGGTTCGGTTTGCTTGGGTTGCTCACCC (SEQ ID NO:9) |
| aspCW130F_forward | GGGTGAGCAACCCAAGCTTTCCGAACCATAAGAGCG (SEQ ID NO: 10) |
| R122G-1*^{α}* | CAAAAAATACCAGCGTTAAGGGAGTGTGGGTGAGCAACC (SEQ ID NO:11) |
| P9T_4*^{α}* | CATTACCGCCGCTACTGCCGACCCGATTC (SEQ ID NO: 12) |
| I68V-1 *^{α}* | CACCAAAAATTACCTCGGCGTAGACGGCATCCCTGAATT (SEQ ID NO: 13) |
| T156A*^{α}* | TGATGCGGAAAATCACGCTCTTGACTTCGATGCAC (SEQ ID NO: 14) |

| | |
|---|---|
| *^{α}* Denotes a primer that was modified by 5' phosphorylation | |

### Expression of HEXaspC Mutant Genes and Analysis of Enzyme Activity

Liquid cultures (5 mL) of Novagen Overnight Express™ Autoinduction System 2 (Catalog # 71366-3; solutions 1-6) were inoculated from fresh plates or frozen glycerol stocks of the following strains:
*E. coli* BL21(DE3)::HEXaspCpET23a
*E. coli* BL21(DE3)::HEXaspCW130FpET23a
*E. coli* BL21(DE3)::HEXaspCT156ApET23a
*E. coli* BL21(DE3)::HEXaspCP9T/T156ApET23a
*E. coli* BL21(DE3)::HEXaspCP9T/R122GpET23a
*E. coli* BL21(DE3)::HEXaspCR122G/T156ApET23a

The cultures were incubated at 37 °C at 230 rpm for 6 - 8 h. The OD₆₀₀ of each culture was determined and the volume of culture necessary to obtain an OD₆₀₀ of 0.03-0.05 in 25 mL was calculated. The calculated volumes of each liquid culture were transferred to flasks containing 25 mL of the same medium. The Overnight Express™ Autoinduction System 2 is a complete, chemically defined medium for high-level expression with IPTG-inducible expression systems that uses lactose as the inducing agent and does not require monitoring of cell growth. The Overnight Express cultures were incubated at 30 °C with shaking at 230 rpm for 18 h. The cells were harvested by centrifugation and washed once with cold 50 mM MOPS, pH 7.0. The cells were then lysed using Bugbuster™ (primary amine free) Extraction Reagent (Novagen Catalog #70923-3) containing 1 µL/mL benzonase nuclease (Novagen Catalog #70746-3), 5 µL/mL Protease Inhibitor Cocktail Set II (Novagen Catalog #539132) and 0.33 µL/10 mL r-Lysozyme (Novagen Catalog #71110-3) following the Novagen recommended protocol. After incubation at 25 °C for 15 min with gentle shaking, the cell debris from each suspension was pelleted by centrifugation at 21,000g for 15 min at 4 °C. The supernatant was carefully decanted and analyzed as the cell free extract. Inclusion body fractions were isolated by suspending the cell debris fractions in 30% Bugbuster™ (primary amine free) Extraction Reagent, centrifuging at 21,000 x g for 10 min; suspending the centrifuged pellets in 10% Bugbuster™ (primary amine free) Extraction Reagent, centrifuging again to isolate the washed pellets.

The cell free extracts and inclusion body fractions were analyzed for protein expression by SDS-PAGE on 4-15% gradient gels (Bio-Rad # 161-1104). For the cell extract samples, twenty micrograms of soluble protein were loaded in each gel lane (premixed with 1X protein loading buffer and heated at 95 °C for 5 min). The inclusion body fractions were dissolved in 1X protein loading buffer (0.2 mL), heated for 10 min at 95 °C and 5 µL of each solution was loaded per gel lane. The amount of each HEX mutant in comparison to the total soluble protein loaded into each lane was calculated by band intensity analysis using Labworks Biolmaging 1D-gel tool (UVP, Inc. Upland, CA), and is reported in Table 22 below:

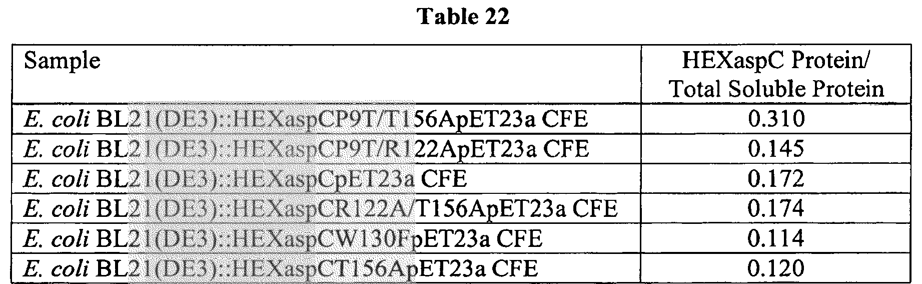

Analysis of the gels showed that the HEXaspCR122A/T156A mutant was the only protein that was found in substantial quantities as inclusion bodies. The HEXaspCP9T/T156A protein gave the highest level of expression, approximately 90% better than HEXaspC protein. In contrast, the W130F, T156A and P9T/R122G proteins were expressed in lower concentrations than HEXaspC.

The activity of the HEXaspC mutant proteins for the production of S,S-monatin was measured using the following reaction conditions: Each 1 mL reaction contained 50 mM TAPS, pH 8.2, 4 mM MgCl₂, 3 mM sodium phosphate, pH 8.0, 200 mM sodium pyruvate (pH adjusted to 8), 5 mM α-ketoglutarate (pH adjusted to 8), 50 mM tryptophan, 0.05 mM pyridoxal 3-phosphate, 50 µg/mL ProA aldolase (added as a cell free extract) and varying concentrations (approximately 50 and 500 µg/mL) of aminotransferase (added as a cell free extract). Deaerated water was used to prepare the stock solutions and to adjust the volume of the reaction mixtures to 1.0 mL. The pyridoxal phosphate was added just prior to the addition of the enzymes. The reaction tubes were incubated at 30° C with gentle shaking for 4 h. Samples (0.01 mL) were withdrawn at 1, 2, and 4 h after the addition of the enzymes, filtered, and analyzed by LC/MS/MS, as described in Example 1. Monatin production was normalized based on the amount of aminotransferase present in the reactions.

Under the conditions of these assays, the HEXaspC and the HEXaspCT156A produced the most total monatin per mg of aminotransferase while the P9T/R122G protein produced the least, followed by HEXaspCW130F. The HEXaspCW130F and P9T/R122G enzymes showed the greatest stereoselectivity for S-MP (greater than 98% S,S-monatin), even when high enzyme concentrations were used (greater than 300 µg/mL). The percentage of S,S-monatin product decreased to less than 90% in the enzymatic reactions containing the P9T/T156A enzyme at high concentration. The other mutants showed a product stereoselectivity very similar to the original HEXaspC mutant (approximately 95% S,S-monatin). Analysis of the product of the reaction containing the HEXaspC enzyme using the FDAA derivitazation reagent described in Example 1 showed that the second stereoisomer formed is R,S-monatin.

### Assaying of Tryptophan and Monatin Aminotransferase Activity

The mutants were tested for transamination activity using S,S monatin and L-tryptophan as substrates. The aminotransferase activity was measured by following the formation of the co-product of the reaction, glutamate, by HPLC with OPA-post-column derivitization as described in Example 1. The reaction mixture contained, in 1.0 mL, 100 mM HEPPS buffer, pH 8.0, 20 mM alpha-ketoglutarate, 0.08 mM pyridoxal phosphate, 25 mM tryptophan or S,S monatin, and enzyme (supplied as 2.5 mg of in cellular extracts protein). All components except the enzyme were mixed together. The enzyme was added to start the reaction and the reaction solution was incubated at 30 °C (gentle shaking) for 90 min. Reactions were done in duplicate, with negative controls in which no enzyme was added. The reaction was stopped by the addition of 10% formic acid (final concentration), the mixture was centrifuged at 21,000 rpm, and the supernatant was carefully removed and filtered. The data were corrected for background levels of glutamate and for the dilution from the addition of acid to precipitate the proteins, then normalized by amount of mutant aminotransferase added. When tryptophan was utilized as a substrate, HEXaspC produced 13.0 mM glutamate per mg of aminotransferase per hour. The relative activity, expressed as a percentage, of the mutants is as follows: HEXaspCW130F (156%), HEXaspCT156A (151%), HEXaspCP9T/T156A (63.7%), HEXaspCP9T/R122G (116%), and HEXaspCR122G/T156A (107%). When S,S monatin was utilized as a substrate, HEXaspC produced 7.43 mM glutamate per mg of aminotransferase per hour. The relative activity, expressed as a percentage, of the mutants is as follows: HEXaspCW130F (113%), HEXaspCT156A (87.7%), HEXaspCP9T/T156A (67.3%), HEXaspCP9T/R122G (11.2%), and HEXaspCR122G/T156A (114%).

The HEXaspCP9T/R122G mutant had increased activity for tryptophan to indole-3-pyruvate conversion, but decreased activity for S,S monatin transamination. The ratio of tryptophan to monatin activity was 18.2 in comparison to 1.75 for HEXaspC, making it a desirable candidate for production of R,R monatin using pathways that require an L-aminotransferase, such as those described in Examples 9 and 10*(2).* As such, the HEXaspCP9T/R122G is an example of an aminotransferase with limited activity on S,S monatin, as well as, MP.

Most of the mutations improved L-tryptophan activity, but only two mutants increase activity toward both L-tryptophan and S,S monatin (HEXaspCW130F and HEXaspCR122G/T156A). Because 25 mM of substrate was used in these assays, the enzymes were most likely saturated and the activity is a reflection of the k_{cat} of the enzymes. However, under the conditions in which the assays for S,S monatin production were performed, described above, it is unlikely that the concentration of S-MP is sufficient to saturate the enzyme, thus there is no overall increase in S,S monatin production because the increase in k_{cat} is offset by an increase in Kₘ. It has been reported, for similar substrates, that some of the mutations made increase the k_{cat} but also increase the apparent Kₘ for the amino acid substrate. If increasing concentrations of substrates were used, it is expected that these two mutants would provide a benefit in production rates of S,S monatin in comparison to HEXaspC. The HEXaspCTl56A mutation appears to have increased tryptophan transamination rates without having a significant effect on MP transamination rate under the conditions above for S,S monatin production.

By comparison of the structures of HEXaspC and one of the *Bacillus* sp. D-aminotransferase enzymes (see, for example, Sugio, S, et al., Biochemistry 34:9661-9669, (1995)), the W130F, R122G, T156A, and HEX mutations of AspC could be mapped to corresponding residues in the D-aminotransferase structure. It is expected that similar mutations in the broad specificity D-aminotransferase would improve the overall production of R,R monatin, as described in Example 3. For example, the functionality provided by tryptophan 130 in AspC is replaced in *Bacillus* D-aminotransferases by hydrogen bonding between the side chains of serines 179-181 and glutamate 166 (YM-1 numbering scheme). To lessen steric hindrance, the glutamate could be mutated to an aspartate residue. Some D-aminotransferases have a threonine residue at position 179, which would increase steric hindrance and should be avoided. The *B. sphaericus* enzyme has an alanine in place of serine at position 181, which may also reduce steric hindrance.

Additional information from studies of aspartate aminotransferase can be applied to the D-aminotransferase as well. While the AspC enzyme has an arginine in the active site that interacts with the side chain of dicarboxylate substrates, the D-aminotransferase has a loop from Ser240 to Ser243. The side chains of Ser240, Thr242, and Ser243 face the same direction and form a pocket with the hydroxyl group of Ser180 which provides a surface for both nonpolar and polar substrates can interact. Ser180 is involved in PLP binding; however, to improve the activity of a D-aminotransferase with R-MP, one can modify the Ser240, Thr242, or Ser243 residues to accept larger substrates or to favor negatively charged substrates. For instance, Thr242 can be mutated to Ser to reduce the side chain length. One of the residues can be mutated to lysine or arginine, such as Ser243. The residues (YM-1 numbering) Val30-Val36 are located in a beta strand across the active site of the D-aminotransferase, and are also important for activity. Tyr31, Val33, Glu32, and Lys35 are thought to face the active site. Tyr31, Glu32, and Val33 are invariant in all the *Bacillus* homologs. Ro, et al., FEBS Lett 398:141-145, (1996)) mutagenized Val33 to Ala and found a slightly increased catalytic efficiency for alpha-ketoglutarate transamination and a significantly improved catalytic efficiency for bulkier substrates (less steric hindrance). In some homologs Lys35 is replaced with Arg, but if steric hindrance is a concern the Lys residue may be preferable. Valines 34 and 36 are also preferable over conservative substitutions such as isoleucine, again due to less steric hindrance for large molecules such as MP. Because the novel D-aminotransferase ("4978") described in Examples 15 and 16 had higher activity than the *B. sphaericus* enzyme and the hybrid DAT described in Example 19 it is the obvious choice for further mutagenesis reactions. The ideas above, based upon crystal structure analysis of YM-1 D-aminotransferase, can be applied to the D-aminotransferase from ATCC strain 4978. The numbering above is one amino acid less than the corresponding amino acid in the 4978 protein sequence.

### Example 7

### Use of Branched Chain Aminotransferases ("BCAT") in the Production of Monatin

AT-102 and AT-104 are branched chain L-transaminases (EC 2.6.1.42) that were purchased from BioCatalytics (Pasadena, CA). The enzymes were tested for transamination activity using S,S and R,R monatin substrates that were produced chemically. Reactions were performed in a total volume of 0.5 mL, and run in duplicate. The assays contained 50 mM Tris pH 7.8, 0.08 mM PLP, 10 mM α-ketoglutarate ("α-KG"), 5 mM monatin, and 1 mg/mL aminotransferase enzyme. Negative controls did not contain exogenous aminotransferase enzyme. The samples were incubated for 2 hours at 30 °C at 100 rpm shaking. The samples were filtered and LC/MS/MS analysis, as described in Example 1, was run to ascertain glutamate levels. Glutamate levels should correlate stoichiometrically with MP production. When R,R was used as the reaction substrate, very low levels of glutamate were present in the negative controls. AT-104 produced slightly more glutamate than the negative controls, indicating a low level of activity with the R,R monatin substrate (a D-amino acid). Both of the branched chain L-aminotransferases showed activity on S,S monatin. AT-102 produced 102 µg/mL glutamate and AT-104 produced 64 µg/mL glutamate. For comparison, a broad specificity aminotransferase (AT-101, also from BioCatalytics) produced 75 µg/mL under these conditions. The high activity with a branched chain aminotransferase is somewhat unexpected because monatin has more structural similarities to dicarboxylic amino acids and aromatic amino acids that normally serve as substrates for the broad specificity or aspartate aminotransferases. However, due to the glutamatic acid backbone of monatin, many of the aminotransferases that can utilize glutamate as an amino donor may also have activity on monatin.

### Monatin Production from Indole-3-Pyruvate using BCAT

AT-102 and AT-104 were tested for production of monatin in coupled reactions using the ProA aldolase from *C*. *testosteroni* (produced as described in WO 03091396 A2). Enzymes and additional components/substrates were added directly to the reaction buffer provided in the kit, which contained 100 mM potassium phosphate buffer pH 7.5, 100 mM L-glutamate, and 0.1 mM PLP. To one mL of reaction buffer were added: 4 mg indole-3-pyruvate, 20 mg pyruvate, approximately 50 µg ProA provided in a cellular extract, 1 µL 2 M MgCl₂, and 2 mg of aminotransferase enzyme to be tested. All reactions were performed in duplicate, and a negative control reaction was done with no additional aminotransferase added. A positive control (AT-101) was utilized for comparison; this enzyme is a broad specificity L-aminotransferase. Background production of monatin is due to native *E. coli* aminotransferases present in the cellular extract of the recombinant ProA enzyme. The reactions were incubated overnight at 30 °C with gentle shaking (100 rpm). The samples were filtered and submitted for reverse phase LC/MS/MS analysis as described in Example 1. The results are presented in Table 23 below.

**Table 23**

| Enzyme | µg/mL Monatin Produced |
|---|---|
| AT-101 | 173.05 |
| AT-102 | 122.05 |
| AT-104 | 133.05 |
| negative | 73.25 |

AT-102 and AT-104 aminotransferases clearly produced more monatin than the negative control and were about 50-60% as active as the positive control.

The branched chain aminotransferase enzyme from *E. coli* has been well studied and crystal structures have been analyzed in detail. Okada, K., et al., (1997) J. Biochem (Tokyo) 121:637-641, (1997). The enzyme has a similar overall fold and significant sequence homology to *Bacillus* D-aminotransferase enzymes such as those mentioned in Examples 2, 3, and 6. In addition, the BCAT enzymes and the D-aminotransferases from *Bacillus* are the only two types of PLP-dependent aminotransferases to show stereospecificity for *re* face addition of hydrogen to PLP. Yoshimura, T., et al., J. Am. Chem. Soc. 115:3897-3900, (1993). BCAT is thought to be the only enzyme in which the alpha-amino acid substrate is bound with its carboxyl group on the same side as the phosphate group, allowing the enzyme to have a similar fold and mechanism to the D-aminotransferases while still retaining specificity for L-amino acids. Peisach, D., et al., Biochemistry 37:4958-4967, (1998). It is thought that the L-specificity of BCAT comes from the fact that the polar amino acid side chains of the D-aminotransferase that position the alpha-carboxyl group of the substrate are replaced by nonpolar residues in BCAT. It is expected that if all, or some, of these residues are mutated to the corresponding amino acids of the *Bacillus* D-aminotransferase, one could convert the BCAT into a D-specific aminotransferase. The following mutations can be made to the *E. coli* BCAT (numbering based on accession number gi:14719463): Phe37 to Tyr, Val110 to His, Met108 to Arg. Other polar amino acid substitutions could be made at these sites as well, to tailor the enzyme active site to accept large dicarboxylic acid substrates as described in Example 6. Tyr165 may need to be converted to Leu as well, to mirror the PLP interaction of the D-aminotransferase; Tyr96 (to Phe), Arg41, and Arg98 may also need to be mutated to prevent binding of the alpha carboxyl group in the incorrect orientation in the BCAT enzyme. Trp127 can also be mutated to Tyr to decrease the likelihood of the hydrophobic side chains binding in a pro-S configuration; Tyr32 and Tyr130 may interact with L-glutamate in the active site of BCAT and can be mutated to negatively charged amino acids to minimize this interaction. Goto, M., et al., Biochemistry 42:3725-3733, (2003); Okada, K., Biochemistry 40:7453-7463, (2001).

Because both the D-aminotransferase enzymes and the branched-chain aminotransferase have activity in production of monatin, it is expected that the BCAT can be converted to a D-aminotransferase with activity in R,R monatin production, while providing another possible D-aminotransferase enzyme to be utilized in the reaction schemes described in many of the Examples. Based on the above results, it is possible that the AT-104 enzyme already shows some activity toward D-amino configurations of monatin.

### Bacillus Branched-Chain Aminotransferase Cloning and Mutagenesis

*Bacillus licheniformis* contains a putative branched-chain aminotransferase that is more closely related to D-aminotransferases than the *E. coli* branched chain aminotransferase is. It was assayed for D-transamination activity, and mutagenized based on predicted active site residues mentioned above for the *E. coli* BCAT.

### Strain

*B. licheniformis* (ATCC number 14580) was grown on Nutrient Agar at 30 °C overnight. Groups of colonies were placed in 100 µL of sterile water and heated for 10 minutes at 95 °C, to disrupt the cells. Three µL was used in subsequent Polymerase Chain Reaction (PCR) amplifications.

### Polymerase Chain Reaction Protocol

Primers were designed for the *B. licheniformis* gene (915 bp) for cloning into pET 28b and pET 30a vectors (Novagen, Madison, WI) and pTRC99a (GE Healthcare Life Sciences), using the Ncol and SalI sites. The pET30 construct contains an N-terminal His-tag and S-tag, whereas the pET 28 construct is untagged. *B. licheniformis bcat* primers:
N term 5'-GGTTAAGGCCATGGGGGACCAGAAAGACCA-3' (SEQ ID NO:44); and
C term: 5'-GGCCTTCCGTCGACTCAGCTGACACTTAAGCT -3' (SEQ ID NO:45)

The coding region was amplified using the following PCR protocol. In a 50 µL reaction, 3 µL template, 1 µM of each primer, 0.4 mM each dNTP, 3.5 U Expand High Fidelity Polymerase, and 1X Expand™ buffer (Roche, Indianapolis, IN) with Mg were used. The thermocycler program used included a hot start at 96 °C for 5 minutes, followed by 30 repetitions of the following steps: 94 °C for 30 seconds, 50 °C for 1 minute 45 seconds, and 72 °C for 2 minutes 15 seconds. After 30 cycles, the sample was maintained at 72 °C for 7 minutes and then stored at 4°C. Clean PCR products of the correct size were obtained (approximately 900 bp).

### Cloning

The PCR product was purified and digested with SalI and NcoI in SalI buffer (New England Biolabs, Ipswich, MA). The digested vectors (pET28, pET30, and pTRC99a) and the insert were purified using the Qiagen QIAquick Gel Extraction Kit. The ligations were done using the Roche Rapid DNA Ligation Kit (Roche) and purified. The ligations were transformed into *Escherichia coli* DH10B using a 0.2 cm cuvette and a Bio-Rad Gene Pulser II system, as described in the Bio-Rad electroporation manual. The cells were allowed to recover in 900 µL SOC medium for 30 minutes at 37°C at 225 rpm. The cells were plated on LB-agar plates containing kanamycin (25 µg/mL). The plasmid DNA was purified using the Qiagen spin miniprep kit and screened for the correct inserts by restriction digest with SalI and NcoI. The sequences of plasmids that appeared to have the correct insert were verified by dideoxy chain termination DNA sequencing at Agencourt BioScience Corporation (Beverly, MA). Sequencing verified the coding sequence found in NCBI accession number CP000002 GI 56160984 2851268..2850354, which produces a protein with amino acid sequence as listed in accession number AAU24468 GI:52004526.

### Gene Expression and Assays

Plasmid DNA (pET vectors) was transformed into *E. coli* expression host BL21(DE3) (Novagen, Madison, WI) for constructs in pET vectors. The cultures were grown and the plasmids were isolated using Qiagen miniprep kit, and analyzed by restriction digest to confirm identity. Induction was typically performed in LB medium containing kanamycin (50 µg/mL). The cells were grown to an OD₆₀₀ of 0.4-0.8 at 37 °C and induced with 0.1 mM IPTG (isopropyl thiogalacatoside) and sampled at 3-4 hours post induction. Cell extracts were prepared according to the protocol accompanying the Novagen BugBuster™ reagent (with benzonase nuclease and Roche complete protease inhibitor cocktail added). High levels of soluble protein were obtained at the predicted molecular weight, as judged by SDS-PAGE. The soluble proteins in the cellular extracts were separated by SDS-PAGE.

Cell extracts were analyzed for D-aminotransferase activity by following production of alanine from pyruvate (or glutamate from alpha-ketoglutarate) and D-tryptophan using the following protocol. Duplicate one mL reactions were typically carried out in 100 mM potassium phosphate buffer (pH 7.5), 50 µM pyridoxal phosphate, 25 mM sodium pyruvate, and 50 mM D-tryptophan. The reactions were initiated by the addition of cell free extracts or purified enzyme and were incubated 15 minutes overnight at 30 °C, with mild shaking. Approximately the same level of D-aminotransferase was added (typically around 0.5 mg) in each assay for comparative purposes, and AT-103 (BioCatalytics) was often used as a benchmark enzyme. Formic acid was added to a final concentration of two percent to stop the reaction, and the precipitated protein was removed by centrifugation. Control reactions without added protein were also performed. Zero time points were also used as negative controls. Alanine and glutamate were detected using OPA derivatization as described in Example 1. The branched-chain aminotransferase had low levels of D-aminotransferase activity in comparison to the AT-103 and *B. sphaericus* enzymes.

The branched chain aminotransferase was also tested for the ability to produce monatin from D-tryptophan (as in Example 3), but did not appear to have activity under the conditions tested.

The pTRC99a construct was transformed into electrocompetent *E. coli* CAG18455 cells, which are auxotrophic for tryptophan production. Cells were grown in M9 minimal medium with Balch's vitamins with 100 mg/L L-tryptophan, 0.4 % glucose, and calcium chloride. Cells were not able to grow without L-tryptophan. Induction was tested at 10, 100 and 1000 µM IPTG, at an OD600 of 0.4 for 4.5 hours. Bands at the correct MW were visible on SDS-PAGE. The plasmid was mutagenized using the QuikChange® Multi Site-Directed Mutagenesis Kit (Stratagene). The primers in Table 24 below were designed as described by the manufacturer.

**Table 24**

| Amino Acid Mutation (*E. coli* Numbering) | Nucleotide Mutation (*B. lich* Numbering) | Primer Sequence |
|---|---|---|
| Y32F | tac 96-->ttc | ATCACGGATTTTTATTCGGGGACGGCGTG (SEQ ID NO:46) |
| Y32D | tac 96-->gac | ATCACGGATTTTTAGACGGGGACGGCGTG (SEQ ID NO:47) |
| F37Y | ttt 111 --> tat | GGACGGCGTGTATGAAGGGATCAGGG (SEQ ID NO:48) |
| R41K | agg 123 -->aag | TGTTTGAAGGGATCAAGGTATACGACGGCAAC (SEQ ID NO:49) |
| F37Y + R41K | | GACGGCGTGTATGAAGGGATCAAGGTATACGACG (SEQ ID NO:50) |
| Y96F | tac276-->ttc | GCTGAAAGACGCTTTCATCCGCTTGGTCG (SEQ ID NO:51) |
| Y96H | tac276 --> cac | GCTGAAAGACGCTCACATCCGCTTGGTC (SEQ ID NO:52) |
| R98Y | cgc282 -->tac | |
| Y96F + R98Y | | |
| Y96H + R98Y | | |
| L108R | ctc 312 --> cgc | GCAGGTGACCGCGGACTCGATCCAAAC (SEQ ID NO:56) |
| L110H | ctc318 --> cac | GCAGGTGACCTCGGACACGATCCAAAC (SEQ ID NO:57) |
| L108R + L110H | | GCAGGTGACCGCGGACACGATCCAAACAATTG (SEQ ID NO:58) |
| L127Y | ttg369 --> tac | |
| L127K | ttg369-->aag | |
| I130E | ata375-->gaa | |
| L127Y + I130E | | |
| L127K + I130E | | |
| Y165L | tac477 --> ttg | |

Amino acid mutations were based on the *E. coli* BCAT crystal structure and the numbering in the above table is for the *E. coli* protein. The numbering for the DNA mutations is based on *the B. licheniformis bcat* gene.

The primers were diluted to 0.1 mg/mL and approximately 100 ng of each oligonucleotide primer was typically used in a 50 µL mutagenesis reaction, proportionately higher concentrations were used for larger primers. For oligonucleotide primers that were essentially competing for annealing to the same template region, sometimes a sum of 100 ng was used for the whole pool of primers in that region. Two hundred nanograms of template (*B. lich bcat* in pTRC99a) were used in the reaction, with 5 µL of 10X QuikChange buffer, 2 µL dNTPs, and 2 µL of the enzyme blend. The amplification products were treated with *Dpn*I restriction endonuclease (Stratagene) (2 µL) for 2 hours at 37°C, and transferred to a thick wall 1.5 mL tube for ethanol precipitation. The resuspended (concentrated) reaction mix was transformed (2.5 µL) into XL10-Gold Ultracomp cells included in the QuikChange kit. Several colonies were miniprepped and sequenced to ensure that mutations were random and to estimate the level of mutagenesis achieved. Colonies were resuspended from the plate and bulk minipreps were done. The miniprep DNA was then transformed into the tryptophan auxotroph strain, and plated on minimal medium (with IPTG) described above or using minimal medium containing D-tryptophan as the sole nitrogen source. A second and third round of mutagenesis was done on the bulk minipreps using primers that did not appear to incorporate well in the previous rounds. At each stage, colonies that grew quickly on the minimal medium (larger colonies) were retained for further analysis. The mutants shown in Table 25 below were isolated from the selection plates. In some cases these same mutants appeared on the selection medium more than one time.

**Table 25**

| Clone | Mutations |
|---|---|
| 4 | F37Y, Y96F |
| 6 | Y96F |
| 28 | F37Y, Y165L |
| 32 | Y96F, L127K |
| 5-1 | F37Y, Y96F, R98Y, L108R, L110H, L127Y |
| 5-2 | F37Y, R41K, Y96F, R98Y, L108R, L110H, L127Y |

The mutant constructs were induced to make recombinant protein in LB media, and cell extracts were prepared as above. The soluble proteins in the cellular extracts were separated on a BioRad Laboratories Experion Automated Electrophoresis Station and analyzed for concentration and percent expression using the Experion Software version 1.1.98.0. Very low levels of soluble recombinant protein were observed; thus quantitation of the band of interest was not possible. Assays were done to test D-tryptophan transamination as above using 50- 250 µL of cellular extracts. Clones 4, 6, 28, and 32 were assayed using both alpha-ketoglutarate and pyruvate as the amino acceptor, and incubated for 2 hours and overnight at 30 °C. The background levels of alanine/glutamate present from the cellular extracts was subtracted. For the assays with 5-1 and 5-2, the protein concentrations estimated by the Experion software for the BCATs were 275.1 ng/µl for the wildtype enzyme, 409.3 ng/µl for BCAT 5-1, and 148.2 ng/µl for BCAT 5-2. The results of the assays are shown in Tables 26-28 below.

**Table 26**

| BCAT | Glutamate (mM) 2 Hours | Glutamate (mM) Overnight |
|---|---|---|
| wildtype (100 µL) | 0.0912 | 0.2304 |
| wildtype (250 µL) | 0.251 | 0.521 |
| 4 (100 µL) | 0.0642 | 0.1202 |
| 4 (250 µL) | 0.154 | 0.295 |
| 6 (100 µL) | 0.053 | 0.112 |
| 6 (250 µL) | 0.141 | 0.289 |
| 28 (100 µL) | 0.0586 | 0.1402 |
| 28 (250 µL) | 0.155 | 0.367 |
| 32 (100 µL) | 0.0616 | 0.1236 |
| 32 (250 µL) | 0.167 | 0.339 |

**Table 27**

| BCAT | Alanine (mM) 2 Hours | Alanine (mM) Overnight |
|---|---|---|
| wildtype (250 µL) | 0.199 | 0.438 |
| 4 (250 µL) | 0.093 | 0.249 |
| 6 (250 µL) | 0.097 | 0.249 |
| 28 (250 µL) | 0.117 | 0.325 |
| 32 (250 µL) | 0.102 | 0.285 |

**Table 29**

| BCAT | Glutamate (mM) 1 Hour | Glutamate (mM) Overnight |
|---|---|---|
| wildtype (50 µL) | 0.018 | 0.075 |
| wildtype (100 µL) | 0.037 | 0.152 |
| 5-1 (50 µL) | 0.005 | 0.017 |
| 5-1 (100 µL) | 0.01 | 0.045 |
| 5-2 (50 µL) | 0.001 | 0.011 |
| 5-2 (100 µL) | 0.003 | 0.031 |

It is evident that like most L-aminotransferases, the enzymes have a preference for alpha-ketoglutarate compared to pyruvate for the amino acceptor. All the mutants did have D-aminotransferase activity, as did the wildtype parent. It is not clear whether the wildtype enzyme had more or less D-aminotransferase activity, because exact quantitation of the BCAT protein from cellular extracts was not possible. However, it is expected that the mutant enzymes have less L-aminotransferase activity than the wildtype; thus the ratio of D- to L-transamination rate is being improved. Continued mutagenesis could provide an alternative enzyme in pathways to monatin.

### Example 8

### Cloning, Expression, and Testing of Glutamate and Aspartate Racemases

This example describes methods used to clone and test amino acid racemase enzymes, which can be used to interconvert between L-glutamate and D-glutamate (or L- and D-aspartate or L- and D-alanine). Glutamate, aspartate, or alanine racemases are useful in a biosynthetic pathway to produce R,R monatin when a step in that pathway produces an L-amino acid (e.g., L-glutamate, L-aspartate, or L-alanine) and another step in the pathway consumes a D-amino acid (e.g., D-glutamate, D-aspartate, or D-alanine). FIG. 4 illustrates a biosynthetic pathway for producing R,R monatin from L-tryptophan using an L-tryptophan-specific aminotransferase, an R-specific aldolase, a D-aminotransferase and a glutamate (or aspartate or alanine) racemase.

Genes were cloned into the pET28 and pET30 vectors to generate both non-tagged proteins and fusion proteins with cleavable N-terminal HIS₆-Tag/T7-Tags. The resulting proteins were purified using immobilized metal affinity chromatography.

### Experimental Overview

Genes encoding glutamate racemases (EC 5.1.1.3) from *Lactobacillus brevis* (Genbank Accession No. D29627, nucleic acid sequence), and *Pediococcus pentosaceus (murI* gene) (Genbank Accession No. L22789) were cloned and expressed in *E. coli.* The extracts were tested for activity in conversion of L-glutamate to D-glutamate and D-glutamate to L-glutamate. BioCatalytics aspartate racemase enzyme (EC 5.1.1.13) was also tested for interconversion between L- and D-aspartate.

### Isolation of Genomic DNA for Cloning

*L. brevis* genomic DNA (ATCC 8287D) was obtained from the American Type Culture Collection. *P. pentosaceus* (ATCC 25745) was grown at 37 °C in *lactobacilli* MRS broth and 2 ml was used for genomic DNA isolation using the method of Mekalanos, J.J., "Duplication and amplification of toxin genes in Vibrio cholerae," Cell 35:253-263, (1983).

### Polymerase Chain Reaction Protocol

Primers were designed with 5' restriction sites and overhangs for cloning into the pET 28 and pET30 vectors (Novagen, Madison, WI).

*L. brevis* glutamate racemase primers:
N term: 5'-GCGGCGCCATGGAAAATGATCCGATTGGTCTAATG -3' (SEQ ID NO: 15), and
C term: 5'-GCGGCGGTCGACGCAATTACAATTGTGTTTGTC-3' (SEQ ID NO:16).

*P. pentosaceus* glutamate racemase primers:
N term: 5'- GCGGCGCCATGGATGTATGTATAATTTTATTTAG -3' (SEQ ID NO: 17), and
C term: 5'-GCGGCGGTCGACAAATTTCATTATTCATTCTAATTT -3' (SEQ ID NO: 18).

The gene derived from *L. brevis* was amplified using the following PCR protocol. In a 50 µL reaction, 0.150 µg template, 1.6 µM of each primer, 0.4 mM each dNTP, 2.8 U Expand High Fidelity™ Polymerase (Roche, Indianapolis, IN), 0.5 U Pfu polymerase (Stratagene, La Jolla, CA) and 1X Expand™ buffer with Mg were used. The thermocycler program used included a hot start at 96 °C for 3 minutes, 8 repetitions of the following steps: 94 °C for 30 seconds, 52 °C for 45 seconds, and 72 °C for 2 minutes, followed by 22 repetitions of the following steps: 94 °C for 30 seconds, 60 °C for 45 seconds, and 72 °C for 2 minutes. After the 22 repetitions, the sample was maintained at 72 °C for 7 minutes and then stored at 4 °C. This PCR protocol produced a product of∼830 bp, as judged by comparison to DNA size markers.

The gene derived from *P. pentosaceus* was amplified using the following PCR protocol. In a 50 µL reaction, 0.15 µg template, 1.6 µM of each primer, 0.4 mM each dNTP, 2.8 U Expand High Fidelity™ Polymerase, 0.5 U Pfu polymerase and 1X Expand™ buffer with Mg were used. The thermocycler program used included a hot start at 96 °C for 3 minutes, followed by 8 repetitions of the following steps: 94 °C for 30 seconds, 37 °C for 45 seconds, and 72 °C for 2 minutes, followed by 8 repetitions of the following steps: 94 °C for 30 seconds, 45 °C for 45 seconds, and 72 °C for 2 minutes, followed by 14 repetitions of the following steps: 94 °C for 30 seconds, 55 °C for 45 seconds, and 72 °C for 2 minutes. After the 14 repetitions, the sample was maintained at 72 °C for 7 minutes and then stored at 4 °C. This PCR protocol produced a product of∼840 bp, as judged by comparison to DNA size markers.

### Cloning

The PCR products were gel purified from 0.8% TAE-agarose gels using the Qiagen gel extraction kit (Valencia, CA). The PCR products were quantified using a SmartSpec 3000™ spectrophotometer. The products were digested with restriction enzymes *NcoI* and *SalI* following the manufacturer's recommended protocols (New England Biolabs, Beverly, MA) and gel purified from 0.8% TAE-agarose gels using the Qiagen gel extraction kit. Vectors pET28 and pET30 were prepared by digestion with restriction enzymes *NcoI* and *SalI,* followed by treatment with shrimp alkaline phosphatase and purification from 0.8% TAE-agarose gels using the Qiagen gel extraction kit.

The digested vectors and inserts were ligated using the Rapid™ DNA Ligation Kit (Roche, Indianapolis, IN). Approximately 50 ng of treated insert, 100 ng of treated vector (3 to 1 molar ratio of insert to vector), 5 U of T4 DNA ligase (included with the Rapid™ DNA Ligation Kit, and 1X ligation buffer were incubated for 5 minutes at room temperature. The ligation reactions were purified using the High Pure PCR Product Purification Kit (Roche) and were used to transform *E. coli* DH10B electrocompetent cells (Invitrogen, Carlsbad, CA). Ten µL of each ligation reaction was added to 40 µL of DH10B cells and were transformed by electroporation using the BioRad Gene Pulser II under the following conditions: 2.5 kV, 25 µF, 200 ohm in a 0.2 cm cuvette. The cells were allowed to recover in 1 mL of room temperature SOC for 1 hour at 37 °C with shaking at 225 rpm. The cells were plated on LB plates containing kanamycin (50 µg/mL).

Plasmid DNA was purified from the resulting transformants using the Qiagen spin miniprep kit and screened for the correct inserts by restriction digest with *Nco*I and *Sal*I*.* The sequences of plasmids appearing to have the correct insert were verified by dideoxy chain termination DNA sequencing.

### Gene Expression and Assays

Plasmid DNA, verified by sequence analysis, was subcloned into *E. coli* expression host BL21(DE3) (Novagen, Madison, WI). The cultures were grown and the plasmids were isolated using Qiagen miniprep kit, and analyzed by restriction digest to confirm identity.

Induction in BL21(DE3) was initially performed with *L. brevis* and *P*. *pentosaceus* glutamate racemases in both pET28 (untagged) and pET 30 (histidine-tagged) vectors. A time course study was performed with cultures grown in 250 mL LB containing kanamycin (50 mg/L) to an OD₆₀₀ of 0.5 - 0.6 and induced with 100 mM IPTG (isopropyl thiogalacatoside) and sampled at 0 and 3 hours post induction. Cells from 600 µL (0 hour) and 275 µL (3 hour) were resuspended in 40 µL sodium dodecyl sulfate buffer containing 2-mercaptoethanol, and heated at 95°C for 10 minutes, and cooled. Aliquots of these total cellular protein samples were analyzed by SDS-PAGE using a 4-15% gradient gel.

Cell extracts were also prepared from the 3 hour cultures by suspending cell pellets from 5 mL of culture in 0.625 mL Novagen BugBuster™ reagent containing 0.625 µL benzonase nuclease and 3 µL protease inhibitor cocktail set #3 (Calbiochem-Novabiochem Corp., San Diego, CA) at room temperature for 20 minutes with gentle shaking, and centrifuging at 16,000 x g to remove cell debris. The supernatants (cell extracts) were loaded onto 4-15% gradient gels for analysis of the cellular soluble proteins.

The 3-hour samples from cloned *L. brevis* glutamate racemase and *P. pentosaceus* glutamate racemase showed both total and soluble protein that corresponded to the correct size (approximately 31 kDa). The *L. brevis* pET30 (histidine-tagged) gene product was over-expressed at a higher level than, and was also more soluble (>20% of soluble protein) than, the *L. brevis* pET 28 (untagged) gene product, as well as the *P. pentosaceus* gene products in both vectors. The *P. pentosaceus* gene product showed equal overexpression and solubility in the pET28 and pET30 vectors, which was significantly less than that observed for the *L. brevis* pET30 gene product.

Cells from the induced cultures (250 mL) were centrifuged and washed once with 0.85 % NaCl. Cell pellets were resuspended in 5 mL/g wet cell weight of BugBuster™ (Novagen) reagent containing 5 µL/mL protease inhibitor cocktail set #3 (Calbiochem-Novabiochem Corp., San Diego, CA) and 1 µL/mL benzonase nuclease. Samples were incubated at room temperature for 20 minutes on an orbital shaker. Insoluble cell debris was removed by centrifugation at 16,000 X g for 20 minutes at 4°C.

Cell extracts were assayed for glutamate racemase activity using the following protocol. 400-µL reactions were carried out in 10 mM potassium phosphate (pH 8.0), 0.2 mM dithiothreitol ("DTT"), and 10 mM L-glutamate or D-glutamate. The reactions were initiated by the addition of 20-100 µL of cell free extracts and were incubated at room temperature. Sample aliquots were taken over a time course of 1 minute, 5 minutes, 10 minutes, 20 minutes and 1 hour (zero minute samples served as control reactions). 2 M formic acid (25 µL) was added to each 40-µL sample aliquot to stop the reaction and the precipitated protein was removed by centrifugation. Supernatants were removed and frozen at -80°C until they were analyzed by LC/MS/MS.

Assay results from cell extracts from pET30 induction with 100 mM IPTG (3 hours) demonstrate that *L. brevis* (Genbank Accession No. BAA06106.1 GI:468450) and *P*. *pentosaceus* (Genbank Accession No. AAA16761.1 GI:349029) enzymes have significant levels of racemase activity on both glutamate isomers. The *P. pentosaceus* racemase (20 µL of cellular extracts) reached equilibrium between L-and D-glutamate in 10-20 minutes starting with either substrate. The *L. brevis* enzyme (20 µL of cellular extracts) also reached equilibrium in approximately 20 minutes.

A partially purified aspartate racemase enzyme (catalog # ASPR-101) purchased from BioCatalytics, Inc. (Pasadena, CA) was assayed for activity on L-aspartate and D-aspartate using a protocol similar to the one above. The commercial enzyme showed racemase activity on both isomers. Using 0.5-1 mg of enzyme, equilibrium was achieved in 20-60 minutes.

All three racemases (*L. brevis* glutamate racemase, *P. pentosaceus* glutamate racemase and BioCatalytics aspartate racemase were also assayed for activity on S,S monatin using the following protocol. 400-µL reactions were carried out in 10 mM potassium phosphate (pH 8.0), 0.2 mM DTT, and 10 mM S,S monatin. The reactions were initiated by the addition of cell free extracts (*L. brevis* and *P. pentosaceus)* or purified enzyme (BioCatalytics aspartate racemase) and were incubated at room temperature. Sample aliquots were taken over a time course of 1 minute, 5 minutes, 10 minutes, 20 minutes and 1 hour (zero minute samples served as control reactions as well as samples without enzyme). 2 M formic acid (25 µL) was added to each 40-µL sample aliquot to stop the reaction and the precipitated protein was removed by centrifugation. Supernatants were removed and frozen at -80 °C until they were analyzed by LC/MS/MS (Example 1). No decrease in S,S monatin concentration was noted over time, nor was there any increase in S,R monatin (present initially as <5% contaminating byproduct, even in the no enzyme control). Therefore, none of the racemases assayed showed activity towards monatin.

### Example 9

### Production of R,R Monatin from L-Tryptophan Using Alanine, Glutamate, or Aspartate Racemases

This example describes methods of producing stereoisomerically-enriched R,R monatin from L-tryptophan using an L-tryptophan (L-tyrosine, or aromatic) aminotransferase, ProA aldolase, alanine, glutamate or aspartate racemase, and a broad specificity D-amino acid aminotransferase. FIG. 5 is a diagram that illustrates the pathway. This approach to production of stereoisomerically enriched R,R monatin requires an enzyme for step 1 that has low activity in the production of monatin from monatin precursor (MP). Based upon earlier results, we used the *Sinorhizobium meliloti* and *Rhodobacter sphaeroides tatA* gene products described in Example 1 from WO 03/091396 A2.

### Materials and Methods

Glutamate racemases from *L. brevis* and *P. pentosaceus* were produced in *E. coli* as described in Example 8. In some cases the His₆-tagged version of these enzymes were purified using His-Bind 900 cartridges according to manufacturer's protocols (Novagen, Madison, WI) and were desalted to remove imidazole using PD-10 columns (G25 Sephadex, Amersham-Pharmacia). The enzymes were eluted in 25 mM potassium phosphate pH 8.0. Aspartate racemase (ASPR-101) and D-aminotransferase (AT-103) were purchased from BioCatalytics, Inc., alanine racemase was purchased from Sigma (St. Louis, MO) (catalog number A8936). *S*. *meliloti* and *R. sphaeroides* tyrosine (aromatic) aminotransferases were prepared as described in Example 1 from WO 03/091396 A2. *Comamonas testosteroni* ProA aldolase was prepared as described in Example 4 from WO 03/091396 A2. Total protein assays were done utilizing the Bio-Rad Protein Assay according to manufacturer's protocols (Hercules, CA).

### Reduction in Amount of S,S Monatin Produced using Racemases

Reaction mixtures (1 mL volume, run in duplicate) contained 100 mM potassium phosphate buffer (pH 8), 2 mM MgCl₂, 0.05 mM pyridoxal 5'-phosphate ("PLP"), 200 mM sodium pyruvate, 5 mM sodium α-ketoglutarate or oxaloacetate, approximately 280 µg/mL *S*. *meliloti* TatA supplied in a cellular extract, 1 mg/mL BioCatalytics D-aminotransferase (AT-103), 100 µL/mL of glutamate racemase cellular extract or 1 mg/mL aspartate racemase, and approximately 100 µg/mL of ProA aldolase provided as a cellular extract. Solid tryptophan was added at a concentration of 10.2 mg/ml. Negative controls did not contain racemase. Samples were incubated at 30 °C (shaking at 250 rpm) for 1 hour, 2 hours, or overnight. Samples were centrifuged to remove precipitate, syringe filtered, and stored at -80°C prior to analysis for monatin using the LC/MS/MS method described in Example 1.

Most of the samples contained >95% S,S monatin, due to the amounts of native L-aminotransferase present in the cellular extracts. However, the samples that contained racemase had a reduced amount of total monatin as a result of the racemase enzymes making L-glutamate less available for transamination of MP. Without racemase, 1545-2355 ppm monatin (predominantly S,S) was produced during the timecourse. With the racemases present, only 340-879 ppm (*L. brevis* enzyme), 444-531 ppm *(P. pentosaceus* enzyme), and 506-1460 ppm monatin (aspartate racemase) were produced. These data indicate that the racemases are active in the reaction conditions required to produce monatin. To minimize formation of S,S monatin from cellular extract enzymes, such as aspartate aminotransferases, further experiments were done with purified enzymes and a higher ratio of D-aminotransferase to L-aminotransferase enzymes.

### Conversion of L-tryptophan to 4-R Containing Isomers of Monatin

The above experiments were repeated using approximately 54 µg of purified L-aminotransferase (either *S. meliloti* or *R. sphaeroides* TatA), 1 mg aspartate aminotransferase (BioCatalytics), 1 mg D-aminotransferase, 5mM oxaloacetate as the amino acceptor, and 75 µg purified aldolase. The reactions were run in duplicate with a 2-hour sampling time and an overnight incubation time. Negative controls were done with *S. meliloti* L-aminotransferase, but with no racemase. In addition to quantification of R,R/S,S and S,R/R,S monatin peak quantification based on reversed phase chromatography, the percentage of each stereoisomer was determined using the FDAA derivitization technique described in Example 1. The results are shown in Table 29 below.

**Table 29**

| L-Aminotransferase | Incubation Time | Total Monatin (ppm) | % S,S | % R,R | % R,S | % S,R |
|---|---|---|---|---|---|---|
| *S. meliloti* TatA | 2h | 17.1 | 10.2 | 58.1 | 0.8 | 31.0 |
| *S. meliloti* TatA | 2h | 15.8 | 13.3 | 55.3 | 1.0 | 30.4 |
| *S. meliloti* TatA | overnight | 77.7 | 25.8 | 40.0 | 1.3 | 32.9 |
| *S. meliloti* TatA | overnight | 67.9 | 29.4 | 37.3 | 1.5 | 31.8 |
| *R. sphaeroides* TatA | 2h | 241.2 | 96.3 | 2.3 | 0.8 | 0.6 |
| *R. sphaeroides* TatA | 2h | 223.2 | 95.7 | 2.7 | 1.0 | 0.6 |
| *R. sphaeroides* TatA | overnight | 600.4 | 96.6 | 1.8 | 0.5 | 1.1 |
| *R. sphaeroides* TatA | overnight | 618.5 | 96.1 | 2.1 | 0.5 | 1.3 |
| no racemase control | 2 h | 7.1 | 92.0 | 1.4 | 6.6 | 0.0 |
| no racemase control | 2h | 5.7 | 94.0 | 1.2 | 4.8 | 0.0 |
| no racemase control | overnight | 44.6 | 93.5 | 1.3 | 4.7 | 0.5 |
| no racemase control | overnight | 37.5 | 95.4 | 0.9 | 3.7 | 0.0 |

Clearly, the presence of the racemase increased the total amount of monatin produced when *S. meliloti* TatA was used as the enzyme for L-tryptophan transamination. Monatin levels increased from an average of 6.4 to 16.5 ppm in the two-hour assay, and from 41-73 ppm in the overnight assay. Additionally, the percent of R,R formed increased from about 1% up to as much as 58% by utilizing the racemase enzyme. The S,R stereoisomer of monatin, another potent sweetener, was the other major component, increasing from nearly 0 in the negative controls to 31%. The *R. sphaeroides* TatA clearly had more activity on S-MP than the *S. meliloti* L-transaminase, demonstrating the importance of having an enzyme that has high substrate specificity for L-tryptophan as compared to MP when 4-R isomers of monatin are the desired products. With about 10% of the total monatin being 4S at the two-hour timepoint, the *S*. *meliloti* TatA could be considered as having limited activity on MP.

The experiments were repeated with the purified S. *meliloti* TatA (54 µg) and the *L. brevis* glutamate racemase. When purified glutamate racemase was used, approximately 64 µg was used per 1 mL reaction. Cellular extracts containing the glutamate racemase were also tested and 1.4 mg of soluble protein was used. A no racemase negative control was utilized again and all samples were run in duplicate. The results are shown in Table 30 below.

**Table 30**

| Glutamate racemase | Incubation Time | Total Monatin (ppm) | % S,S | % R,R | % R,S | % S,R |
|---|---|---|---|---|---|---|
| *L. brevis* (purified) | 2h | 3.3 | 49.1 | 34.2 | 3.7 | 13.0 |
| *L. brevis* (purified) | 2h | 3.6 | 47.9 | 35.2 | 3.5 | 13.4 |
| *L. brevis* (purified) | overnight | 29.3 | 58.9 | 24.7 | 3.2 | 13.2 |
| *L. brevis* (purified) | overnight | 40.2 | 55.1 | 25.0 | 4.7 | 15.3 |
| *L. brevis* (cell extract) | 2h | 10.5 | 45.8 | 35.9 | 1.1 | 17.2 |
| *L. brevis* (cell extract) | 2h | 10.5 | 47.4 | 33.9 | 1.1 | 17.6 |
| *L. brevis* (cell extract) | overnight | 79.4 | 70.3 | 17.9 | 1.3 | 10.5 |
| *L. brevis* (cell extract) | overnight | 80.1 | 69.1 | 19.1 | 1.1 | 10.7 |
| none | 2 h | 2.7 | 84.1 | 7.1 | 6.3 | 2.4 |
| none | 2h | 3.2 | 84.9 | 6.0 | 6.8 | 2.2 |
| none | overnight | 36.5 | 92.3 | 2.3 | 4.2 | 1.2 |
| none | overnight | 30.5 | 92.7 | 2.0 | 4.1 | 1.3 |

Again, it is clear that the addition of the racemase increases the total monatin produced from L-tryptophan, as well as increases the relative amounts of 4R-containing isomers of monatin as compared to S,S monatin. The use of purified aldolase, racemase, and L-aminotransferase greatly improves the ability to control the desired stereoisomer formation. The ratio of L to D aminotransferase is also a way to manipulate stereochemistry of the final product.

When comparing results shown in Tables 1 and 2 in Example 2, to results with reaction conditions similar to the conditions above, one can see that approximately 7-29 ppm of monatin were formed from indole-3-pyruvate and the percentages of R,R monatin formed were approximately 51-90%. Using the aspartate racemase increased the total amount of monatin produced to 16-78 ppm monatin, with % R,R of approximately 40-58%. Additionally, a more stable and less expensive raw material (L-tryptophan) was utilized. In Example 3, approximately 73 ppm monatin was produced from D-tryptophan at a ratio of R,R:S,R of approximately 1.7:1. The total amount of 4R isomers was >80% of the total monatin. Because both R,R-monatin and R,S-monatin are potent sweeteners (>1000 times sweeter than sucrose), the ability to enrich for these isomers, without the need for expensive D-amino acid substrates, is critical.

It is expected that the availability of a non-specific or R-specific aldolase would increase the reaction rate as well as increasing the percentage of R,R monatin formed. *See* Example 5. Although the ProA aldolase from C. *testosteroni* used in these assays is reported to predominantly favor substrates in the S-configuration for fission reactions, this Pro A aldolase clearly does produce R-MP. Thus, aldolases that more preferentially produce MP in the R-configuration can help generate even greater percentages of R,R monatin. Additionally, it is expected that finding an L-tryptophan aminotransferase with even lower activity for monatin production would also decrease the amount of S,S and R,S monatin formed. Lastly, improvements can be made to the D-aminotransferase enzyme, or alternative D-aminotransferase enzymes can be used, that would have increased substrate specificity for R-MP versus S-MP. This would also increase formation of the R,R product, if so desired.

The aspartate racemase experiments were repeated to compare the activity of R-selective aldolase of SEQ ID NO:22 with the activity of the ProA aldolase from C. *testosteroni.* Approximately 50 µg of purified L-aminotransferase *(S. meliloti* TatA), 1 mg aspartate racemase (BioCatalytics), 1 mg D-aminotransferase (AT-103, BioCatalytics), 5 mM oxaloacetate as the amino acceptor, and 50 µg of the appropriate purified aldolase. The reactions were run in duplicate and incubated overnight at 30 °C. The percentage of each stereoisomer was determined using the FDAA derivitization technique described in Example 1. The results are shown below in Table 31.

**Table 31**

| Aldolase | Total Monatin (ppm) | % S,S | % R,R | % R,S | % S,R |
|---|---|---|---|---|---|
| SEQ ID NO:22 | 211 | | 72.7 | | 27.3 |
| *C. testosteroni* | 422 | 30.2 | 38.5 | | 31.3 |

The *C. testosteroni* ProA distribution of isomers is consistent with the earlier experiments above, whereas when the R-selective aldolase of SEQ ID NO:22 is used, the percent R,R is much higher, undetectable amounts of S,S are formed, and the amount of S,R monatin is lower.

As described in Examples 2 and 3, D-alanine can serve as the amino donor for transamination of MP to monatin. Many L-aminotransferases have the ability to utilize pyruvate as an amino acceptor to some extent and produce L-alanine. Because the above-mentioned reactions use high concentrations of pyruvate, it is likely that some of the pyruvate is converted to L-alanine. For example, during transamination of L-tryptophan, the HexAspC enzyme described in Example 6 has been found to convert 10-18% of pyruvate (50-200 mM initial concentrations) to L-alanine in 2 hours if alpha-ketoglutarate is absent. The enzyme showed a 10-fold preference for alpha-ketoglutarate when both amino acceptors were present at high (>50 mM) concentrations. AspC (described in WO 03/091396 A2) also produced some L-alanine from pyruvate. Therefore, it was expected that one can omit the addition of alpha-ketoglutarate or oxaloacetate in the above reactions and utilize an alanine racemase (EC 5.1.1.1) in place of glutamate or aspartate racemase.

Alanine racemase enzymes were first identified in *Brucella abortus* and *Streptococcus faecalis.* Marr, AG., and Wilson, P.W., Arch. Biochem. Biophys., 49:424-433, (1954); Wood, W.A., and Gunsalus, I.C., J. Biol. Chem. 190:403-416, (1951). The *dadB* gene in *Salmonella typhimurium* was identified as the source of alanine racemase activity and several hundred homologs can be found in genomics databases. Other known sources of alanine racemase activity are *Escherichia coli, Bacillus subtilis, Pseudomonas aeruginosa, Vibrio cholerae, Schizosaccaroyces pombe,* and *Bacillus cereus.* A basidiomycetous mushroom, *Lentinus edodes,* also contains a broad activity alanine racemase. A thermostable homolog from *Bacillus stearothermophilus* is available for purchase from Sigma-Aldrich (catalog # A8936) and has been immobilized for commercial applications. Inagaki, K., Biochemistry 25: 3268 (1986).

### Monatin Production with Alanine Racemase

Monatin production was tested using ProA aldolase from C. *testosteroni.* Approximately 50 µg of purified L-aminotransferase *(S. meliloti* TatA), 1 mg D-aminotransferase (AT-103, BioCatalytics), pyruvate as the amino acceptor, 50 µg purified aldolase, and 70 µg alanine racemase purchased from Sigma (St. Louis, MO) (catalog number A8936). The reactions were run in duplicate and incubated overnight. The percentage of each stereoisomer was determined using the FDAA derivitization technique described in Example 1. Controls with no racemase were included. The results are shown in Table 32 below.

**Table 32**

| Conditions | Total Monatin | % SS | % RS | % RR | % SR |
|---|---|---|---|---|---|
| Ala racemase (1 hour) | 4 | 66 | 21 | 12 | 1 |
| No ala racemase (1 hour) | 2.7 | 69 | 26 | 5 | 0 |
| Ala racemase (24 hours) | 82.9 | 90 | 5 | 4 | 2 |
| No ala racemase (24 hours) | 170.3 | 89 | 5 | 4 | 2 |

There was three-fold more R,R monatin in the one hour timepoint when alanine racemase was present compared to the sample with no alanine racemase. This result shows that it is possible to produce R,R monatin using alanine racemase. The percentage of R,R monatin produced could be improved using an aldolase that selectively produces R-monatin precursor, an L-aminotransferase that does not work or has limited activity on R-monatin precursor and a D-aminotransferase that does not work or has limited activity on indole-3-pyruvate.

### Example 10

### D-phenylglycine Aminotransferase (D-4-Hydroxyphenylglycine Aminotransferase)

As shown in FIG. 3, a stereoinverting aminotransferase is useful in a biosynthetic pathway for the production of monatin. For example, a D-phenylglycine aminotransferase or mutant thereof could produce R,R monatin from R-MP with L-glutamate as the amino donor.

### (1) PCR Synthesis of P. stutzeri 4 D- hydroxyphenylglycine Aminotransferase from Oligonucleotide Primers

This example describes methods that were used to synthesize 4 D-hydroxyphenylglycine aminotransferase, a stereoinverting enzyme that can be used to convert R monatin precursor to R,R monatin using L-glutamate as the amino donor.

### Primer Design

The published sequence (Genbank Accession No. AY319935, nucleic acid sequence; Genbank Accession No. AAQ8290, protein sequence) for *Pseudomonas stutzeri* 4 D-hydroxyphenylglycine aminotransferase (4 D-HPG AT) was used as a template for PCR primer design. Alternatively, the 4-D-hydroxyphenylglycine aminotransferase from *Pseudomonas putida,* (CAD42450 (protein), AX467211 (nucleotide)) is used as a sequence template. A total of 34 forward primers and 35 reverse primers were designed; forward and reverse primers were 40-mers sharing 20 overlapping base pairs. In addition, 2 outer primers were designed with 5' restriction sites and overhangs for cloning into the pET 28 and pET30 vectors (Novagen, Madison, WI).

*P. stutzeri* 4 D-HPG AT outer primers: N term (with *Nde*I Site):
5'-GGCCGGCATATGTCGATCCTTAACGACTACAAACGT -3'(SEQ ID NO: 19), and C term (*with Xho*I site):
5'-GGAAGGCTCGAGTCATGATTGGTTTCCAGACAAATT -3' (SEQ ID NO:20).

### Polymerase Chain Reaction Protocol

The gene sequence from *P. stutzeri* was amplified using the following protocols. The primary 100 µL PCR reaction included 0.05 µM of each of the internal 69 primers, 0.4 mM each dNTP, 10 U r*Tth* Polymerase XL (Roche, Indianapolis, IN), 0.625 U *Pfu* polymerase (Stratagene, La Jolla, CA), 1X XL buffer and 1 mM Mg(OAc)₂. The thermocycler program used included a hot start at 94 °C for 3 minutes, 15 repetitions of the following steps: 94 °C for 30 seconds, 42 °C for 30 seconds, and 68 °C for 15 seconds, followed by 10 repetitions of the following steps: 94 °C for 30 seconds, 52 °C for 30 seconds, and 68 °C for 30 seconds, followed by 10 repetitions of the following steps: 94 °C for 30 seconds, 60 °C for 30 seconds, and 68 °C for 1 minute and 15 seconds. After the final 10 cycles, the sample was maintained at 68 °C for 7 minutes and then stored at 4°C. This PCR protocol produced a smear of product at ~0.5 kb on a 0.8% TAE-agarose gel.

The secondary PCR reaction was set up using the primary PCR reaction as template. The secondary 100 µL PCR reaction included 2.5 µL of the primary PCR reaction, 0.5 µM of each of the 2 outer primers (with *Nde*I and *Xho*I restriction sites), 0.4 mM each dNTP, 10 U r*Tth* Polymerase XL, 0.625 U Pfu polymerase, 1X XL buffer and 1 mM Mg(OAc)₂. The thermocycler program used included a hot start at 94 °C for 3 minutes, 10 repetitions of the following steps: 94 °C for 30 seconds, 52 °C for 30 seconds, and 68 °C for 1 minute 30 seconds, followed by 15 repetitions of the following steps: 94 °C for 30 seconds, 60°C for 30 seconds, and 68 °C for 1 minute 30 seconds. After the 15 repetitions, the sample was maintained at 68 °C for 7 minutes and then stored at 4 °C. This PCR protocol produced a distinctive product band at ∼1.4 kb on a 0.8% TAE- agarose gel.

The PCR product was gel purified from 0.8% TAE-agarose gel using the Qiagen gel extraction kit (Valencia, CA). The product was TOPO cloned and transformed into TOP 10 cells according to manufacturer's protocol (Invitrogen, Carlsbad, CA). Plasmid DNA was purified from the resulting transformants using the Qiagen spin miniprep kit and screened for the correct inserts by restriction digest with *Nde*I and *Xho*I*.* The sequences of plasmids appearing to have the correct insert were verified by dideoxy chain termination DNA sequencing with universal M13 forward and M13 Reverse primers. Of the 10 clones sequenced, all had at least one mutation from the desired sequence. The best clone had a single base-pair mutation that resulted in an amino acid change. The sequence of this clone was corrected using the QuickChange Mutagenesis protocol according to manufacturer recommendations (Stratagene, La Jolla, CA).

The corrected TOPO clone was digested with restriction enzymes *Nde*I and *Xho*I following the manufacturer's recommended protocols (New England Biolabs, Beverly, MA) and gel purified from 0.8% TAE-agarose gels using the Qiagen gel extraction kit. Vectors pET 28 and pET 30 were prepared by digestion with restriction enzymes *Nde1* and *Xho1,* followed by treatment with shrimp alkaline phosphatase and purification from 0.8% TAE-agarose gels using the Qiagen gel extraction kit.

The digested vectors and insert were ligated using the NEB Quick Ligation Kit (Beverly, MA). Approximately 50 ng of treated insert, 100 ng of treated vector (3 to 1 molar ratio of insert to vector), 5 U of T4 DNA ligase, and 1X ligation buffer were incubated for 5 minutes at room temperature. The ligation mixture was transformed into TOP10F' chemically competent cells (Invitrogen). The cells were allowed to recover in 0.25 mL of room temperature SOC for 1 hour at 37 °C with shaking at 225 rpm. The cells were plated on LB plates containing kanamycin (50 µg/mL). The plasmid DNA was purified from the resulting transformants using the Qiagen spin miniprep kit and screened for the correct inserts by restriction digest with *Nde*I and *Xho*I*.*

### Gene Expression and Assays

Plasmid DNA was transformed into *E. coli* expression host BL21(DE3) (Novagen, Madison, WI). The cultures were grown and the plasmids were isolated using Qiagen miniprep kit and analyzed by restriction digest to confirm identity.

Induction in BL21(DE3) was performed with *P. stutzeri* D-4-hydroxyphenylglycine aminotransferase in both pET 28 (histidine-tagged) and pET 30 (untagged) vectors. A time course study was performed with cultures grown in 250 mL LB containing kanamycin (50 mg/L) to an OD₆₀₀ of 0.5 - 0.6, induced with 100 mM isopropyl thiogalacatoside ("IPTG") and sampled at 0 and 3 hours post induction. An appropriate volume of cells from 0 hours and 3 hours was resuspended in 40 µL sodium dodecyl sulfate buffer containing 2-mercaptoethanol, heated at 95 °C for 10 min, and cooled. Aliquots of these total cellular protein samples was analyzed by SDS-PAGE using a 4-15% gradient gel.

The cell extracts were also prepared from the 3 hour cultures by suspending cell pellets from 5 mL of culture in 0.625 mL Novagen BugBuster™ reagent containing 0.625 µl benzonase nuclease and 3 µL protease inhibitor cocktail set #3 (Calbiochem-Novabiochem Corp., San Diego, CA) at room temperature for 20 minutes with gentle shaking and centrifuging at 16,000 x g to remove cell debris. The supernatants (cell extracts) were loaded onto 4-15% gradient gels for analysis of the cellular soluble proteins. When noted, the protein was purified using His-Bind 900 cartridges according to manufacturer's protocols (Novagen, Madison, WI) and were desalted to remove imidazole using PD-10 columns (G25 Sephadex, Amersham-Pharmacia).

### (2) Isolation of Organisms with D-Phenylglycine Aminotransferase ("DPGAT")

Organisms of the genus *Pseudomonas* and like genera, with a stereoinverting D-phenylglycine aminotransferase (also called D-4-hydroxyphenylglycine aminotransferase) are isolated in the following manner. Soil samples are incubated on petri plates with the following medium: (per liter) 15 g agar, 3.4 g KH₂PO₄, 3.55 g Na₂HPO₄, 0.2 g MgSO₄·7H₂O, 8 mg CaCl₂·2H₂O, 10 mg yeast extract, 1 ml 1000x trace elements solution (Balch, W.E., et al., "Methanogens: reevaluation of a unique biological group," Microbiol. Rev. 43:260-296, (1979)), and 1 g D-phenylglycine (D-4-hydroxyphenylglycine).

Isolates are tested by PCR for the presence of a stereoinverting aminotransferase (primers are designed from known D-phenylglycine aminotransferases) or are further enriched for the presence of a stereoinverting aminotransferase as follows: isolates from the plates could be grown in liquid medium as above, without the agar, at 30 °C with shaking to an OD₆₀₀ of about 1.0. The cells are harvested by centrifugation and washed twice with 0.85% NaCl. A 10 mg (wet weight) sample is suspended in 1 ml potassium phosphate buffer (pH 7.0) and 5 mM D-phenylglycine or D-4-hydroxyphenylglycine. Neutralized 15 mM (aminooxy)acetic acid is added to duplicate samples prepared as described above. Consumption of D-phenylglycine (or D-4-hydroxyglycine) is measured by HPLC.

Isolates capable of degrading D-phenlyglycine (or D-4-hydroxyphenylglycine), but do so at a slower rate in the presence of (aminooxy)acetic acid, are selected for further analysis. Isolates are tested, by PCR, for the presence of a stereoinverting aminotransferase (primers are designed from known D-phenylglycine aminotransferases).

The presence of the stereoinverting aminotransferase is confirmed by growing a culture in a liquid medium as described above, harvesting the cells and making a cell-free crude extract ("CFE") and testing for D-phenylglycine aminotransferase or D-4-hydroxyphenylglycine aminotransferase enzyme activity. CFE is added to a reaction mixture with the following final concentrations: 0.1 M 3-(cyclohexylamino)-1-propanesulfonic acid ("CAPS") (pH 9.5), 60 mM L-glutamate (sodium salt), 5 mM benzoylformate or 4-hydroxybenzoate and 50 µM PLP.

The reverse reaction is measured by adding CFE to a reaction mixture with the following concentrations: 50 mM potassium phosphate (pH 7.0), 60 mM D-phenylglycine or D-4-hydroxyphenylglycine, 5 mM α-ketoglutarate, and 50 µM PLP. The assays are incubated at 35 °C and aliquots are taken at time points and stopped by boiling for 2 minutes. The product will be quantitated by the HLPC method of Gil-Av, E., et al., "Resolution of underivatized amino acids by reversed phase chromatography," J. Am. Chem. Soc., 102:5115-5117, (1980), or by the methods described in Example 1 directed to the measurement of glutamate formation.

As an alternative to PCR based methods, the stereoinverting D-phenylglycine aminotransferase is purified from the isolated bacteria by conventional protein purification techniques, including ammonium sulfate fractionation, and conventional column chromatography. Once the protein has been purified to a reasonable degree, peptide microsequencing techniques or conventional Edman type amino acid sequencing are utilized (*see* http://golgi.harvard.edu/microchem/ for descriptions of the protocols and equipment used for this type of work). Degenerate primers are designed based on the sequence available from the closest known relative of the protein source. Degenerate PCR and genome walking is then performed according to established protocols to isolate the stereoinverting D-phenylglycine aminotransferase coding sequence.

### (3) DPGAT Monatin Production

D-hydroxyphenylglycine aminotransferases, as described in *(1)* and (2) above, are used in crude cell free protein extracts, or purified as described in (*1*) above. *S. meliloti* and *R. sphaeroides* tyrosine (aromatic) aminotransferases are prepared as described in Example 1 from WO 03/091396 A2. *Comamonas testosteroni* ProA aldolase is prepared as described in Example 4 from WO 03/091396 A2. Total protein assays are done utilizing the Bio-Rad Protein Assay according to manufacturer's protocols (Hercules, CA).

Reaction mixtures (1 mL volume, run in duplicate) contain 100 mM potassium phosphate buffer (pH 8), 2 mM MgCl₂, 0.05 mM pyridoxal 5'-phosphate ("PLP"), 200 mM sodium pyruvate, 5 mM sodium α-ketoglutarate, approximately 280 µg/mL *S. meliloti* TatA supplied in a cellular extract, 100 µL/mL of D-hydroxyphenylglycine aminotransferase cellular extract or 1 mg/mL purified D-hydroxyphenylglycine aminotransferase, and approximately 100 µg/mL of ProA aldolase provided as a cellular extract. Solid tryptophan is added at a concentration of 10.2 mg/ml. Negative controls are set up without D-hydroxyphenylglycine aminotransferase. The samples are incubated at 30 °C with gentle shaking for ~1 hour or overnight. The samples are centrifuged to remove precipitate, syringe filtered, and stored at -80 °C prior to analysis for monatin using the LC/MS/MS method described in Example 1.

D-hydroxyphenylglycine aminotransferases with improved activity for monatin production are made using mutagenesis techniques known to those in the art, including: mutagenic PCR, passage through mutagenic strains, site-directed mutagenesis, error-prone PCR, or by methods such as DNA shuffling or other directed evolution technologies. The improved D-hydroxyphenylglycine aminotransferases are selected by growth on minimal medium with R,R-monatin as the source of nitrogen. Initially, the selection is based on growth, but as improved aminotransferases are selected, the screen is growth rate based. That is, cells with mutated versions of the gene are grown and the gene is expressed in minimal medium with R,R-monatin as the nitrogen source. The growth rates of the cells with the mutated versions of the gene are compared to the unmutated version. Those cells with a faster growth rate are selected and the aminotransferase is analyzed further. The D-hydroxyphenylglycine aminotransferase may be further mutagenized until the desired activity is obtained.

### (4) DPGAT Assay

The un-His-tagged version of the DPGAT was expressed as described in (1) above and extracts were used in assays. Assays were set up and included 100 mM potassium phosphate pH 7.0, 60 mM D-phenylglycine, 5 mM α-ketoglutarate, and 50 µM pyridoxal-5'-phosphate. The assays were started by adding 100 µL of extract, prepared as described above in this example, per ml of assay volume. Samples were taken at several timepoints (0, 1, 2, 5, 10, 30, 60, and 120 minutes) and were stopped with an equal volume of 2 M formic acid. A sample was also taken after overnight incubation (∼1200 minutes). The samples were analyzed for glutamate production by the OPA method described in Example 1. The results are summarized in Table 33 below.

**Table 33**

| Condition | Time (Minutes) | µmole/mL L-Glutamate |
|---|---|---|
| No substrate | 0 | 0.033 |
| | 1 | 0.033 |
| | 2 | 0.033 |
| | 5 | 0.035 |
| | 10 | 0.034 |
| | 30 | 0.036 |
| | 60 | 0.044 |
| | 120 | 0.038 |
| | ∼1200 | 0.058 |
| D-phenylglycine | 0 | 0.055 |
| | 1 | 0.112 |
| | 2 | 0.169 |
| | 5 | 0.315 |
| | 10 | 0.387 |
| | 30 | 0.892 |
| | 60 | 1.304 |
| | 120 | 1.514 |
| | ∼1200 | 1.056 |

The enzyme clearly has some activity on the D-phenylglycine. The enzyme activity was also tested on R,R monatin. The assay was set up as described above and R,R monatin was included at a concentration of 60 mM. The results are indicated below in Table 34.

**Table 34**

| Condition | Time (Minutes) | µmole/mL L-Glutamate |
|---|---|---|
| R,R monatin | 0 | 0.041 |
| | 1 | 0.040 |
| | 2 | 0.041 |
| | 5 | 0.041 |
| | 10 | 0.041 |
| | 30 | 0.042 |
| | 60 | 0.041 |
| | 120 | 0.040 |
| | ∼1200 | 0.045 |

There did not appear to be any detectable activity on R,R monatin. However, it is expected that the random or SDM methods described in this part (3) of this Example could be utilized to improve the transamination activity on R,R monatin or R-MP. For instance, the crystallization and preliminary analysis of the *P. stutzeri* enzyme has been done. Kongsaeree, P., et al., Acta Cryst. D59:953-954, (2003). Once the structure is published, docking experiments can be done using software such as Accelrys, to determine where steric hindrances or ionic repulsion may be prohibiting the R,R monatin from binding to the D-hydroxyphenylglycine substrate binding site. D-hydroxyphenylglycine is a somewhat large amino acid, as is R,R monatin. Both compounds have hydrophobic regions and hydroxyl groups. Modifications can be done to the binding pocket, as described in Example 6, to make the enzyme more amenable to dicarboxylic acid substrates. For instance, a residue near the second carboxyl group may be modified to a base such as arginine. Additionally, The *P. putida* gene described in part (1) and the additional genes that may be isolated as described in (2) can be used as templates for gene shuffling. Additionally, the *P. stutzeri* gene assembled in this Example can be mutagenized using oligonucleotide shuffling or other random mutagenesis methods, and screened as described in (3) above.

### Example 11

### Discovery of a D-Methionine Aminotransferase Gene

### Background

D-methionine-pyruvate aminotransferase (EC 2.6.1.41) is thought to be another example, although rare, of a stereoinverting transaminase. This enzyme catalyzes the reversible conversion of D-methionine and pyruvate to L-alanine and 4-methylthio-2-oxobutanoate. Oxaloacetate, phenylpyruvate, 2-oxobutyrate, 2-oxovalerate, 2-oxoheptanoate, glyoxylate, and oxoglutarate can also serve as amino acceptors.

Transamination of D or L methionine is thought to be part of a pathway to ethylene production in higher plants (cauliflower, tomato, apple, pea stem, banana, peanut), as well as in soil microorganisms (*Escherichia coli, Pseudomonas pisi, Pseudomonas aeruginosa, Bacillus mycoides, Acinetobacter calcoaceticus, Aeromonas hydrophila* B12E, *Rhizobium trifolii* N2P7, *Penicillium digitatum, Saccharomyces cerevisiae, Corynebacterium* D7F). Billington, D.C., et al., Biochem J. 182:827-836, (1978). In bacteria, L-methionine is typically used as the substrate in the ethylene production studies and broad specificity enzymes such as TyrB or AspC from *E. coli* are thought to be responsible for the transamination. However, Primrose, S.B., J. Gen. Microbiol. 95:159-65, (1976) and Primrose, S.B., J. Gen. Microbiol. 98:519-528, (1977) showed that *E*. *coli* strain SPA O (University of Warwick culture collection) produced nearly as much ethylene from D-methionine as from L-methionine in batch cultures. Because no broad specificity D-aminotransferase has been identified in *E. coli,* one possible explanation could be that the *E. coli* D-amino acid dehydrogenase (encoded by the *dadA* gene) converts the D-methionine to 4-methylthio-2-oxobutanoate. It is also possible that there is a methionine racemase in *E. coli*; however, no such enzyme has been described in the literature.

In contrast to *E. coli,* in cauliflower florets (mitochondrial extract preparations) and germinating peanut seeds production of ethylene was higher when D-methionine and pyruvate were supplied to the enzyme extract as compared to L-methionine and pyruvate. Mapson, L.W., et al., Biochem J. 115:653-661, (1969); Durham, J.I., et al., Phytochemistry 12:2123-2126, (1973). Therefore, the possibility of a combination of methionine racemase and an L-aminotransferase is not supported by the data. Dehydrogenase activity was ruled out by dialysis of cellular extracts of cauliflower; no NAD was present in the assay mixtures. Oxidase activity was ruled out as no consumption of oxygen was noted and there was no requirement for FAD. The D-methionine aminotransferase from peanut tissues was purified, shown to be dependent on PLP, and shown to be independent of L-methionine aminotransferase activity. There is a possibility that these D-methionine-pyruvate aminotransferases actually produce D-alanine as a byproduct (similar to the *Bacillus* enzymes described in Examples 2 and 3) and that the cells contain alanine racemase to recycle the D-alanine back to L-alanine (or an analogous amino donor). In either case, discovery of the broad specificity D-aminotransferase from higher plants is advantageous for development of processes that produce R,R monatin or S,R monatin.

### Experimental Overview

D-methionine aminotransferase is partially purified from cauliflower florets and germinating peanut embryos using standard chromatography protocols and a Pharmacia AKTA Explorer system. The protein sequences of homologous proteins are determined by LC/MS/MS fingerprinting techniques and database searching performed by Harvard Microchemistry facility. The coding regions of the plant genes are cloned from a cDNA library using standard PCR protocols or by synthesis of the gene as described in Example 10(*1*).

Alternatively, cDNA expression libraries are constructed (Stratagene, La Jolla, CA) from cauliflower tissue or peanut seeds grown in the presence of D-methionine (and producing ethylene). The libraries are transformed into *E. coli* methionine auxotrophs from the *E*. *coli* Genetic Stock Center (Yale) such as strains RC519 or AB1931. Plasmids of strains capable of growth on minimal media containing D-methionine contain the coding region of interest (see Example 4(*1*), an analogous screening technique).

Once the coding regions of interest are obtained and are expressed in a standard *E*. *coli* laboratory strain, the resulting gene products can be used in assays to produce R,R monatin, as described in Example 10(*3*), in place of the D-hydroxyphenylglycine aminotransferase, with the exception of the pH being 7.5 (the optimal pH for the aminotransferase). If the D-methionine aminotransferase has a strict requirement for D-amino acid donor substrates, the enzyme can be used to make R,R monatin as described in Example 2 and 3. The gene can be mutagenized and screened for increased activity as described in Example 10(*3*).

### Methods

### Isolation from Cauliflower

Four hundred grams of freshly picked cauliflower florets are extracted with 400 mL of a 4 °C sucrose/buffer solution (0.4 M sucrose and 0.1 M sodium phosphate buffer pH 7.4) by alternating soaking and mixing using a blender. Cell debris is removed by filtration with cheesecloth and the resulting solution is centrifuged at 40,000 x g for 30 minutes at 4 °C. The solid material (containing mitochondrial organelles) is resuspended in 20 mL 10 mM sodium phosphate buffer pH 7.4 and enzymes are extracted with 200 mL cold (-30 °C) acetone. The suspension is recentrifuged and the precipitate is dried using a Savant Speed Vac. The solid material is dissolved in 10 mM sodium phosphate buffer pH 7.4 and residual acetone is removed using a PD-10 column.

Aminotransferase activity is assayed by incubation of the enzyme preparation with 5 mM D-methionine, 1 mM pyruvate, 0.05 mM PLP and 2 mM EDTA in 0.1 M sodium phosphate buffer pH 7.4. Assays are performed at 25 °C for 16 hours. The 4-methylthio-2-oxobutanoate is measured by formation of the 2,4-dinitrophenylhydrazone derivative, using LC/MS (m/z of 328) and similar methodology described in Example 1. A 0.4% (w/v) solution of 2,4-dinitrophenylhydrazine in 2M sulfuric acid is prepared and a half volume is added to the assay mixture after incubation. The mixture is mixed with gentle shaking at 30 °C for 30 minutes and the precipitate is collected by centrifugation and analyzed by LC/MS. Protein fractions separated by standard chromatographic techniques are assayed for activity in a similar manner, but the co-product alanine is measured by the OPA post-column derivitization technique described in Example 1.

### Isolation from Peanut (Arachia hypogea L. cv. Starr)

The D-methionine aminotransferase enzyme from germinating peanut embryo homogenate (minus the cotyledons) is purified according to the method of Durham, J.I., et al., Phytochemistry 12:2123-2126, (1973). Reducing agents are used during the preparation of crude extracts to stabilize the enzymes and the cell debris is removed by centrifugation at 33,000 x g. A 35-50% ammonium sulfate fraction is further purified by incubation at low temperature and by removal of the proteins in the precipitate. The supernatant is further fractionated using acetone. The active pools are then further purified by gel filtration chromatography (Sephadex 200 G.E. Healthcare, Piscataway, NJ).

As protein fractions become enriched with the transaminase protein, 2D-gel electrophoresis is utilized to separate the enzyme of interest for microsequencing. After elucidation of homologous coding regions in plant sequences deposited at NCBI, the D-aminotransferase protein is produced recombinantly in *Escherichia coli* using standard molecular biology techniques. It is expected that the cellular extracts from cauliflower florets or peanut seeds or recombinantly produced homologous enzymes can be used in production of R,R monatin as described in Example 10(3) (if a stereoinverting transaminase) or Examples 2 and 3 (if a broad specificity D-aminotransferase).

### Example 12

### L-Alanine Aminotransferase/Alanine Racemase/D-Alanine Aminotransferase

Figure 8 illustrates the biosynthetic pathway for producing stereoisomerically-enriched R,R monatin from L-tryptophan using L-amino acid aminotransferases (such as L-aromatic aminotransferases, L-alanine-aminotransferases and/or L-tryptophan-aminotransferases), an R-specific aldolase, an alanine racemase and a D-alanine aminotransferase.

A tryptophan-specific aminotransferase is described in Example 6. Alternatively, *S. meliloti* and *R. sphaeroides* tyrosine (aromatic) aminotransferases are prepared as described in Example 1 from WO 03/091396 A2. *Comamonas testosteroni* ProA aldolase is prepared as described in Example 4 from WO 03/091396 A2. Total protein assays are done utilizing the Bio-Rad Protein Assay according to manufacturer's protocols (Hercules, CA). Alanine racemase is purchased from Sigma (St. Louis, MO) (catalog number A8936). D-alanine aminotransferase is purchased from BioCatalytics (Pasadena, CA) (catalog number AT-103).

L-alanine aminotransferases are widely distributed in eukaryotes, bacteria, and archaea. The following organisms have been identified (based on sequence homology) as containing an L-alanine aminotransferase (EC 2.6.1.2): *Arabidopsis thaliana, Ashbya gossypii, Azotobacter vinelandii, Bifidobacterium longum, Caenorhabditis elegans, Candida albicans, Candida glabrata, Chlamydomonas reinhardtii, Cryptococcus neoformans, Debaryomyces hansenii, Homo sapiens, Hordeum vulgare, Kluyveromyces lactis, Magnaporthe grisea, Medicago truncatula, Mus musculus, Neurospora crassa, Oryza sativa, Phanerochaete chrysosporium, Pinus taeda, Pseudomonas putida, Pyrococcus abyssi, Pyrococcus furiosus, Pyrococcus horikoshii, Rattus norvegicus, Saccharomyces cerevisiae, Schizosaccharomyces pombe, Takifugu rubripes, Trypanosoma cruzi, Vibrio cholerae, Vibrio parahaemolyticus, Vibrio vulnificus, Yarrowia lipolytica,* and *Zea mays.* Additionally, many aminotransferases have low-level alanine aminotransferase activity and given high levels of L-glutamate and pyruvate can convert it to L-alanine and α-ketoglutarate. An enzyme with low activity is improved with standard mutagenesis techniques, such as error-prone PCR and passage through mutagenic strains, or by directed evolution techniques. The gene for an L-alanine aminotransferase is cloned using publicly available sequences to design primers and using standard techniques to amplify, clone, express and purify the gene/enzyme.

The reaction mixtures (1 mL volume, run in duplicate) contain 100 mM potassium phosphate buffer (pH 8), 2 mM MgCl₂, 0.05 mM pyridoxal 5'-phosphate ("PLP"), 200 mM sodium pyruvate, 5 mM sodium α-ketoglutarate, approximately 280 µg/mL *S. meliloti* TatA supplied in a cellular extract (or other L-tryptophan specific aminotransferase) (as in Example 4 section (5), 100 µg of an L-alanine aminotransferase, 100 µL/mL of alanine racemase cellular extract or 1 mg/mL purified alanine racemase (Sigma), approximately 280 µg/mL of a broad specificity D-alanine aminotransferase supplied in a cellular extract (Examples 15 and 18 have examples of D-aminotransferases that could work for this reaction) and approximately 100 µg/mL of ProA aldolase provided as a cellular extract. Solid tryptophan is added at a concentration of 10.2 mg/ml. Negative controls are set up without alanine racemase. The samples are incubated at 30°C with gentle shaking for ~1 hour or overnight. The samples are centrifuged to remove precipitate, syringe filtered, and stored at -80°C prior to analysis for monatin using the LC/MS/MS method described in Example 1.

### Example 13

### Purification of R,R-Monatin from an Enzymatic Reaction Mixture

The product, R,R-monatin, was purified from the following reaction mixture. In 0.33 liter, 50 mM ammonium bicarbonate, pH 8.2, 4 mM MgCl₂, 0.05 mM pyridoxal phosphate ("PLP"), 200 mM sodium pyruvate, and 50 mM D-tryptophan were mixed at room temperature in a 500 mL glass bottle until the tryptophan dissolved. The liquid was flushed with nitrogen for several minutes and then 3.0 mg/mL Biocatalytics, Inc. (Pasadena, CA) Broad range D-transaminase (catalog # AT-103) and 0.1 mg/mL purified aldolase of SEQ ID NO:22 were added. The reaction mixture was stirred gently at room temperature. The aldolase was purified as described in Example 3. Additional aliquots of 50 mM D-tryptophan were added as a solid 15 hours and 22 hours after the mixture was initially prepared. The head space was flushed with nitrogen after each addition. All of the added tryptophan did not dissolve, but the concentration was maintained at about 50 mM. After 40 hours, the remaining solid tryptophan was filtered off. Analysis of the reaction mixture by post column fluorescence detection liquid chromatography (see Example 1) showed that the concentration of tryptophan in the solution was 49 mM and the concentration of monatin was 3.9 mM.

The product monatin was purified utilizing two ion exchange chromatography steps. The filtered reaction solution was first applied to a column of BioRad AG50W-X8 resin (140 mL; binding capacity of 1.7 meq/mL). The column was washed with 2 x 150 mL H₂O and then eluted with 1 M NH₄OH (1 x 450 mL, followed by 3 x 150 mL). The NH₄OH fractions were combined, neutralized with HCl and filtered successively through Whatman (Maidstone, England) glass microfibre filters and Gelman Sciences (Ann Arbor, MI) 0.45 µm filters. The clarified solution was then ultrafiltered using an Amicon (Millipore; Billerica, MA) ultrafiltration stirred cell (Model 8200) with a YM100 (MWCO 100 kDa). The filtrate from the ultrafiltration was evaporated to approximately 160 mL using a roto-evaporator with a tepid water bath. The liquid was again clarified by filtering through glass microfibre filters.

The resulting solution was applied to a 1 L Fast Flow DEAE Sepharose (Amersham Biosciences) column previously converted to the bicarbonate form by washing with 0.5 L 1 M NaOH, H₂O, and1.0 M ammonium bicarbonate, pH 8.3, followed by an additional washing using H₂O. The solution was loaded at <2 mL/min and the column was washed with water at 3-4 mL/min until the absorbance at 280 nm was <1. The R,R-monatin was eluted with 50 mM ammonium bicarbonate, pH 8.3 (2.5 L). This fraction was evaporated using a roto-evaporator with a tepid water bath. The resulting syrup was incubated at 4 °C for several days until crystals formed. The crystals were collected, washed with cold 100% ethanol and dried in a vacuum dessicator (0.38 g).

Analysis of the solid product for isomeric purity using FDAA derivitization, followed by LC/MS/MS multiple reaction monitoring, (see Example 1) showed that the sample was 96.3% R,R monatin and 3.7% S,R-monatin.

The sample was also analyzed for purity with respect to other organic compounds using the total monatin method (see Example 1). The UV absorbance was scanned from 200-500 nm using a Photodiode Array detector. Based upon the integrated peak areas, monatin accounted for 96.1% of the area (including both R,R and S,R peaks).

Analysis of the sample by post column fluorescence detection liquid chromatography showed that the amino acid composition of the sample was 98.8% monatin with trace amounts of tryptophan (1.2%) and alanine (0.02%).

Elemental analysis was performed at Midwest Microlab, LLC (Indianapolis, IN). This analysis indicated that the sample contained 1% non-combustible (inorganic) material by weight, and ammonium and bicarbonate residuals.

### Example 14

### Improvement of D-Aminotransferase Activity Retention During Purification

### Standard Procedure for the Purification of B. sphaericus HIS₆₋D-alanine Aminotransferase

Starting from a fresh culture plate (LB agar with 50 µg/mL kanamycin) of BL21(DE3)::*B*. *sphaericus dat* pET30a (Example 18), the cells were grown in 5 mL of Luria-Bertani broth ("LB") with 50 µg/ml kanamycin, at 37 °C and 225 rpm for 3 - 5 hours. Subsequently, the culture was transferred at 0.25% (v/v) into flasks containing Novagen Overnight Express System II solutions 1-6 (EMD Bioscience, Madison, WI) plus 50 µg/mL kanamycin. The cells were grown at 37°C and 225 rpm overnight (16-18 hours). When the OD₆₀₀ was approximately 8.0, the cells were harvested by centrifugation in a Beckman (Fullerton, CA) J25II centrifuge with a JS-16.25 rotor at 10,000 rpm for 10 minutes. The cell pellet was washed once with cold 50 mM EPPS buffer (pH 8.2), and the cells were centrifuged again. The washed cell pellet was harvested and used immediately or frozen at -80°C until needed for purification.

To prepare cell-free extract containing the *B. sphaericus* HIS₆-D-alanine aminotransferase (HIS₆-BsphDAT) protein, the cells were suspended in 3-4 volumes of 50 mM EPPS, pH 8.2 and then disrupted using a Microfluidics (Newton, MA) homogenizer (3 passes at 20,000 psi), maintaining the temperature of the suspension below 15°C. All subsequent purification steps were carried out at 4°C. The cell extract was centrifuged for 15 minutes at 15,000 x g to remove the cell debris. The supernatant was decanted and used immediately or frozen at -80°C. An aliquot of the cell free extract was applied either to Novagen HIS-Bind columns (catalog # 70971-4) or to a column of GE Healthcare (Piscataway, NJ) Chelating Sepharose™ Fast Flow resin (nickel(II) form) (at a ratio of 1.2 - 1.5 v/v) that had been previously equilibrated with 50 mM EPPS, pH 8.2, containing 200 mM sodium chloride. After loading the sample, the column was washed/eluted successively with 3 - 5 volumes of the equilibration buffer, 3 - 5 volumes of the equilibration buffer containing 25 mM imidazole, 3 - 5 volumes of the equilibration buffer containing 100 mM imidazole and 3 - 5 volumes of the equilibration buffer containing 500 mM imidazole. The HIS₆-BsphDAT protein eluted in the last wash. The 500 mM imidazole wash was concentrated 2 - 10X with Amicon (Billerica, MA) Centricon-70 or Ultra-15 centrifugal filter devices (MWCO 10 kDa). The imidazole and sodium chloride were removed by passage through disposable GE Healthcare PD10 desalting columns previously equilibrated with 50 mM EPPS, pH 8.2, containing 50 µM PLP.

The protein concentration of the desalted solution was determined using the Pierce BCA assay kit (Rockford, IL). The purity of each fraction and the level of expression in the cell free extract fraction were determined using a Bio-Rad (Hercules, CA) Experion Pro260 microcapillary chip system or by SDS-PAGE with 4-15% gradient gels. Typically, this procedure produces more than 300 mg of enzyme (from 600 mL of Overnight Express II culture) that is ∼90% pure as judged by the Experion software. Aliquots (1-5 mL) of the purified enzyme were stored at -80 °C until use.

### Improved Procedure

Cell-free extract was prepared as described above. His₆-BsphDAT protein was similarly purified with the following changes: all buffers used for cell disruption and protein purification contained 100 mM potassium phosphate, pH 7.8, with 50 µM PLP. The protein was purified exclusively with GE Healthcare Chelating Sepharose™ Fast Flow resin (nickel(II) form).

### Activity Assay

The formation of indole-3-pyruvate and alanine from tryptophan and pyruvate was assayed using the enzyme prepared by both purification procedures. The reaction mixtures contained 100 mM potassium phosphate, pH 7.8, 0.05 mM pyridoxal phosphate, 100 mM sodium pyruvate, 40 mM D-tryptophan, and 0.03 - 0.1 mg/mL of purified enzyme. The tryptophan was added as a solid. All components except the enzyme were mixed together and incubated at 30 °C until the tryptophan dissolved. The enzyme was then added and the reaction solution was incubated at room temperature. At predetermined time points, the reactions were sampled and the samples immediately stored on ice and diluted for alanine analysis by the post-column fluorescence detection liquid chromatography method described in Example 1. Table 34 below lists the specific activity of the enzyme preparations as the concentration of alanine formed per mg of enzyme per minute.

**Table 34: Effect of Improved Purification Procedure on Enzyme Activity**

| Enzyme Preparation | Specific Activity (µmole Alanine(mg)⁻¹(min)⁻¹) |
|---|---|
| HIS₆-BsphDAT purified without 50 µM PLP | 2.9 |
| HIS₆-BsphDAT purified with 50 µM PLP | 14.2 |

The results shown in Table 34 indicate that the use of pyridoxal phosphate during the purification process resulted in an enhanced activity.

### Example 15

### Cloning of Two Novel Bacillus D-Amino Acid Aminotransferases

Several *Bacillus* D-amino acid aminotransferases (EC 2.6.1.21, also known as D-alanine aminotransferase or D-aspartate aminotransferase) were produced recombinantly for use in coupled assays for production of R,R monatin, as described in Example 18. These enzymes are homologous to D-aminotransferases described previously for production of monatin (U.S. Publication No. 20040063175 and U.S. Publication No. 2005282260). An approach used for the selection of strains that could be candidates containing novel D-amino acid aminotransferases ("DAATs") was to review the list of *B. sphaericus* strains deposited in ATCC and analyze some that were previously deposited under different species names. The following organisms were ordered from the ATCC: ATCC 4978 - *Bacillus sphaericus* originally deposited as *Bacillus rotans* and ATCC 7063 *- Bacillus sphaericus* originally deposited as *Bacillus serositidis* and ATCC 21538 - *Bacillus sphaericus* originally deposited as *Bacillus circulans.* Known DAAT protein sequences from *Bacillus sphaericus, Bacillus halodurans, Geobacillus stearothermophilus, Bacillus cereus, Bacillus subtilis,* and *Bacillus licheniformis* were aligned to obtain sequence regions that were conserved in the various DAAT proteins. Primers were designed in the regions of protein sequence conservation and used for polymerase chain reactions ("PCR") amplification of DAAT gene sequences from the ATCC strains mentioned above.

Five PCR primers were designed based on conserved regions in alignment of published *Bacillus* DAAT sequences (see alignment in FIG. 9).

### Polymerase Chain Reaction Protocol

Primers were designed as mentioned above based on conserved regions in an alignment of DAATs. Oligonucleotide Primer Sequences are indicated below: 5' GAAGACCGTGGTTATCAATTT 3' (SEQ ID NO:65) (forward primer), 5' GATGGTATTTACGAAGTAATC 3' (SEQ ID NO:66) (forward primer), 5' AGATTTAATATCACAACGTAAC 3' (SEQ ID NO:67) (reverse primer), 5' GCCAAGTAAAATTTAAGATTTA 3'(SEQ ID NO:68) (reverse primer), 5' ATTTGCTGGGTGCGTATAAAG 3' (SEQ ID NO:69) (reverse primer). Expected sizes of PCR fragments based on primer combinations alignment with known DAATs: SEQ ID NO:65 and SEQ ID NO:67 - approx. 380 bp, SEQ ID NO:65 and SEQ ID NO:68 - approx. 395 bp, SEQ ID NO:65 and SEQ ID NO:69 - approx. 534 bp, SEQ ID NO:66 and SEQ ID NO:67 - approx. 336 bp, SEQ ID NO:66 and SEQ ID NO:68 - approx. 346 bp, SEQ ID NO:66 and SEQ ID NO:69 - approx. 510 bp.

Combinations of the above primers were used for colony PCR from the following ATCC strains: ATCC 4978 - *Bacillus sphaericus,* originally deposited as *Bacillus rotans;* ATCC 7063 - *Bacillus sphaericus,* originally deposited as *Bacillus serositidis;* and ATCC 21538 - *Bacillus sphaericus,* originally deposited as *Bacillus circulans.*

The three above mentioned strains were grown on nutrient agar at 30°C. A single colony was scraped from plates and resuspended in 25 µL sterile distilled water. The cells were lysed at 96 °C for 10 minutes. PCR was carried out as follows: per 50 µL reaction, 5 µL lysed cells, 0.8 µL of each primer, 2 µL dNTPs, 0.8 µL Expand High Fidelity Polymerase (Roche, Indianapolis, IN) and 1X Expand™ buffer were added. A 3 minute hot start was done at 94°C, followed by 15 cycles of 94 °C for 30 seconds, 40 °C for 45 seconds, and 72 °C for 2 minutes. Fifteen more cycles were done with an increased annealing temperature of 45 °C. Lastly, a chain extension step was done for seven minutes at 72 °C. Several primer combinations gave expected PCR product sizes for the above strains. PCR products were cloned using the Zero Blunt TOPO^{®} cloning kit as per manufacturers' protocols (Invitrogen) and sequenced by dideoxy chain termination DNA sequencing at Agencourt BioScience Corporation (Beverly, MA). Sequences at both the DNA and amino acid level were aligned with the *B. sphaericus* DAAT sequence. Valid DAAT/DAT sequences were obtained from all three strains, ATCC 4978, ATCC 7063 and ATCC 21538. Two specific strains, ATCC 4978 and ATCC 7063 gave PCR products which when translated yielded protein sequences with distinct amino acid residue changes when compared to the *B. sphaericus* D-aminotransferase sequence.

Genome walking was carried out to obtain the complete gene sequences for the ATCC 4978 and ATCC 7063 strains. Strain ATCC 4978 was grown up in nutrient broth at 30 °C. Strain ATCC 7063 was grown up on nutrient agar. Genomic DNA was prepared from each strain using the Gentra Kit (Gentra Systems, Minneapolis, MN) as per manufacturer's protocols. Four libraries were constructed for each strain as per manufacturer's protocols (BD GenomeWalker™ Universal Kit, Clontech, www.Clontech.com). Gene-specific primers were designed as per GenomeWalker™ manufacturer's protocols based on sequences obtained using conserved primer combinations (see above), allowing for a few hundred homologous base pair overlap with original product. These gene-specific primers were subsequently used with GenomeWalker™ adaptor primers for PCR of upstream and downstream sequences to complete DAT ORFS.

The gene-specific oligonucleotide primer sequences are indicated below:
4978 DAT GSP1 Upstrm 5' GACATGCTCCTCCGCTGTAAATAATTCACC 3' (SEQ ID NO:70)
4978 DAT GSP1 Dwnstr 5' CCCTGGTGATGAAGTGAAGCCAGTATTAAC 3' (SEQ ID NO:71)
4978 DAT GSP2 Upstrm 5' ATCGCCAAATTGATAACCACGGTCTTC 3' (SEQ ID NO:72)
4978 DAT GSP2 Dwnstr 5' ACGTCCCGTAGCAAACTTTGAAAAAGGTGT 3' (SEQ ID NO:73)
7063 DAT GSP1 Upstrm 5' TGCATAGAATCGGTCGATATGTTCAGTAGC 3' (SEQ ID NO:74)
7063 DAT GSP1 Dwnstr 5' GCGGAGAAACGATTACAGAAGGTTCTTCAA 3' (SEQ ID NO:75)
7063 DAT GSP2 Upstrm 5' GTCACCAAATTGATAACCACGGTCTTC 3' (SEQ ID NO:76)
7063 DAT GSP2 Dwnstr 5' GGTGTACTTTATACGCACCCAGCAAAT 3' (SEQ ID NO:77)

Adaptor oligonucleotide primer sequences:
AP1 5' GTAATACGACTCACTATAGGGC 3' (SEQ ID NO:78)
AP2 5' ACTATAGGGCACGCGTGGT 3' (SEQ ID NO:79)

Primary GenomeWalker™ PCRs were carried out as follows: per 50 reaction, 2.5 µL of DNA library, 2 µL of each primer (AP1 (SEQ ID NO:78) and the appropriate GSP1), 1.5 µL dNTPs, 1X XL PCR buffer, 1 mM magnesium acetate, and 1 µL RT^{TH} polymerase (Roche, Indianapolis, IN) were added. A 3 minute hot start was done at 94°C, followed by 10 cycles of 94 °C for 30 seconds, 55 °C for 30 seconds, and 68 °C for 1 minute. Twenty more cycles were done with a decreased annealing temperature of 48°C. Lastly, a chain extension step was done for seven minutes at 68 °C. Secondary Genome Walker™ PCRs were carried out as follows: per 50 µL reaction, 1.0 µL (of a 1:50 dilution) of the primary PCR reaction, 2 µL of each primer (AP2 (SEQ ID NO:79) and the appropriate GSP2), 1.5 µL dNTPs, 1X XL PCR buffer, 1 mM magnesium acetate, and 1 µL RT^{TH} polymerase were added. A 3 minute hot start was done at 94 °C, followed by 10 cycles of 94 °C for 30 seconds, 55 °C for 30 seconds, and 68 °C for 1 minute. Fifteen more cycles were done with a decreased annealing temperature of 48 °C. Lastly, a chain extension step was done for seven minutes at 68 °C.

Several libraries gave PCR products ranging in size from ∼200 bp to ∼1.5 Kb. PCR products were TOPO cloned (as above) and sequenced by dideoxy chain termination DNA sequencing at Agencourt BioScience Corporation (Beverly, MA); these new sequences were aligned with initial sequences obtained using conserved primer combinations and start and stop codons were identified. In this way the DAAT complete ORFs were obtained. New primer pairs were designed (with restriction sites for cloning) based on the specific complete DAAT sequences to PCR the entire DAAT gene from ATCC strains 4978 and 7063 individually.

Oligonucleotide Primer Sequences are indicated below:
ATCC4978DAATNde1F 5' GGCCTTGGCATATGAGTTATAGCTTATGGAATGACC 3' (SEQ ID NO:80)
ATCC4978DAATBamH1R 5' GGCCTTAAGGATCCTTATGCGCGAATACCTTTTGGG 3' (SEQ ID NO:81)
ATCC7063DAATNde1F 5' GGCCTTGGCATATGAGCTACACTTTATGGAATGA 3' (SEQ ID NO: 82)
ATCC7063DAATBamH1R2a 5' GGCCAAGGATCCGCTACCCACTAATCATTAGA 3' (SEQ ID NO:83)

The coding regions of the ATCC 4978 and ATCC 7063 DAAT genes were amplified using the following PCR protocol. In a 50 µL reaction, 3 µL genomic DNA, 0.8 µL of each primer, 2 µL dNTPs, 0.8 µL Expand High Fidelity Polymerase (Roche, Indianapolis, IN), 1X Expand™ buffer with Mg, and 0.2 µL Pfu polymerase (Stratagene, La Jolla, CA) were added. The thermocycler program used included a hot start at 94 °C for 3 minutes, followed by 8 repetitions of the following steps: 94 °C for 30 seconds, 50 °C for 30 seconds, and 72 °C for 90 seconds. Twenty-two subsequent cycles were done with an annealing temperature of 58 °C. Lastly, a chain extension step was done for seven minutes at 72 °C. Clean PCR products of the correct size (approximately 850 bp) were obtained for both strains.

The PCR products for ATCC 4978 and ATCC 7063 DAAT genes were purified using the Qiagen QIAquick PCR purification kit (Valencia, CA), and digested with *Nde*I and *Bam*HI in *Bam*HI buffer (New England Biolabs, Ipswich, MA). *Nde*I and *Bam*HI digested vectors (pET28 and pET30) and insert were purified using the Qiagen QIAquick Gel Extraction Kit. The ligations were done using the Roche Rapid DNA Ligation Kit (Roche) and purified using the QIAquick PCR purification kit. The ligations were transformed into *Escherichia coli* DH10B using a 0.2 cm cuvette and a Bio-Rad Gene Pulser II system as described in the Bio-Rad electroporation manual. The cells were allowed to recover in 900 µL SOC medium for 30 minutes at 37°C at 225 rpm. The cells were plated on LB-agar plates containing kanamycin (50 µg/mL). The plasmid DNA was purified using the Qiagen spin miniprep kit and screened for the correct inserts by PCR and restriction digestion with *Nde*I and *Bam*HI*.* The sequences of plasmids that appeared to have the correct insert were verified by dideoxy chain termination DNA sequencing at Agencourt BioScience Corporation (Beverly, MA). Sequence analyses verified the coding sequence for DAAT genes from ATCC 4978 and ATCC 7063, which produced the DNA sequences of SEQ ID NO:84 (ATCC 4978 DAAT DNA sequence) and SEQ ID NO:85 (ATCC 7063 DAAT DNA sequence) and the amino acid sequence of SEQ ID NO:86 (ATCC 4978 DAAT amino acid sequence) and SEQ ID NO:87 (ATCC 7063 DAAT amino acid sequence).

The alignments of the two novel DAATs from ATCC 4978 and ATCC 7063 with the *B. sphaericus* DAAT (cloned in Example 18) shown in FIG. 10.

We obtained novel D-aminotransferases from strains ATCC 4978 and ATCC 7063 with protein sequences that have distinct amino acid residue changes when compared to the *B. sphaericus* D-aminotransferase. The DAATs from ATCC 4978 and ATCC 7063 have only 72% and 67% identity with the DAAT from *B. sphaericus* (ATCC 10208). While both of these strains are currently listed as *B. sphaericus* in the ATCC, they were deposited as *B. rotans* and *B. serositidis.* Based on the sequence alignments and the highlighted differences between these two novel DAATs and the DAAT from *B. sphaericus,* a number of candidate residues are identified that can be evaluated for their role (individually or in combination) in increasing DAAT activity for R,R monatin biosynthesis, in these, as well as other DAAT sequences.

### Example 16

### Gene Expression and Assays for ATCC 4978 and ATCC 7063 DAAT Proteins

The novel DAATs from ATCC 4987 and ATCC 7063, as described in Example 15, (in pET vectors) were transformed into the *E. coli* expression host BL21(DE3) (Novagen, Madison, WI). The cultures were grown using the protocols described above and the plasmids were isolated using Qiagen miniprep kit and analyzed by restriction digestion, as described above, to confirm plasmid identity.

Induction of the DAAT gene was typically performed in LB medium containing kanamycin (50 µg/mL). The cells were grown to an OD₆₀₀ of 0.4-0.8 at 37 °C, induced with 0.1 mM IPTG (isopropyl thiogalacatoside) and sampled at 3-4 hours post induction. Cell extracts were prepared according to the protocol accompanying the Novagen BugBuster™ reagent (with benzonase nuclease and Roche complete protease inhibitor cocktail added). Soluble proteins were obtained at the predicted molecular weight, as judged by SDS-PAGE, for both ATCC 4978 and ATCC 7063 gene products in pET vectors. Higher levels of soluble protein were observed using constructs without His tags (pET 30). Soluble proteins in the cellular extracts were separated on a BioRad Laboratories Experion Automated Electrophoresis Station (Hercules, CA) and analyzed for concentration and percent expression using the Experion Software version 1.1.98.0.

Protein extracts from cells with untagged (pET30) constructs were analyzed for D-aminotransferase activity by following production of alanine from pyruvate and D-tryptophan (or R,R monatin) using the following protocol. Duplicate 500 µL reactions were, unless specified below, carried out in 100 mM potassium phosphate buffer (pH 7.5), 80 µM pyridoxal phosphate, 25 mM sodium pyruvate, and 50 mM D-tryptophan or R,R monatin. The reactions were initiated by the addition of cell free extracts (4978 or 7063) or purified enzyme (*B. sphaericus)* and were incubated 15 minutes-2 hours at 30 °C, with mild shaking. Approximately the same level of total protein was added (1.0 mg), unless specified below, in each assay for comparative purposes. Purified *B. sphaericus* (ATCC number 10208) aminotransferase was used as a benchmark enzyme. Formic acid was added to a final concentration of two percent to stop the reaction and the precipitated protein was removed by centrifugation. Control reactions without added protein were also performed. Alanine was detected using OPA derivatization as described in Example 1. The averaged results of the duplicate reactions are shown in Tables 35 and 36 below.

**Table 35: Transamination Activity of ATCC 4978 and ATCC 7063 D-Aminotransferases (15 min)**

| D-aminotransferase | Alanine (mM) D-Tryptophan as Substrate | Alanine (mM) R,R Monatin as Substrate |
|---|---|---|
| ATCC 4978 | 7.78 | 0.32 |
| ATCC 7063 | 0.28 | 0.025 |
| *B. sphaericus* (untagged) | 11.93 | 3.57 |

**Table 36: Transamination Activity of ATCC 4978 and ATCC 7063 D-Aminotransferases (2 Hours)**

| D-aminotransferase | Alanine (mM) D-Tryptophan as Substrate | Alanine (mM) R,R Monatin as Substrate |
|---|---|---|
| ATCC 4978 | 16.46 | 2.33 |
| ATCC 7063 | 2.51 | 0.21 |
| *B. sphaericus* (untagged) | 13.73 | 12.23 |

Thus, we demonstrated that the D-amino acid aminotransferases from ATCC 4978 and ATCC 7063 indeed possessed D-aminotransferase activity and have the capability to make R,R monatin. The activity of the ATCC 4978 DAAT was higher than that observed for the ATCC 7063 DAAT. Quantitative comparison between 4978 and *B. sphaericus* could not be made since 4978 was unpurified.

### Example 17

### Production of R,R Monatin using the DAAT from ATCC 4978

The aminotransferase from ATCC 4978 was also tested for ability to produce monatin from D-tryptophan (as in Example 3). The following were added per 1 mL of reaction mixture: approximately 50 µg aldolase (*C. testosteroni* ProA aldolase or the aldolase of SEQ ID NO:22, purified), 4 mM MgCl₂, 50 mM D-tryptophan (supplied as solid), 1.0 mg D-aminotransferase, 100 mM sodium pyruvate, 100 mM potassium phosphate buffer pH 7.5, and 0.05 mM PLP. Experiments were run in duplicate, with negative controls in which no aminotransferase was added. Samples were incubated for various lengths of time at 30°C with gentle shaking. The only stereoisomers detected when producing monatin using these methods are R,R and S,R. The total monatin and percent R,R monatin were detected as described in Example 1 are listed in Tables 37-39 below. The results shown in each of Tables 37-39 is the average value from duplicate reactions.

**Table 37: Comparison of B. sphaericus and ATCC 4978 D-Aminotransferases for Production of Monatin using Approximately 50 µg C. testosteroni ProA**

| D-Aminotransferase | Total Monatin (mg per g DAT protein) 15 Minutes | Total Monatin (mg per g DAT protein) 30 Minutes | Total Monatin (mg per g DAT protein) 1 Hour | Total Monatin (mg per g DAT protein) 2 Hours |
|---|---|---|---|---|
| ATCC 4978 | 419.3 | 598 | 1017 | 1348 |
| *B. sphaericus* (tagged) | 46.5 | 128 | 232 | 241 |

**Table 38: Comparison of B. sphaericus and ATCC 4978 D-Aminotransferases for Production of Monatin using Approximately 50 µg C. testosteroni ProA**

| D-Aminotransferase | % R,R Monatin 15 Minutes | % R,R Monatin 30 Minutes | % R,R Monatin 1 Hour | % R,R Monatin 2 Hours |
|---|---|---|---|---|
| *ATCC 4978* | 48.9 | 38.4 | 34.4 | 33.25 |
| *B. sphaericus* (tagged) | 72.3 | 63.4 | 56.1 | 53.5 |

**Table 39: Comparison of B. sphaericus and ATCC 4978 D-Aminotransferases for Production of Monatin using Approximately 50 µg of the Aldolase of SEQ ID NO:22**

| D-Aminotransferase | Total Monatin (mg per g DAT Protein) 2 Hours | % R,R Monatin 2 Hours |
|---|---|---|
| *ATCC 4978* | 501 | 92.1 |
| *B. sphaericus* (tagged) | 201 | 95.6 |

Thus, we demonstrated that the D-amino acid aminotransferase from ATCC 4978 has the capability to make R,R monatin. The activity of the ATCC 4978 DAAT, when comparing total monatin production in terms of mg monatin per gram protein, was higher than that observed for the *B. sphaericus* DAAT. The use of an R-specific aldolase of SEQ ID NO:22 clearly made an improvement in percentage of R,R monatin formed in comparison to the amount of total monatin produced.

### Example 18

### Cloning of Published Bacillus D-Amino Acid Aminotransferases

Several *Bacillus* D-amino acid aminotransferases (EC 2.6.1.21, also known as D-alanine aminotransferase or D-aspartate aminotransferase) were produced recombinantly for use in coupled assays for production of R,R monatin. These enzymes are homologous to D-aminotransferases described previously for production of monatin (U.S. Publication No. 20040063175 and U.S. Publication No. 2005282260).

### Strains

*B. sphaericus* (ATCC number 10208) and *B. licheniformis* (ATCC 10716) were grown on Nutrient Agar at 30 °C overnight. Groups of colonies were placed in 100 µL of sterile water and heated for 5 minutes at 95 °C, to disrupt the cells. Three µL was used in subsequent Polymerase Chain Reaction ("PCR") amplifications. Genomic DNA was ordered for *B. halodurans* (ATCC number BAA-125D) and resuspended in water to a concentration of 100 ng/µL. *Bacillus cereus* genomic DNA (ATCC numbers 1-987D and 14579D) was ordered for cloning as well.

### Polymerase Chain Reaction Protocol

Primers were designed for the *B. sphaericus dat* gene for cloning into pET 28b and pET 30a vectors (Novagen, Madison, WI), using the *Nco*I and *Bam*HI sites. The pET30 construct contains an N-terminal His-tag and S-tag, whereas the pET 28 construct is untagged.

### Bacillus sphaericus dat primers:

N term: 5'-GATATACCATGGCATACTCATTATGGAATG-3' (SEQ ID NO:88) and C term: 5'-GTTATCGGATCCTTAGGCATTAATTGAAATTG-3' (SEQ ID NO:89).

The *B. licheniformis* primers and *B. halodurans* primers were designed for cloning into pET 28b and pET 30a vectors using *Nde*I and *Bam*HI sites. The pET30 constructs were untagged in this case, whereas the pET 28 constructs contain a small N-terminal his-tag.

### B. licheniformis dat primers:

N term 5'-GGCCGGTTCATATGAAAGTTCTTTTTAACGGC (SEQ ID NO:90) and C term: 5'-CCTTCCGGATCCTTAAACCGTTTTGGCTGTCT-3' (SEQ ID NO:91)

### B. halodurans primers:

N term 5'-GATATACATATGGATTATTGCCTTTACCAA-3' (SEQ ID NO:92) and C term: 5'-GAATCCGGATCCTCACTGCTTCATCGCTGTTTG-3' (SEQ ID NO:93)

Primers were designed for the *B. cereus* coding sequences. One set of primers yielded the sequence listed in NCBI as accession AE016877 gi:29899096 5138634... 5139506 (873 bp). One set of primers yielded a product with an additional 12 bp upstream, similar to the *B. thuringiensis* predicted *dat* NCBI accession number AE017355 gi:49328240 4965653... 4966537 (885 bp). Both sets of primers were designed with *Nde*I for the N-terminal region and *Bam*HI restriction site for the C-terminal region. The primers were designed for cloning into pBAD-TOPO TA cloning.

### B. cereus primers:

N term 5'-TAAGAGGAATAACATATGGCATACGAAAGATTT-3' (SEQ ID NO:94) and C-term 5'-GAATTCGGATCCTTAAGAAGATGACATATTGG-3' (shorter PCR product) (SEQ ID NO:95)
N term 5-TAAGAGGAATAACATATGGGATCGAAATTGGCA-3' (longer PCR product) (SEQ ID NO:96)

The coding regions of the *B. sphaericus, B. halodurans,* and *B. licheniformis dat* genes were amplified using the following PCR protocol. In a 50 µL reaction, 3 µL template (2 µL for genomic DNA), 1.6 µM of each primer, 0.25 mM each dNTP, 3.5 U Expand High Fidelity Polymerase (Roche, Indianapolis, IN), and 1X Expand™ buffer with Mg were used. The thermocycler program used included a hot start at 94 °C for 3 minutes, followed by 8 repetitions of the following steps: 94 °C for 30 seconds, 52 °C for 30 seconds, and 72 °C for 2 minutes. Twenty-two subsequent cycles were done with an annealing temperature of 58 °C. After 30 cycles the sample was maintained at 72 °C for 7 minutes and then stored at 4°C. Clean PCR products of the correct size were obtained (approximately 850 bp for the *dat* gene).

*Geobacillus stearothermophilus dat* (accession number J04460 gi: 142541), which codes for protein accession number AAA22252 (gi:142542) was constructed using assembly PCR techniques. The source of this gene/protein is often described as *Bacillus sp.,* thermostable *Bacillus* species, or *Bacillus* YM-1. The assembly process is as follows: 43 oligonucleotides (40 mers) were ordered from IDT based on the gene sequence above and its complementary DNA sequence, with 20 basepair overlaps between the sense and antisense strands. The primers were diluted to 250 µM in water and 5 µL of each primer was mixed together in a microfuge tube. PCR was carried out as follows: per 100 µL reaction, 1.5 µL of the primer pool, 4 µL dNTPs, 1X XL PCR buffer, 1 mM magnesium acetate, 2 µL r*Tth* polymerase (Roche, Indianapolis, IN), and 0.25 µL Pfu polymerase (Stratagene, La Jolla, CA) were added. A 3 minute hot start was done at 94 °C, followed by 15 cycles of 94 °C for 30 seconds, 40 °C for 30 seconds, and 68 °C for 15 seconds. Ten more cycles were done with an increased annealing temperature of 44°C and an extension time of 30 seconds (at 68 °C). Ten more cycles were performed at an annealing temperature of 48 °C and an extension time of 75 seconds. Lastly, a chain extension step was done for seven minutes at 68 °C. A secondary PCR was done using the following primers, designed for cloning with *Nde*I (N-term) and *Bam*HI (C-term):
N-term-5'- GGCCTTGGCATATGGGATACACTTTATGGAATGACC-3' (SEQ ID NO:97) and
C-term-5'- TTGGAACCGGATCCTTATATATGAAGCGGTTTTGG-3' (SEQ ID NO:98)

The PCR contained per 100 µL, 2.5 µL of the primary reaction, 0.4 µL of each primer, 3 µL dNTPs, 1X XL PCR buffer, 1 mM magnesium acetate, 2 µL r*Tth* polymerase, and 0.25 µL Pfu polymerase. A 3 minute hot start was done at 94°C, followed by 10 cycles of 94 °C for 30 seconds, 42 °C for 30 seconds, and 68 °C for 90 seconds. Fifteen more cycles were done with an increased annealing temperature of 48 °C, and lastly a chain extension step was done for seven minutes at 68 °C. A third PCR reaction was done using template from the second PCR, and using the same conditions as the second PCR reaction. A product of approximately 900 bp was visible on an agarose gel.

### Cloning

The PCR product for *B. sphaericus* DAT was purified using the Qiagen QIAquick PCR purification kit (Valencia, CA) and digested with *Bam*HI and *Nco*I in BamHI buffer (New England Biolabs, Ipswich, MA). The digested vectors (pET28 and pET30) and insert were purified using the Qiagen QIAquick Gel Extraction Kit. The ligations were done using the Roche Rapid DNA Ligation Kit (Roche) and purified using the QIAquick PCR purification kit. The ligations were transformed into *Escherichia coli* DH10B using a 0.2 cm cuvette and a Bio-Rad Gene Pulser II system as described in the Bio-Rad electroporation manual. The cells were allowed to recover in 900 µL SOC medium for 30 minutes at 37 °C at 225 rpm. The cells were plated on LB-agar plates containing kanamycin (25 µg/mL). The plasmid DNA was purified using the Qiagen spin miniprep kit and screened for the correct inserts by restriction digest with *Bam*HI and *Nco*I*.* The sequences of plasmids that appeared to have the correct insert were verified by dideoxy chain termination DNA sequencing at Agencourt BioScience Corporation (Beverly, MA). Sequencing verified the coding sequence found in NCBI accession number AF081278 Region: 134..985 (gi: 3513754), which produces a protein with amino acid sequence as listed in accession number AAC33964 (gi: 3513755).

The PCR products for *B. licheniformis* DAT (∼850 bp) and *G. stearothermophilus* were gel purified and cloned using the Zero Blunt TOPO^{®} cloning kit as per manufacturers' protocols (Invitrogen). The plasmids were transformed into TOP 10 chemically competent cells for initial screening. The plasmid DNA was screened by restriction digest and the sequences were verified to match the coding sequence found in NCBI. For *B. licheniformis,* the sequence matched accession number U26947 region 247..1098 (gi:857560), which produces a protein with an amino acid sequence as listed in accession number P54692 (gi:1706292), with the exception of one silent mutation at position 429 from A to G. For G. *stearothermophilus,* the sequence matched the accession number listed above. The coding regions were subcloned by restriction digest (*Nde*I*lBam*HI), ligated into the pET vectors, and transformed into electrocompetent DH10B cells for amplification.

The PCR product for *B. halodurans* DAT was gel purified and digested with *Nde*I and *Bam*HI and ligated into pET 28 and pET 30 vectors as above. Amplification of the vector was done in DH10B cells. The miniprep DNA was screened by PCR and the sequence was verified. The gene sequence can be found in accession number NC_002570 (gi:57596592) 2934903..2935754 coding for a protein with amino acid sequence listed in accession number NP_243677(gi:15615374).

The *B. cereus* coding sequences were amplified using a typical PCR protocol and cloned according to manufacturer's protocols (Invitrogen).

### Gene Expression and Assays

Plasmid DNA was subcloned into *E. coli* expression host BL21(DE3) (Novagen, Madison, WI) for constructs in pET vectors. The cultures were grown and the plasmids were isolated using Qiagen miniprep kit, and analyzed by restriction digest to confirm identity. Induction was typically performed in LB medium containing kanamycin (50 µg/mL). The cells were grown to an OD₆₀₀ of 0.4-0.8 at 37 °C, induced with 0.1 mM IPTG (isopropyl thiogalacatoside) and sampled at 3-4 hours post induction. The cell extracts were prepared according to the protocol accompanying the Novagen BugBuster™ reagent (with benzonase nuclease and Roche complete protease inhibitor cocktail added). High levels of soluble protein were obtained at the predicted molecular weight, as judged by SDS-PAGE, for both *B*. *halodurans* gene products, both *B. sphaericus* gene products, both G. *stearothermophilus* gene products, and the untagged *B. licheniformis* gene product. For reactions in which purified protein was used, the His-tagged gene products were purified using His-Bind cartridges following manufacturer's protocols (Novagen, Madison, WI). The eluent fractions were desalted on PD-10 (Amersham Biosciences, Piscataway, NJ) columns and eluted in 25-100 mM potassium phosphate buffer, pH 7.5. Total protein assays were done using the Pierce BCA kit, and percent expression was estimated from SDS-PAGE. Alternatively, the soluble proteins in the cellular extracts were separated on a BioRad Laboratories Experion Automated Electrophoresis Station and analyzed for concentration and percent expression using the Experion Software version 1.1.98.0. The pBAD-TOPO constructs containing the *B. cereus* genes were expressed as recommended by Invitrogen, but the levels of expression of the DAATs was such that the recombinant protein could not be distinguished from the other proteins during SDS-PAGE analysis.

The cell extracts were analyzed for D-aminotransferase activity by following production of alanine from pyruvate and D-tryptophan (or R,R monatin) using the following protocol. Duplicate one mL reactions were typically carried out in 100 mM potassium phosphate buffer (pH 7.5), 50 µM pyridoxal phosphate, 25 mM sodium pyruvate, and 50 mM D-tryptophan or R,R monatin. The reactions were initiated by the addition of cell free extracts or purified enzyme and were incubated 15 minutes-overnight at 30 °C, with mild shaking. Approximately the same level of D-aminotransferase was added (typically around 0.5 mg) in each assay for comparative purposes. AT-103 (BioCatalytics) was used as a positive control (or benchmark). Formic acid was added to a final concentration of two percent to stop the reaction and the precipitated protein was removed by centrifugation. Control reactions, without added protein, were also performed. Zero time points were also used as negative controls. Alanine was detected using OPA derivatization as described in Example 1.

The aminotransferases were also tested for their ability to produce monatin from D-tryptophan (as in Example 3). The following were added per 1 mL of reaction mixture: approximately 50-100 µg aldolase (typically *C. testosteroni* ProA aldolase, purified), 4 mM MgCl₂, 50 mM D-tryptophan (supplied as solid), 0.5-2 mg D-aminotransferase, 200 mM sodium pyruvate, 100 mM potassium phosphate buffer pH 7.5, and 0.05 mM PLP. Experiments were run in duplicate, with negative controls in which no aminotransferase was added. The samples were incubated 1 hour, 2 hours, and overnight (17-20 hours) at 30 °C with gentle shaking. The only stereoisomers detected when producing monatin using these methods are R,R and S,R. The percent R,R is listed below, and was determined by reversed-phase LC peak area. The results of the transamination activity of *B. sphaericus, B. licheniformis,* and *B. halodurans* D-aminotransferases after 1 hour is shown in Table 40 below. The data was normalized to 0.5 mg of the D-aminotransferase per mL.

**Table 40: Transamination Activity of B. sphaericus, B. licheniformis, and B. halodurans D-Aminotransferases**

| D-Aminotransferase | Alanine (mM) D-Tryptophan as Substrate | Alanine (mM) R,R Monatin as Substrate |
|---|---|---|
| *B. halodurans* (tagged) | 15.5 | 1.3 |
| *B. halodurans* (untagged) | 17.5 | 1.4 |
| *B. licheniformis* (untagged) | 28.4 | 0.21 |
| *B. sphaericus* (untagged) | 29.0 | 22.5 |
| *B. sphaericus* (tagged) | 17.1 | 12.0 |

The production of monatin using *B. sphaericus, B. licheniformis,* and *B. halodurans* D-aminotransferases is shown in Table 41 below. Each reaction contained approximately 90 µg *C. testosteroni* Pro A. The data for the total monatin produced was normalized to the use of 0.5 mg of the D-aminotransferase.

**Table 41: Comparison of B. sphaericus, B. licheniformis, and B. halodurans D-Aminotransferases for Production of Monatin**

| D-Aminotransferase | Total Monatin (ppm) 3 Hours | Total Monatin (ppm) Overnight | % R,R 3 Hours | % R,R Overnight |
|---|---|---|---|---|
| *B. halodurans* (tagged) | 3.2 | 13.7 | 100 | 99.3 |
| *B. halodurans* (untagged) | 4 | 15.5 | 100 | 99.3 |
| *B. licheniformis* (untagged) | 0.6 | 8.1 | 100 | 29.3 |
| *B. sphaericus* (untagged) | 279.6 | 577.6 | 61.55 | 65.7 |
| *B. sphaericus* (tagged) | 111.2 | 246 | 61.0 | 63.1 |

The *B. sphaericus* D-aminotransferase (untagged) had the highest activity for production of monatin from D-tryptophan, but the *B. halodurans* enzyme had much higher selectivity for R-MP versus S-MP than the other enzymes, resulting in higher stereopurity of R,R monatin. The *B. cereus* cell extracts did not have detectable amounts of activity under the conditions tested, although the genes may not have been expressed in the hosts chosen.

The *G. stearothermophilus* DAT (untagged, which expressed better) was assayed as above and compared to the purified *B. sphaericus* DAT and AT-103 (BioCatalytics). The results are shown in Tables 42 and 43 below. The transamination activity of G. *stearothermophilus,* AT-103, and *B. sphaericus* D-aminotransferase was tested using 0.5 mg of D-aminotransferase per mL (Table 42).

**Table 42: Transamination Activity of G. stearothermophilus, AT-103, and B. sphaericus (Purified) D-Aminotransferases**

| D-Aminotransferase | Alanine (mM)-15 Minutes D-Tryptophan as Substrate | Alanine (mM)-15 Minutes R,R Monatin as Substrate | Alanine (mM)-2 Hours D-Tryptophan as Substrate | Alanine (mM)-2 Hours R,R Monatin as Substrate |
|---|---|---|---|---|
| AT-103 | 8.91 | 1.21 | 9.47 | 6.13 |
| *B. sphaericus* (tagged) | 8.91 | 1.65 | 9.53 | 7.17 |
| *G. stearothermophilus* (untagged) | 2.05 | 0.053 | 8.10 | 0.78 |

**Table 43: Comparison of G. stearothermophilus, AT-103, and B. sphaericus (purified) for Total Monatin Production**

| D-Aminotransferase | Total Monatin (ppm) 2 Hours | Total Monatin (ppm) Overnight | % R,R 2 Hours | % R,R Overnight |
|---|---|---|---|---|
| AT-103 | 450 | 645 | 65.5 | 60.6 |
| *B. sphaericus* (tagged) | 110 | 175 | 64 | 54 |
| *G. stearothermophilus* (untagged) | nd | 10 | n/a | 27 |

The native *G. stearothermophilus* enzyme is clearly less active for monatin transamination than the AT-103 and *B. sphaericus* enzymes.

### Example 19

### Creation of a Hybrid D-Aminotransferase

Several *Bacillus* D-amino acid aminotransferases were described in Examples 18 and 15. Although the G. *stearothermophilus* enzyme had low transamination activity on monatin, causing less total monatin to be produced from D-tryptophan, it still had structural elements of interest and it is a thermostable enzyme. Therefore, a hybrid protein was created between the higher activity enzyme (*B. sphaericus*) and the *Geobacillus* enzyme.

### Assembly of hybrid DAT coding sequence

The target protein sequence that was designed is SEQ ID NO:99. SEQ ID NO: 100, the coding sequence corresponding to SEQ ID NO:99, was designed based on *E.* co/*i* codon usage.

The hybrid DAT was constructed using assembly PCR techniques. The assembly process is as follows: 43 oligonucleotides (40 mers) were ordered from IDT based on the gene sequence above and its complementary DNA sequence, with 20 basepair overlaps between the sense and antisense strands. The primers were diluted to 250 µM in water and 5 µL of each primer was mixed together in a microfuge tube. PCR was carried out as follows: per 100 µL reaction, 1.5 µL of the primer pool, 4 µL dNTPs, 1X XL PCR buffer, 1 mM magnesium acetate, 2 µL r*Tth* polymerase (Roche, Indianapolis, IN), and 0.25 µL Pfu polymerase (Stratagene, La Jolla, CA) were added. A 3 minute hot start was done at 94°C, followed by 15 cycles of 94 °C for 30 seconds, 40 °C for 15 seconds, and 68 °C for 30 seconds. Ten more cycles were done with an increased annealing temperature of 44 °C and an increased annealing time of 30 seconds. Ten more cycles were performed at an annealing temperature of 48 °C and an extension time of 75 seconds. Lastly, a chain extension step was done for seven minutes at 68 °C. A secondary PCR was done using the following primers, designed for cloning with *Nde*I (N-term) and *Bam*HI (C-term):
N-term-5'-GGCCTTGGCATATGGGATACACTTTATGGAATGACCA -3' (SEQ ID NO:101) and C-term-5'-TTGGAACCGGATCCTTAGCTGTTAAGGCTCAGTGGAA -3' (SEQ ID NO:102)

The PCR contained per 100 µL, 2.5 µL of the primary reaction, 3 µL dNTPs, 1X XL PCR buffer, 1mM magnesium acetate, 2 µL r*Tth,* and 0.25 µL Pfu polymerase. A 3 minute hot start was done at 94 °C, followed by 10 cycles of 94 °C for 30 seconds, 42 °C for 30 seconds, and 68 °C for 75 seconds. Fifteen more cycles were done with an increased annealing temperature of 48 °C, and lastly a chain extension step was done for seven minutes at 68°C. A product of approximately 850 bp was visible on an agarose gel.

### Cloning

The PCR product was gel purified using the Qiagen QIAquick Gel Extraction Kit (Valencia, CA), and cloned using the Zero Blunt TOPO^{®} cloning kit as per manufacturers' protocols (Invitrogen). The plasmids were transformed into TOP 10 chemically competent cells for initial screening by PCR. The plasmid DNA was screened by restriction digest and the DNA sequence was verified.

The plasmid minipreps were digested with *Bam*HI and *Nde*I (New England Biolabs, Ipswich, MA). The digested vectors (pET28 and pET30) and insert were ligated using the Roche Rapid DNA Ligation Kit (Roche) and purified using the Roche High-Pure PCR Product Purification Kit. The ligations were transformed into *Escherichia coli* DH10B using a 0.2 cm cuvette and a Bio-Rad Gene Pulser II system as described in the Bio-Rad electroporation manual. The cells were allowed to recover in 900 µL SOC medium for 30 minutes at 37 °C at 225 rpm. The cells were plated on LB-agar plates containing kanamycin (25 µg/mL). The plasmid DNA was purified using the Qiagen spin miniprep kit and screened for the correct inserts by restriction digest with *Bam*HI and *Nde*I*.*

### Gene Expression and Assays

Plasmid DNA was transformed into *E. coli* expression host BL21(DE3) according to manufacturers' protocols (Novagen, Madison, WI). The cultures were grown and the plasmids were isolated using Qiagen miniprep kit and analyzed by PCR to confirm identity. The induction was performed in LB medium containing kanamycin (50 µg/mL). The cells were grown to an OD₆₀₀ of 0.5 at 37 °C, induced with 0.1 mM IPTG (isopropyl thiogalacatoside) and sampled at 3 hours post induction. The cell extracts were prepared according to the protocol accompanying the Novagen BugBuster™ reagent (with benzonase nuclease and Roche complete protease inhibitor cocktail added). High levels of total protein were obtained at the predicted molecular weight, as judged by SDS-PAGE, for both gene products. However, the soluble levels of protein were lower. The untagged version of the gene product expressed better and was assayed as a cellular extract. The soluble proteins in the cellular extracts were separated on a BioRad Laboratories Experion Automated Electrophoresis Station and analyzed for concentration and percent expression using the Experion Software version 1.1.98.0, to normalize the amount of D-aminotransferase used in comparative assays.

The cell extracts were analyzed for D-aminotransferase activity by following production of alanine from pyruvate and D-tryptophan (or R,R monatin) using the following protocol. Duplicate one mL reactions were carried out in 100 mM potassium phosphate buffer (pH 7.5), 50 µM pyridoxal phosphate, 25 mM sodium pyruvate, and 50 mM D-tryptophan or R,R monatin (unless otherwise noted). The reactions were initiated by the addition of cell free extracts or purified enzyme and were incubated 15 minutes-overnight at 30°C, with mild shaking. Approximately the same level of D-aminotransferase was added (0.5 mg) in each assay for comparative purposes (unless otherwise noted). AT-103 (BioCatalytics) or *B. sphaericus* D-aminotransferase (Example 18) was used as a benchmark enzyme. Formic acid was added to a final concentration of two percent to stop the reaction and the precipitated protein was removed by centrifugation. Control reactions without added protein were also performed. Zero time points were also used as negative controls. Alanine was detected using OPA post-column derivatization as described in Example 1. The results of the reactions using 0.5 mg D-aminotransferase per 1 mL reaction volume are shown in Table 44 below.

**Table 44: Transamination Activity of B. sphaericus (purified), G. stearothermophilus, and Hybrid D-Aminotransferases**

| D-Aminotransferase | Alanine (mM)-15 Minutes D-Tryptophan as Substrate | Alanine (mM)-15 Minutes R,R Monatin as Substrate | Alanine (mM)-2 Hours D-Tryptophan as Substrate | Alanine (mM)-2 Hours R,R Monatin as Substrate |
|---|---|---|---|---|
| Hybrid DAT (untagged) | 13.5 | 0.084 | 14.2 | 0.54 |
| *B. sphaericus* (tagged) | 13.6 | 4.60 | 13.9 | 10.6 |
| *G. stearothermophilus* (untagged) | 6.6 | 0.18 | 13.5 | 2.2 |

The aminotransferases were also tested for their ability to produce monatin from D-tryptophan (as in Example 3). The following were added per 1 mL of reaction mixture: approximately 50-100 µg purified *C. testosteroni* ProA aldolase, 4 mM MgCl₂, 50 mM D-tryptophan (supplied as solid), 0.5-2 mg D-aminotransferase, 200 mM sodium pyruvate, 100 mM potassium phosphate buffer pH 7.5, and 0.05 mM PLP. Experiments were run in duplicate, with negative controls in which no aminotransferase was added. The samples were incubated 1 hour, 2 hours, and overnight (17-20 hours) at 30 °C with gentle shaking. The only stereoisomers detected when producing monatin using these methods were R,R and S,R. The percent R,R is listed in Table 45 below, and was determined by reversed-phase LC peak area. At low monatin concentrations, the percent R,R is not as accurate as judged by RPLC peak area. Therefore, some of the samples were further analyzed by the FDAA derivatization method described in Example 1. The numbers from those results are shown in the table in parentheses.

**Table 45: Comparison of G. stearothermophilus, Hybrid DAT, and B. sphaericus (purified) for Total Monatin Production**

| D-Aminotransferase | Total Monatin (ppm) 2 Hours | Total Monatin (ppm) Overnight | % R,R 2 Hours | % R,R Overnight |
|---|---|---|---|---|
| Hybrid DAT (untagged) | 9.5 | 42.5 | 84.1 (79.8) | 81.1 (69.6) |
| *B. sphaericus* (tagged) | 68.5 | 182.5 | 62.7 (53.8) | 55.1 (53.5) |
| *G. stearothermophilus* (untagged) | 4.5 | 15.0 | 34.1 (20.7) | 32.1 (21.7) |

The Hybrid DAT makes more monatin than the *G. stearothermophilus* enzyme, although the monatin transamination rate of the Hybrid DAT is lower. It is possible that under the conditions for monatin production (where there are low MP concentrations), the Hybrid performs better possibly due to a lower Kₘ. Also, the Hybrid DAT makes a higher percentage of R,R than either of the parent enzymes. This enzyme appears to have a higher enantioselectivity for R-MP than the parent enzymes. The same assays were done (4 hour incubation time) using the *Sinorhizobium* aldolase described in Example 3 with the Hybrid DAT. The Hybrid DAT produced similar amounts of monatin as above, but using the alternative aldolase, produced 95% R,R (according to FDAA derivatization), as opposed to 80% with the C. *testosteroni* ProA aldolase.

The hybrid DAT was also tested for transamination activity of R-MP versus S-MP (produced as described in Example 1). Two hour and overnight assays were conducted at 30 °C using 10 mM R-MP or S-MP, 50 mM D-alanine, 100 mM potassium phosphate pH 7.5, 0.5 mg/mL D-aminotransferase, and 50 µM PLP. Experiments were run in duplicate and the background levels of monatin from the MP samples was subtracted. The ratios of monatin produced from each substrate are reported for both D-aminotransferases in Table 46 below. Similar trends were observed when pyruvate (produced) ratios were plotted. It is clear that the Hybrid DAT is more selective for R-MP than the AT-103 D-aminotransferase, which does not appear to be selective.

**Table 46: Comparison of Hybrid DAT and AT-103 for S-MP and R-MP Transamination**

| D-Aminotransferase | R-Activity/S-Activity 2 Hours | R-Activity/S-Activity Overnight |
|---|---|---|
| Hybrid DAT (untagged) | 8.6 | 2.2 |
| AT-103 | 0.68 | 1.68 |

In an effort to further improve the Hybrid DAT activity, site directed mutagenesis was done. Primers were designed as suggested in the QuikChange Multi Site-Directed Mutagenesis Kit (Stratagene). Two different mutants were created: Hybrid DAT 2 and Hybrid DAT 3. The Hybrid DAT 2 includes a mutation at amino acid position 153 from alanine to arginine and a deletion of serine 181. The alanine to arginine mutation was designed to help coordinate the second carboxyl group in the monatin precursor substrate, as has been shown to be present in the AspC L-aminotransferase. The serine deletion was an attempt to remove some steric hindrance such that the larger monatin precursor molecule can get to the active site more easily. The Hybrid DAT 3 contains a deletion of serines 180-182, replaced by one arginine. Two additional mutants were created, having only the 153 ala to arg mutation or the serine deletion, respectively. All three of the mutants that contained deletions did not make soluble protein, although they overexpressed at very high concentrations. Clearly it is important structurally not to remove amino acids in this region. The ala153arg mutant did not produce monatin under the conditions tested (as above). There is a fair amount of steric hindrance near the 153 position which would make it more difficult to fit the monatin precursor substrate in without the deletions in the 180-182 region. It is expected that mutating the serines to smaller amino acids, such as glycine or alanine, would improve activity toward monatin precursor, particularly when combined with the ala153arg mutation.

### EXAMPLE 20 (Canceled)

### Example 21

### Immobilization of B. sphaericus D-Alanine Aminotransferase

The *Bacillus sphaericus* D-alanine aminotransferase was purified as the HIS₆-tagged protein as described in Example 14.

The enzyme was immobilized onto Eupergit® C resin beads according to the procedure of Mateo, C, *et* a/., *Biotechnology Progress 18*:629-634, (2002). The purified enzyme (4 mL at 6.0 mg/mL) was dialyzed in 0.4 L of 0.5 M potassium phosphate, pH 7.8 using a Pierce Slide-A-Lyzer Dialysis Cassette (7K MWCO; catalog # 66370; Rockford, IL) for 1 hour at ambient temperature. The buffer was changed and the dialysis was continued for 1 hour. Pyridoxal phosphate ("PLP") was added to a final concentration of 0.05 mM and the resulting solution was mixed with 0.2 g of Eupergit^{®} C resin purchased from Sigma-Aldrich (Fluka catalog #46115; St. Louis, MO). The enzyme-resin suspension was incubated at ambient temperature with gentle mixing overnight. The resin beads were separated from the enzyme solution by centrifugation at 4000 x g for 5 minutes. The supernatant was removed and the resin was washed with 3 x 3 mL of 100 mM potassium phosphate, pH 7.8 containing 0.05 mM PLP. The mixture was centrifuged at 3000 x g for 5 minutes between washes. The amount of protein bound to the resin was determined by measuring the amount of protein in each supernatant and subtracting the sum from the original amount of protein to be immobilized. The protein concentrations were measured using a Pierce BCA™ Protein Assay Kit with bovine serum albumin as the standard (catalog #23225; Rockford, IL). The washed immobilized- enzyme beads were finally suspended in 4 mL of 100 mM potassium phosphate, pH 7.8 containing 0.05 mM PLP. The unreacted epoxy groups of the immobilized-enzyme beads were blocked by incubation with 1.9 M alanine at ambient temperature with gentle mixing. After 24 hours, the beads were washed, as described above, to remove the excess alanine and finally resuspended in 100 mM potassium phosphate, pH 7.8 containing 0.05 mM PLP. The final concentration of immobilized enzyme was 118 mg protein per g resin bead.

### Example 22

### Immobilization of S. meliloti ProA Aldolase

The *Sinorhizobium meliloti* HMG aldolase ("*proA*") was purified as the HIS₆-tagged protein using a procedure similar to the one described in Example 14 for the HIS₆-tagged *B. sphaericus* D-alanine aminotransferase.

Starting from a fresh culture plate (LB agar with 50 µg/mL kanamycin) of BL21(DE3)::*S*. *meliloti proA* pET30(Xa/LIC), cells were grown in 5 ml of Luria-Bertani broth ("LB") with 50 µg/ml kanamycin, at 37 °C and 225 rpm overnight. Subsequently, the culture was transferred at 0.5-0.6% (v/v) into flasks containing 800 mL of LB broth with 50 µg/ml kanamycin. The cells were grown at 37 °C and 225 rpm until the OD₆₀₀ reached 0.6-0.7. The gene expression was induced by the addition of 0.2 mM IPTG. The cultures were further incubated at 30 °C for 4 hours at 225 rpm and then harvested by centrifugation in a Beckman (Fullerton, CA) J25II centrifuge with a JS-16.25 rotor at 10,000 rpm for 10 minutes. The cell pellet was washed once with cold 50 mM EPPS buffer, pH 8.2, and the cells were centrifuged again. The washed cell pellet was harvested and used immediately. To prepare cell-free extract containing the *S. meliloti* HIS₆-proA aldolase (HIS₆-SmelproA) protein, the cells were suspended in 3-4 volumes of 50 mM EPPS, pH 8.2, containing 100 mM NaCl, and then disrupted using a Microfluidics (Newton, MA) homogenizer (3 passes at 20,000 psi), maintaining the temperature of the suspension below 15°C. All subsequent purification steps were carried out at 4°C. The cell extract was centrifuged for 15 minutes at 15,000 x g to remove the cell debris. Aliquots of the cell free extract, each containing between 15 and 20 mg of soluble protein, were applied to Novagen HIS-Bind columns (catalog # 70971-4) that had been previously equilibrated with the Novagen Bind buffer. The columns were washed with 2 x 10 mL of the Novagen Bind buffer and 1 x 10 mL of the Novagen Wash buffer diluted 1:1 with the Bind buffer. The HIS₆-SmelproA was eluted with 5 mL of the Novagen Elute buffer from each column. The elution fractions from each column were combined and concentrated 2X with Amicon (Billerica, MA) Ultra-15 centrifugal filter devices (MWCO 10 kDa). The buffer was exchanged by passage through disposable GE Healthcare PD10 desalting columns (catalog #17-0851-01) previously equilibrated with 50 mM EPPS, pH 8.2, containing 100 mM NaCl.

The protein concentration of the desalted solution was determined using the Pierce BCA™ Protein Assay Kit (catalog #23225; Rockford, IL). The purity of each fraction and the level of expression in the cell free extract fraction were determined by SDS-PAGE with a Bio-Rad Protean II minigel system (Hercules, CA) and 4-15% gradient gels. Typically, this procedure produced about 60-70 mg of enzyme from 3200 mL of LB culture with a purity of ∼90%. Aliquots (1-5 mL) of the purified enzyme were stored at -80 C until use.

The enzyme was immobilized onto Eupergit^{®} C resin beads according to the procedure of Mateo, C., et a/., (2002) Biotechnology Progress 18:629-634, (2002) and as described in Example 21 for the *B. sphaericus* D-alanine aminotransferase, except that 4 mM magnesium chloride was present in the buffer during immobilization instead of 0.05 mM PLP. After blocking with glycine, the washed immobilized enzyme was suspended in 100 mM potassium phosphate, pH 7.8 containing 4 mM magnesium chloride. The final concentration of *S*. *meliloti* proA aldolase was 52 mg protein per gram resin bead.

### Example 23

### Production of R,R-Monatin Using Immobilized Enzymes

The *B. sphaericus* HIS₆-tagged D-alanine aminotransferase and the *R. meliloti* HIS₆-tagged proA aldolase were purified and immobilized as described in Examples 21 and 22.

Solutions of 50 mM sodium pyruvate, 40 mM D-tryptophan, 4 mM MgCl₂, and 50 µM PLP in 100 mM potassium phosphate, pH 7.8 were prepared in 15-mL polypropylene tubes with screw caps. To each of these solutions was added both of the immobilized enzymes to a final volume of 4 mL. The resulting suspensions were incubated at room temperature with gentle mixing for up to 24 hours. The progress of each reactions was followed by HPLC and/or LC-MS analyses, measuring D-tryptophan, D-alanine, R,R-monatin, and pyruvic acid. The isomeric purity of the product monatin was determined using chiral LC/MS/MS. All analytical methods are described in Example 1. Typical results from experiments using immobilized enzymes are shown in Table 47 below. Analysis of the isomeric purity of the monatin formed during the reaction showed that the product of the enzymatic reactions was between 74 and 80% R,R.

**Table 47: Production of R,R-Monatin Using Immobilized Enzymes**

| proA Aldolase Concentration (µg/mL) | D-Alanine Aminotransferase Concentration (µg/mL) | Monatin Concentration (mM) (4 Hour Timepoint) | Tryptophan Concentration (mM) (4 Hour Timepoint) | Alanine Concentration (mM) (4 Hour Timepoint) |
|---|---|---|---|---|
| 50 | 500 | 0.06 | 17.75 | 20.51 |
| 50 | 1000 | 0.29 | 15.03 | 24.71 |
| 100 | 1000 | 0.33 | 15.17 | 24.73 |
| 100 | 2000 | 0.54 | 14.40 | 29.45 |

## Claims

1. A method for producing R,R monatin, or a salt thereof, comprising reacting monatin precursor and one or more D-aminotransferases chosen from a *Bacillus halodurans* D-aminotransferase comprising the amino acid sequence listed in accession number NP_243677, a hybrid D-aminotransferase comprising the amino acid sequence of SEQ ID NO: 99, a D-aminotransferase of ATCC 4978 comprising the amino acid sequence of SEQ ID NO: 86, a D-aminotransferase of ATCC 7063 comprising the amino acid sequence of SEQ ID NO: 87, and homologs thereof with at least 90% sequence homology thereto.

2. The method of claim 1, further comprising reacting D-tryptophan and said one or more D-aminotransferases.

3. The method of claim 2, wherein at least about 75% of monatin produced is R,R monatin.

4. The method of any one of claims 1 to 3, wherein the D-aminotransferase is a *Bacillus halodurans* D-aminotransferase comprising the amino acid sequence listed in accession number NP_243677 or an amino acid sequence with at least 90% sequence homology thereto.

5. The method of any one of claims 1 to 3, wherein the D-aminotransferase is a hybrid D-aminotransferase comprising the amino acid sequence of SEQ ID NO: 99 or an amino acid sequence with at least 90% sequence homology thereto.

6. The method of any one of claims 1 to 3, wherein the D-aminotransferase is a D-aminotransferase of ATCC 4978 comprising the amino acid sequence of SEQ ID NO.86 or an amino acid sequence with at least 90% sequence homology thereto.

7. The method of any one of claims 1 to 3, wherein the D-aminotransferase is a D-aminotransferase of ATCC 7063 comprising the amino acid sequence of SEQ ID NO.87 or an amino acid sequence with at least 90% sequence homology thereto.

8. A hybrid D-aminotransferase comprising SEQ ID NO:99, or a homolog thereof with at least 90% sequence homology thereto.

9. A D-aminotransferase of ATCC 4978 comprising SEQ ID NO.86, or a homolog thereof with at least 90% sequence homology thereto.

10. A D-aminotransferase of ATCC 7063 comprising SEQ ID NO:87, or a homolog thereof with at least 90% sequence homology thereto.

11. A use of one or more D-aminotransferases chosen from a *Bacillus halodurans* D-aminotransferase comprising the amino acid sequence listed in accession number NP_243677, a hybrid D-aminotransferase comprising the amino acid sequence of SEQ ID NO: 99, a D-aminotransferase of ATCC 4978 comprising the amino acid sequence of SEQ ID NO: 86, a D-aminotransferase of ATCC 7063 comprising the amino acid sequence of SEQ ID NO: 87, and homologs thereof with at least 90% sequence homology thereto, for producing R,R monatin, or a salt thereof.

12. The use of claim 11, wherein said one or more D-aminotransferases are reacted with monatin precursor and/or D-tryptophan.

13. The use of claim 11 or 12, wherein the D-aminotransferase is a hybrid D-aminotransferase comprising the amino acid sequence of SEQ ID NO: 99 or an amino acid sequence with at least 90% sequence homology thereto.

14. The use of claim 11 or 12, wherein the D-aminotransferase is a D-aminotransferase of ATCC 4978 comprising the amino acid sequence of SEQ ID NO.86 or an amino acid sequence with at least 90% sequence homology thereto.

15. The use of claim 11 or 12, wherein the D-aminotransferase is a D-aminotransferase of ATCC 7063 comprising the amino acid sequence of SEQ ID NO.87 or an amino acid sequence with at least 90% sequence homology thereto.

## Patentansprüche

1. Verfahren zur Herstellung von R,R-Monatin oder eines Salzes davon, umfassend eine reagierende Monatin-Vorstufe und eine oder mehrere D-Aminotransferasen, die aus einer *Bacillus Halodurans* D-Aminotransferase, die die in der Zugangsnummer NP_243677 aufgelistete Aminosäuresequenz umfasst, einer Hybrid-D-Aminotransferase, die die Aminosäuresequenz von SEQ ID NR.: 99 umfasst, einer D-Aminotransferase von ATCC 4978, die die Aminosäuresequenz von SEQ ID NR.: 86 umfasst, einer D-Aminotransferase von ATCC 7063, die die Aminosäuresequenz von SEQ ID NR.: 87 umfasst, und Homologen davon mit wenigstens 90 % Sequenzhomologie dazu ausgewählt sind.

2. Verfahren gemäß Anspruch 1, weiterhin umfassend das Reagieren von D-Tryptophan und die genannten eine oder mehrere D-Aminotransferasen.

3. Verfahren gemäß Anspruch 2, bei dem wenigstens rund 75 % des produzierten Monatins R,R-Monatin ist.

4. Verfahren gemäß einem der Ansprüche 1 bis 3, bei dem die D-Aminotransferase eine *Bacillus Halodurans* D-Aminotransferase ist, die die in der Zugangsnummer NP_243677 aufgelistete Aminosäuresequenz oder eine Aminosäuresequenz mit wenigstens 90 % Sequenzhomologie dazu umfasst.

5. Verfahren gemäß Anspruch 1 bis 3, bei dem die D-Aminotransferase eine D-Aminotransferase ist, umfassend die Aminosäuresequenz von SEQ ID NR.: 99 oder eine Aminosäuresequenz mit wenigstens 90 % Sequenzhomologie dazu.

6. Verfahren gemäß einem der Ansprüche 1 bis 3, bei dem die D-Aminotransferase eine D-Aminotransferase von ATCC 4978 ist, die die Aminosäuresequenz von SEQ ID NR. 86 oder eine Aminosäuresequenz mit wenigstens 90 % Sequenzhomologie dazu umfasst.

7. Verfahren gemäß einem der Ansprüche 1 bis 3, bei dem die D-Aminotransferase eine D-Aminotransferase von ATCC 7063 ist, die die Aminosäuresequenz von SEQ ID NR. 87 oder eine Aminosäuresequenz mit wenigstens 90 % Sequenzhomologie dazu umfasst.

8. Hybrid-D-Aminotransferase, die die SEQ ID NR.: 99 oder ein Homolog davon mit wenigstens 90 % Sequenzhomologie davon umfasst.

9. D-Aminotransferase von ATCC 4978, die die SEQ ID NR. 86 oder ein Homolog davon mit wenigstens 90 % Sequenzhomologie dazu umfasst.

10. D-Aminotransferase von ATCC 7053, die die SEQ ID NR.: 87 oder ein Homolog davon mit wenigstens 90 % Sequenzhomologie dazu umfasst.

11. Verwendung von einer oder mehrerer D-Aminotransferase, die aus *Bacillus Halodurans* D-Aminotransferase, die die in der Zugangsnummer NP_243677 aufgelistete Aminosäuresequenz umfasst, einer die Aminosequenz von SEQ ID NR.: 99 umfassenden Hybrid-D-Aminotransferase, einer die Aminosäuresequenz von SEQ ID NR.: 86 umfassenden D-Aminotransferase von ATCC 4978, einer die Aminosäuresequenz von SEQ ID NR.: 87 umfassenden Aminotransferase von ATCC 7063 und Homologen davon mit wenigstens 90 % Sequenzhomologie dazu ausgewählt sind, zur Produktion von R,R-Monatin oder einem Salz davon.

12. Verwendung gemäß Anspruch 11, bei der eine oder mehrere D-Aminotransferasen mit einem Monatin-Vorläufer und / oder D-Tryptophan umgesetzt werden.

13. Verwendung gemäß Anspruch 11 oder 12, bei der die D-Aminotransferase eine Hybrid-D-Aminotransferase ist, die die Aminosäuresequenz von SEQ ID NR.: 99 oder eine Aminosäuresequenz mit wenigstens 90 % Sequenzhomologie dazu umfasst.

14. Verwendung gemäß Anspruch 11 oder 12, bei der die D-Aminotransferase eine D-Aminotransferase von ATCC 4978 ist, die die Aminosäuresequenz von SEQ ID NR. 86 oder eine Aminosäuresequenz mit wenigstens 90 % Sequenzhomologie dazu umfasst.

15. Verwendung gemäß Anspruch 11 oder 12, bei der die D-Aminotransferase eine D-Aminotransferase von ATCC 7063 ist, die die Aminosäuresequenz von SEQ ID NR. 87 oder eine Aminosäuresequenz mit wenigstens 90 % Sequenzhomologie dazu umfasst.

## Revendications

1. Procédé pour produire de la monatine R,R, ou un sel de celle-ci, comprenant la réaction d'un précurseur de monatine et d'une ou plusieurs D-aminotransférases choisies à partir d'une *Bacillus halodurans* D-aminotransférase comprenant la séquence d'acides aminés listée dans le numéro matricule NP_243677, une D-aminotransférase hybride comprenant la séquence d'acides aminés de SEQ ID n° 99, une D-aminotransférase d'ATCC 4978 comprenant la séquence d'acides aminés de SEQ ID n° 86, une D-aminotransférase d'ATCC 7063 comprenant la séquence d'acides aminés de SEQ ID n° 87, et des homologues de celles-ci avec une homologie de séquence d'au moins 90 % à celles-ci.

2. Procédé selon la revendication 1, comprenant en outre la réaction de D-tryptophane et desdites une ou plusieurs D-aminotransférases.

3. Procédé selon la revendication 2, dans lequel au moins environ 75 % de monatine produite est de la monatine R,R.

4. Procédé selon n'importe laquelle des revendications 1 à 3, dans lequel la D-aminotransférase est une *Bacillus halodurans* D-aminotransférase comprenant la séquence d'acides aminés listée dans le numéro matricule NP_243677 ou une séquence d'acides aminés avec une homologie de séquence d'au moins 90 % à celle-ci.

5. Procédé selon n'importe laquelle des revendications 1 à 3, dans lequel la D-aminotransférase est une D-aminotransférase hybride comprenant la séquence d'acides aminés de SEQ ID n° 99 ou une séquence d'acides aminés avec une homologie de séquence d'au moins 90 % à celle-ci.

6. Procédé selon n'importe laquelle des revendications 1 à 3, dans lequel la D-aminotransférase est une D-aminotransférase d'ATCC 4978 comprenant la séquence d'acides aminés de SEQ ID n° 86 ou une séquence d'acides aminés avec une homologie de séquence d'au moins 90 % à celle-ci.

7. Procédé selon n'importe laquelle des revendications 1 à 3, dans lequel la D-aminotransférase est une D-aminotransférase d'ATCC 7063 comprenant la séquence d'acides aminés de SEQ ID n° 87 ou une séquence d'acides aminés avec une homologie de séquence d'au moins 90 % à celle-ci.

8. D-aminotransférase hybride comprenant SEQ ID n° 99, ou un homologue de celle-ci avec une homologie de séquece d'au moins 90 % à celle-ci.

9. D-aminotransférase d'ATCC 4978 comprenant SEQ ID n° 86, ou un homologue de celle-ci avec une homologie de séquence d'au moins 90 % à celle-ci.

10. D-aminotransférase d'ATCC 7063 comprenant SEQ ID n° 87, ou un homologue de celle-ci avec une homologie de séquence d'au moins 90 % à celle-ci.

11. Utilisation d'une ou plusieurs D-aminotransférases choisies à partir d'une *Bacillus halodurans* D-aminotransférase comprenant la séquence d'acides aminés listée dans le numéro matricule NP_243677, une D-aminotransférase hybride comprenant la séquence d'acides aminés de SEQ ID n° 99, une D-aminotransférase d'ATCC 4978 comprenant la séquence d'acides aminés de SEQ ID n° 86, une D-aminotransférase d'ATCC 7063 comprenant la séquence d'acides aminés de SEQ ID n° 87 et des homologues de celle-ci avec une homologie de séquence d'au moins 90 % à celle-ci, pour produire de la monatine R,R, ou un sel de celle-ci.

12. Utilisation selon la revendication 11, dans laquelle lesdites une ou plusieurs D-aminotransférases réagissent avec un précurseur de monatine et/ou D-tryptophane.

13. Utilisation selon la revendication 11 ou 12, dans laquelle la D-aminotransférase est une D-aminotransférase hybride comprenant la séquence d'acides aminés de SEQ ID n° 99 ou une séquence d'acides aminés avec une homologie de séquence d'au moins 90 % à celle-ci.

14. Utilisation selon la revendication 11 ou 12, dans laquelle la D-aminotransférase est une D-aminotransférase d'ATCC 4978 comprenant la séquence d'acides aminés de SEQ ID n° 86 ou une séquence d'acides aminés avec une homologie de séquences d'au moins 90 % à celle-ci.

15. Utilisation selon la revendication 11 ou 12, dans laquelle la D-aminotransférase est une D-aminotransférase d'ATCC 7063 comprenant la séquence d'acides aminés de SEQ ID n° 87 ou une séquence d'acides aminés avec une homologie de séquence d'au moins 90 % à celle-ci.


### SEQUENCE LISTING

<110> Cargill, Incorporated
   Brazeau, Brian J.
   Burke, Ellen
   de Souza, Mervyn
   Gort, Steven J.
   Hicks, Paula M.
   Kollmann, Sherry R.
   Luginbuhl, Peter
   McFarlan, Sara C.
   Richardson, Toby
   Sanchez-Riera, Fernando A.
   Solheid, Christopher
   Weiner, David
   Zhao, Lishan
<120> Polypeptides and Biosynthetic Pathways for the Production of Stereoisomers of Monatin and Their Precursors
<130> 2464.014PC01/MAC/M-R
<150> 60/674,932
   <151> 2005-04-26
<160> 102
<170> PatentIn version 3.3
<210> 1
   <211> 684
   <212> DNA
   <213> Comamonas testosteroni
<400> 1
   atgtacgaac tgggagttgt ctaccgcaat atccagcgcg ccgaccgcgc tgctgctgac      60
   ggcctggccg ccctgggctc cgccaccgtg cacgaggcca tgggccgcgt cggtctgctc     120
   aagccctata tgcgccccat ctatgccggc aagcaggtct cgggcaccgc cgtcacggtg     180
   ctgctgcagc ccggcgacaa ctggatgatg catgtggctg ccgagcagat tcagcccggc     240
   gacatcgtgg tcgcagccgt caccgcagag tgcaccgacg gctacttcgg cgatctgctg     300
   gccaccagct tccaggcgcg cggcgcacgt gcgctgatca tcgatgccgg cgtgcgcgac     360
   gtgaagacgc tgcaggagat ggactttccg gtctggagca aggccatctc ttccaagggc     420
   acgatcaagg ccaccctggg ctcggtcaac atccccatcg tctgcgccgg catgctggtc     480
   acgcccggtg acgtgatcgt ggccgacgac gacggcgtgg tctgcgtgcc cgccgcgcgt     540
   gccgtggaag tgctggccgc cgcccagaag cgtgaaagct tcgaaggcga aaagcgcgcc     600
   aagctggcct cgggcatcct cggcctggat atgtacaaga tgcgcgagcc cctggaaaag     660
   gccggcctga aatatattga ctaa                                            684
<210> 2
   <211> 227
   <212> PRT
   <213> Comamonas testosteroni
<400> 2
   Met Tyr Glu Leu Gly Val Val Tyr Arg Asn Ile Gln Arg Ala Asp Arg
   1               5                   10                  15
   Ala Ala Ala Asp Gly Leu Ala Ala Leu Gly Ser Ala Thr Val His Glu
               20                  25                  30
   Ala Met Gly Arg Val Gly Leu Leu Lys Pro Tyr Met Arg Pro Ile Tyr
           35                  40                  45
   Ala Gly Lys Gln Val Ser Gly Thr Ala Val Thr Val Leu Leu Gln Pro
       50                  55                  60
   Gly Asp Asn Trp Met Met His Val Ala Ala Glu Gln Ile Gln Pro Gly
   65                  70                  75                  80
   Asp Ile Val Val Ala Ala Val Thr Ala Glu Cys Thr Asp Gly Tyr Phe
                   85                  90                  95
   Gly Asp Leu Leu Ala Thr Ser Phe Gln Ala Arg Gly Ala Arg Ala Leu
               100                 105                 110
   Ile Ile Asp Ala Gly Val Arg Asp Val Lys Thr Leu Gln Glu Met Asp
           115                 120                 125
   Phe Pro Val Trp Ser Lys Ala Ile Ser Ser Lys Gly Thr Ile Lys Ala
       130                 135                 140
   Thr Leu Gly Ser Val Asn Ile Pro Ile Val Cys Ala Gly Met Leu Val
   145                 150                 155                 160
   Thr Pro Gly Asp Val Ile Val Ala Asp Asp Asp Gly Val Val Cys Val
                   165                 170                 175
   Pro Ala Ala Arg Ala Val Glu Val Leu Ala Ala Ala Gln Lys Arg Glu
               180                 185                 190
   Ser Phe Glu Gly Glu Lys Arg Ala Lys Leu Ala Ser Gly Ile Leu Gly
           195                 200                 205
   Leu Asp Met Tyr Lys Met Arg Glu Pro Leu Glu Lys Ala Gly Leu Lys
       210                 215                 220
   Tyr Ile Asp
   225
<210> 3
   <211> 63
   <212> DNA
   <213> Artificial sequence
<220>
   <223> pykA Knockout Primer 1
<400> 3
   atgtccagaa ggcttcgcag aacaaaaatc gttaccacgt taggtgtagg ctggagctgc     60
   ttc                                                                   63
<210> 4
   <211> 64
   <212> DNA
   <213> Artificial sequence
<220>
   <223> pykA Knockout Primer 2
<400> 4
   ctctaccgtt aaaatacgcg tggtattagt agaacccacg gtaccatatg aatatcctcc     60
   ttag                                                                  64
<210> 5
   <211> 64
   <212> DNA
   <213> Artificial sequence
<220>
   <223> pykF Knockout Primer 1
<400> 5
   aggacgtgaa cagatgcggt gttagtagtg ccgctcggta ccagcatatg aatatcctcc     60
   ttag                                                                  64
<210> 6
   <211> 63
   <212> DNA
   <213> Artificial sequence
<220>
   <223> pykF Knockout Primer 2
<400> 6
   atgaaaaaga ccaaaattgt ttgcaccatc ggaccgaaaa ccggtgtagg ctggagctgc     60
   ttc                                                                   63
<210> 7
   <211> 30
   <212> DNA
   <213> Artificial sequence
<220>
   <223> HEXaspC Primer 1
<400> 7
   gcggaacata tgtttgagaa cattaccgcc 30
<210> 8
   <211> 32
   <212> DNA
   <213> Artificial sequence
<220>
   <223> HEXaspC Primer 2
<400> 8
   ataaccggat ccttacagca ctgccacaat cg 32
<210> 9
   <211> 36
   <212> DNA
   <213> Artificial sequence
<220>
   <223> aspCW130F Backward Primer
<400> 9
   cgctcttatg gttcggtttg cttgggttgc tcaccc 36
<210> 10
   <211> 36
   <212> DNA
   <213> Artificial sequence
<220>
   <223> aspCW130F Forward Primer
<400> 10
   gggtgagcaa cccaagcttt ccgaaccata agagcg 36
<210> 11
   <211> 39
   <212> DNA
   <213> Artificial sequence
<220>
   <223> R122G-1 Primer
<400> 11
   caaaaaatac cagcgttaag ggagtgtggg tgagcaacc 39
<210> 12
   <211> 29
   <212> DNA
   <213> Artificial sequence
<220>
   <223> P9T_4 Primer
<400> 12
   cattaccgcc gctactgccg acccgattc 29
<210> 13
   <211> 39
   <212> DNA
   <213> Artificial sequence
<220>
   <223> I68V-1 Primer
<400> 13
   caccaaaaat tacctcggcg tagacggcat ccctgaatt 39
<210> 14
   <211> 35
   <212> DNA
   <213> Artificial sequence
<220>
   <223> T156A Primer
<400> 14
   tgatgcggaa aatcacgctc ttgacttcga tgcac 35
<210> 15
   <211> 35
   <212> DNA
   <213> Artificial sequence
<220>
   <223> L. brevis Glutamate Racemase Primer 1
<400> 15
   gcggcgccat ggaaaatgat ccgattggtc taatg 35
<210> 16
   <211> 33
   <212> DNA
   <213> Artificial sequence
<220>
   <223> L. brevis Glutamate Racemase Primer 2
<400> 16
   gcggcggtcg acgcaattac aattgtgttt gtc 33
<210> 17
   <211> 34
   <212> DNA
   <213> Artificial sequence
<220>
   <223> P. pentosaceus Glutamate Racemase Primer 1
<400> 17
   gcggcgccat ggatgtatgt ataattttat ttag 34
<210> 18
   <211> 36
   <212> DNA
   <213> Artificial sequence
<220>
   <223> P. pentosaceus Glutamate Racemase Primer 2
<400> 18
   gcggcggtcg acaaatttca ttattcattc taattt 36
<210> 19
   <211> 36
   <212> DNA
   <213> Artificial sequence
<220>
   <223> P. stutzeri 4 D-HPG AT Outer Primer 1
<400> 19
   ggccggcata tgtcgatcct taacgactac aaacgt 36
<210> 20
   <211> 36
   <212> DNA
   <213> Artificial sequence
<220>
   <223> P. stutzeri 4 D-HPG AT Outer Primer 2
<400> 20
   ggaaggctcg agtcatgatt ggtttccaga caaatt 36
<210> 21
   <211> 693
   <212> DNA
   <213> Aldolase (Bacillus subtilis)
<400> 21
   atgatttatc agccggggac aacaggcatc gtcgtgcagg atattgcacg cgctgatcaa     60
   gccattatcg atggcctagc agaatgtggt gtggcgacgg tgcatgaggc acaggggcgc    120
   aagggcctgt tggcggatta tatgacgccg atttactcgg gcgcgcgcat cgctggatct    180
   gcggtgacca ttctggcacc gccgtgtgac aattggatga tccatgtggc ggtagaacag    240
   ttgcaaaagg gcgatgtgtt gctgctgggc acgatcacac cgtccaatgc tggctatttc    300
   ggtgacttgc tggccacgtc agccatggcg cacggttgtc gcggattgat cattgatggc    360
   ggtgtgcgcg atgtgcaaga gctgacggat atgggctttc cggtttggtc caaggccgta    420
   catgcccaag gcacaatcaa agaaacgctg ggatcggtca acgtgccagt tgtctgcggc    480
   caagagttgg taaaccccgg tgatattgtg gtggccgacg atgacggggt gtgcgttgtg    540
   cgccgcgaag aagctgctga tgtgctggct aaggcgcggg cgcgcgagag caatgaagcg    600
   gccaagcgcg cgcgttttga ggccggtgag ctggggctgg atatctatga catgcgcgcg    660
   cggctggccg aaaaaggact gaaatacgtc tga                                 693
<210> 22
   <211> 230
   <212> PRT
   <213> Aldolase (Bacillus subtilis)
<400> 22
   Met Ile Tyr Gln Pro Gly Thr Thr Gly Ile Val Val Gln Asp Ile Ala
   1               5                   10                  15
   Arg Ala Asp Gln Ala Ile Ile Asp Gly Leu Ala Glu Cys Gly Val Ala
               20                  25                  30
   Thr Val His Glu Ala Gln Gly Arg Lys Gly Leu Leu Ala Asp Tyr Met
           35                  40                  45
   Thr Pro Ile Tyr Ser Gly Ala Arg Ile Ala Gly Ser Ala Val Thr Ile
       50                  55                  60
   Leu Ala Pro Pro Cys Asp Asn Trp Met Ile His Val Ala Val Glu Gln
   65                  70                  75                  80
   Leu Gln Lys Gly Asp Val Leu Leu Leu Gly Thr Ile Thr Pro Ser Asn
                   85                  90                  95
   Ala Gly Tyr Phe Gly Asp Leu Leu Ala Thr Ser Ala Met Ala His Gly
               100                 105                 110
   Cys Arg Gly Leu Ile Ile Asp Gly Gly Val Arg Asp Val Gln Glu Leu
           115                 120                 125
   Thr Asp Met Gly Phe Pro Val Trp Ser Lys Ala Val His Ala Gln Gly
       130                 135                 140
   Thr Ile Lys Glu Thr Leu Gly Ser Val Asn Val Pro Val Val Cys Gly
   145                 150                 155                 160
   Gln Glu Leu Val Asn Pro Gly Asp Ile Val Val Ala Asp Asp Asp Gly
                   165                 170                 175
   Val Cys Val Val Arg Arg Glu Glu Ala Ala Asp Val Leu Ala Lys Ala
               180                 185                 190
   Arg Ala Arg Glu Ser Asn Glu Ala Ala Lys Arg Ala Arg Phe Glu Ala
           195                 200                 205
   Gly Glu Leu Gly Leu Asp Ile Tyr Asp Met Arg Ala Arg Leu Ala Glu
       210                 215                 220
   Lys Gly Leu Lys Tyr Val
   225                 230
<210> 23
   <211> 36
   <212> DNA
   <213> Artificial sequence
<220>
   <223> Aldolase Primer
<400> 23
   gaggagctcg agtcagacgt atttcagtcc tttttc 36
<210> 24
   <211> 29
   <212> DNA
   <213> Artificial sequence
<220>
   <223> Aldolase Primer
<400> 24
   agaagacata tgatttatca gccggggac 29
<210> 25
   <211> 20
   <212> DNA
   <213> Artificial sequence
<220>
   <223> Alanine Racemase Primer
<400> 25
   atggacgagt ttcaccgcga 20
<210> 26
   <211> 20
   <212> DNA
   <213> Artificial sequence
<220>
   <223> Alanine Racemase Primer
<400> 26
   ttatgcatcg cttcatccgc 20
<210> 27
   <211> 31
   <212> DNA
   <213> Artificial sequence
<220>
   <223> Alanine Racemase Primer
<400> 27
   ataataggat cctcatccgc ggccaacggc g 31
<210> 28
   <211> 43
   <212> DNA
   <213> Artificial sequence
<220>
   <223> Alanine Racemase Primer
<400> 28
   gggaaaggta ccgaggaata ataaatggac gagtttcacc gcg 43
<210> 29
   <211> 43
   <212> DNA
   <213> Artificial sequence
<220>
   <223> Mutagenic Primer
<220>
   <221> misc_feature
   <222> (20)..(20)
   <223> n is a, c, g, or t
<220>
   <221> misc_feature
   <222> (21)..(21)
   <223> n is a, c, g, or t
<220>
   <221> misc_feature
   <222> (22)..(22)
   <223> k is g or t
<400> 29
   gccggacgac acgcacattn nkgcggtcgt gaaggcgaac gcc 43
<210> 30
   <211> 45
   <212> DNA
   <213> Artificial sequence
<220>
   <223> Mutagenic Primer
<220>
   <221> misc_feature
   <222> (22)..(23)
   <223> n is a, c, g, or t
<220>
   <221> misc_feature
   <222> (24)..(24)
   <223> k is g or t
<400> 30
   gtgaaggcga acgcctatgg annkggggat gtgcaggtgg caagg 45
<210> 31
   <211> 45
   <212> DNA
   <213> Artificial sequence
<220>
   <223> Mutagenic Primer
<220>
   <221> misc_feature
   <222> (21)..(22)
   <223> n is a, c, g, or t
<220>
   <221> misc_feature
   <222> (23)..(23)
   <223> k is g or t
<400> 31
   cctcccgcct ggcggttgcc nnkttggatg aggcgctcgc tttaa 45
<210> 32
   <211> 45
   <212> DNA
   <213> Artificial sequence
<220>
   <223> Mutagenic Primer
<220>
   <221> misc_feature
   <222> (22)..(23)
   <223> n is a, c, g, or t
<220>
   <221> misc_feature
   <222> (24)..(24)
   <223> k is g or t
<400> 32
   caaccaggcg aaaaggtgag cnnkggtgcg acgtacactg cgcag 45
<210> 33
   <211> 45
   <212> DNA
   <213> Artificial sequence
<220>
   <223> Mutagenic Primer
<220>
   <221> misc_feature
   <222> (23)..(24)
   <223> n is a, c, g, or t
<220>
   <221> misc_feature
   <222> (25)..(25)
   <223> k is g or t
<400> 33
   gatcgggacg attccgatcg gcnnkgcgga cggctggctc cgccg 45
<210> 34
   <211> 43
   <212> DNA
   <213> Artificial sequence
<220>
   <223> Mutagenic Primer
<220>
   <221> misc_feature
   <222> (21)..(22)
   <223> n is a, c, g, or t
<220>
   <221> misc_feature
   <222> (23)..(23)
   <223> k is g or t
<400> 34
   gccatttgga aacgatcaac nnkgaagtgc cttgcacgat cag 43
<210> 35
   <211> 43
   <212> DNA
   <213> Artificial sequence
<220>
   <223> Racemase Primer
<400> 35
   gggaaaggta ccgaggaata ataaatggac gagtttcacc gcg 43
<210> 36
   <211> 27
   <212> DNA
   <213> Artificial sequence
<220>
   <223> Racemase Primer
<400> 36
   gcggcgccat ggacgagttt caccgcg 27
<210> 37
   <211> 43
   <212> DNA
   <213> Artificial sequence
<220>
   <223> Alanine Racemase Primer
<400> 37
   gccatttgga aacgatcaac tatgaagtgc cttgcacgat cag 43
<210> 38
   <211> 45
   <212> DNA
   <213> Artificial sequence
<220>
   <223> Alanine Racemase Primer
<400> 38
   ctcccgcctg gcggttgcct tcttggatga ggcgctcgct ttaag 45
<210> 39
   <211> 43
   <212> DNA
   <213> Artificial sequence
<220>
   <223> Alanine Racemase Primer
<400> 39
   gccggacgac acgcacatta tggcggtcgt gaaggcgaac gcc 43
<210> 40
   <211> 1152
   <212> DNA
   <213> Alanine Racemase (Geobacillus stearothermophilus)
<400> 40
   atggacgagt ttcaccgcga tacgtgggcg gaagtggatt tggacgccat ttacgacaat 60
   gtggagaatt tgcgccgttt gctgccggac gacacgcaca ttatggcggt cgtgaaggcg 120
   aacgcctatg gacatgggga tgtgcaggtg gcaaggacag cgctcgaagc gggggcctcc 180
   cgcctggcgg ttgccttttt ggatgaggcg ctcgctttaa gggaaaaagg aatcgaagcg 240
   ccgattctag ttctcggggc ttcccgtcca gctgatgcgg cgctggccgc ccagcagcgc 300
   attgccctga ccgtgttccg ctccgactgg ttggaagaag cgtccgccct ttacagcggc 360
   ccttttccta ttcatttcca tttgaaaatg gacaccggca tgggacggct tggagtgaaa 420
   gacgaggaag agacgaaacg aatcgtagcg ctgattgagc gccatccgca ttttgtgctt 480
   gaaggggtgt acacgcattt tgcgactgcg gatgaggtga acaccgatta tttttcctat 540
   cagtataccc gttttttgca catgctcgaa tggctgccgt cgcgcccgcc gctcgtccat 600
   tgcgccaaca gcgcagcgtc gctccgtttc cctgaccgga cgttcaatat ggtccgcttc 660
   ggcattgcca tgtatgggct tgccccgtcg cccggcatca agccgctgct gccgtatcca 720
   ttaaaagaag cattttcgct ccatagccgc ctcgtacacg tcaaaaaact gcaaccaggc 780
   gaaaaggtga gctatggtgc gacgtacact gcgcagacgg aggagtggat cgggacgatt    840
   ccgatcggct atgcggacgg ctggctccgc cgcctgcagc actttcatgt ccttgttgac    900
   ggacaaaagg cgccgattgt cggccgcatt tgcatggacc agtgcatgat ccgcctgcct    960
   ggtccgctgc cggtcggcac gaaggtgaca ctgattggtc gccaagggga cgaggtaatt   1020
   tccattgatg atgtcgctcg ccatttggaa acgatcaact acgaagtgcc ttgcacgatc   1080
   agttatcgag tgccccgtat ttttttccgc cataagcgta taatggaagt gagaaacgcc   1140
   gttggccgcg ga                                                       1152
<210> 41
   <211> 384
   <212> PRT
   <213> Alanine Racemase (Geobacillus stearothermophilus)
<400> 41
   Met Asp Glu Phe His Arg Asp Thr Trp Ala Glu Val Asp Leu Asp Ala
   1               5                   10                  15
   Ile Tyr Asp Asn Val Glu Asn Leu Arg Arg Leu Leu Pro Asp Asp Thr
               20                  25                  30
   His Ile Met Ala Val Val Lys Ala Asn Ala Tyr Gly His Gly Asp Val
           35                  40                  45
   Gln Val Ala Arg Thr Ala Leu Glu Ala Gly Ala Ser Arg Leu Ala Val
       50                  55                  60
   Ala Phe Leu Asp Glu Ala Leu Ala Leu Arg Glu Lys Gly Ile Glu Ala
   65                  70                  75                  80
   Pro Ile Leu Val Leu Gly Ala Ser Arg Pro Ala Asp Ala Ala Leu Ala
                   85                  90                  95
   Ala Gln Gln Arg Ile Ala Leu Thr Val Phe Arg Ser Asp Trp Leu Glu
               100                 105                 110
   Glu Ala Ser Ala Leu Tyr Ser Gly Pro Phe Pro Ile His Phe His Leu
           115                 120                 125
   Lys Met Asp Thr Gly Met Gly Arg Leu Gly Val Lys Asp Glu Glu Glu
       130                 135                 140
   Thr Lys Arg Ile Val Ala Leu Ile Glu Arg His Pro His Phe Val Leu
   145                 150                 155                 160
   Glu Gly Val Tyr Thr His Phe Ala Thr Ala Asp Glu Val Asn Thr Asp
                   165                 170                 175
   Tyr Phe Ser Tyr Gln Tyr Thr Arg Phe Leu His Met Leu Glu Trp Leu
               180                 185                 190
   Pro Ser Arg Pro Pro Leu Val His Cys Ala Asn Ser Ala Ala Ser Leu
           195                 200                 205
   Arg Phe Pro Asp Arg Thr Phe Asn Met Val Arg Phe Gly Ile Ala Met
       210                 215                 220
   Tyr Gly Leu Ala Pro Ser Pro Gly Ile Lys Pro Leu Leu Pro Tyr Pro
   225                 230                 235                 240
   Leu Lys Glu Ala Phe Ser Leu His Ser Arg Leu Val His Val Lys Lys
                   245                 250                 255
   Leu Gln Pro Gly Glu Lys Val Ser Tyr Gly Ala Thr Tyr Thr Ala Gln
               260                 265                 270
   Thr Glu Glu Trp Ile Gly Thr Ile Pro Ile Gly Tyr Ala Asp Gly Trp
           275                 280                 285
   Leu Arg Arg Leu Gln His Phe His Val Leu Val Asp Gly Gln Lys Ala
       290                 295                 300
   Pro Ile Val Gly Arg Ile Cys Met Asp Gln Cys Met Ile Arg Leu Pro
   305                 310                 315                 320
   Gly Pro Leu Pro Val Gly Thr Lys Val Thr Leu Ile Gly Arg Gln Gly
                   325                 330                 335
   Asp Glu Val Ile Ser Ile Asp Asp Val Ala Arg His Leu Glu Thr Ile
               340                 345                 350
   Asn Tyr Glu Val Pro Cys Thr Ile Ser Tyr Arg Val Pro Arg Ile Phe
           355                 360                 365
   Phe Arg His Lys Arg Ile Met Glu Val Arg Asn Ala Val Gly Arg Gly
       370                 375                 380
<210> 42
   <211> 1152
   <212> DNA
   <213> Artificial sequence
<220>
   <223> Mutagenized Ala Racemase
<400> 42
   atggacgagt ttcaccgcga tacgtgggcg gaagtggatt tggacgccat ttacgacaat      60
   gtggagaatt tgcgccgttt gctgccggac gacacgcaca tttgtgcggt cgtgaaggcg     120
   aacgcctatg gacatgggga tgtgcaggtg gcaaggacag cgctcgaagc gggggcctcc     180
   cgcctggcgg ttgccgagtt ggatgaggcg ctcgctttaa gggaaaaagg aatcgaagcg     240
   ccgattctag ttctcggggc ttcccgtcca gctgatgcgg cgctggccgc ccagcagcgc     300
   attgccctga ccgtgttccg ctccgactgg ttggaagaag cgtccgccct ttacagcggc     360
   ccttttccta ttcatttcca tttgaaaatg gacaccggca tgggacggct tggagtgaaa     420
   gacgaggaag agacgaaacg aatcgtagcg ctgattgagc gccatccgca ttttgtgctt     480
   gaaggggtgt acacgcattt tgcgactgcg gatgaggtga acaccgatta tttttcctat     540
   cagtataccc gttttttgca catgctcgaa tggctgccgt cgcgcccgct gctcgtccat      600
   tgcgccaaca gcgcagcgtc gctccgtttc cctgaccgga cgttcaatat ggtccgcttc     660
   ggcattgcca tgtatgggct tgccccgtcg cccggcatca agccgctgct gccgtatcca     720
   ttaaaagaag cattttcgct ccatagccgc ctcgtacacg tcaaaaaact gcaaccaggc     780
   gaaaaggtga gctatggtgc gacgtacact gcgcagacgg aggagtggat cgggacgatt     840
   ccgatcggct atgcggacgg ctggctccgc cgcctgcagc actttcatgt ccttgttgac     900
   ggacaaaagg cgccgattgt cggccgcatt tgcatggacc agtgcatgat ccgcctgcct     960
   ggtccgctgc cggtcggcac gaaggtgaca ctgattggtc gccaagggga cgaggtaatt    1020
   tccattgatg atgtcgctcg ccatttggaa acgatcaacg cggaagtgcc ttgcacgatc    1080
   agttatcgag tgccccgtat ttttttccgc cataagcgta taatggaagt gagaaacgcc    1140
   gttggccgcg ga                                                        1152
<210> 43
   <211> 384
   <212> PRT
   <213> Artificial sequence
<220>
   <223> Mutagenized Ala Racemase
<400> 43
   Met Asp Glu Phe His Arg Asp Thr Trp Ala Glu Val Asp Leu Asp Ala
   1               5                   10                  15
   Ile Tyr Asp Asn Val Glu Asn Leu Arg Arg Leu Leu Pro Asp Asp Thr
               20                  25                  30
   His Ile Cys Ala Val Val Lys Ala Asn Ala Tyr Gly His Gly Asp Val
           35                  40                  45
   Gln Val Ala Arg Thr Ala Leu Glu Ala Gly Ala Ser Arg Leu Ala Val
       50                  55                  60
   Ala Glu Leu Asp Glu Ala Leu Ala Leu Arg Glu Lys Gly Ile Glu Ala
   65                  70                  75                  80
   Pro Ile Leu Val Leu Gly Ala Ser Arg Pro Ala Asp Ala Ala Leu Ala
                   85                  90                  95
   Ala Gln Gln Arg Ile Ala Leu Thr Val Phe Arg Ser Asp Trp Leu Glu
               100                 105                 110
   Glu Ala Ser Ala Leu Tyr Ser Gly Pro Phe Pro Ile His Phe His Leu
           115                 120                 125
   Lys Met Asp Thr Gly Met Gly Arg Leu Gly Val Lys Asp Glu Glu Glu
       130                 135                 140
   Thr Lys Arg Ile Val Ala Leu Ile Glu Arg His Pro His Phe Val Leu
   145                 150                 155                 160
   Glu Gly Val Tyr Thr His Phe Ala Thr Ala Asp Glu Val Asn Thr Asp
                   165                 170                 175
   Tyr Phe Ser Tyr Gln Tyr Thr Arg Phe Leu His Met Leu Glu Trp Leu
               180                 185                 190
   Pro Ser Arg Pro Leu Leu Val His Cys Ala Asn Ser Ala Ala Ser Leu
           195                 200                 205
   Arg Phe Pro Asp Arg Thr Phe Asn Met Val Arg Phe Gly Ile Ala Met
       210                 215                 220
   Tyr Gly Leu Ala Pro Ser Pro Gly Ile Lys Pro Leu Leu Pro Tyr Pro
   225                 230                 235                 240
   Leu Lys Glu Ala Phe Ser Leu His Ser Arg Leu Val His Val Lys Lys
                   245                 250                 255
   Leu Gln Pro Gly Glu Lys Val Ser Tyr Gly Ala Thr Tyr Thr Ala Gln
               260                 265                 270
   Thr Glu Glu Trp Ile Gly Thr Ile Pro Ile Gly Tyr Ala Asp Gly Trp
           275                 280                 285
   Leu Arg Arg Leu Gln His Phe His Val Leu Val Asp Gly Gln Lys Ala
       290                 295                 300
   Pro Ile Val Gly Arg Ile Cys Met Asp Gln Cys Met Ile Arg Leu Pro
   305                 310                 315                 320
   Gly Pro Leu Pro Val Gly Thr Lys Val Thr Leu Ile Gly Arg Gln Gly
                   325                 330                 335
   Asp Glu Val Ile Ser Ile Asp Asp Val Ala Arg His Leu Glu Thr Ile
               340                 345                 350
   Asn Ala Glu Val Pro Cys Thr Ile Ser Tyr Arg Val Pro Arg Ile Phe
           355                 360                 365
   Phe Arg His Lys Arg Ile Met Glu Val Arg Asn Ala Val Gly Arg Gly
       370                 375                 380
<210> 44
   <211> 30
   <212> DNA
   <213> Artificial sequence
<220>
   <223> B. licheniformis BCAT Primer
<400> 44
   ggttaaggcc atgggggacc agaaagacca 30
<210> 45
   <211> 32
   <212> DNA
   <213> Artificial sequence
<220>
   <223> B. licheniformis BCAT Primer
<400> 45
   ggccttccgt cgactcagct gacacttaag ct 32
<210> 46
   <211> 29
   <212> DNA
   <213> Artificial sequence
<220>
   <223> Mutagenic Primer
<400> 46
   atcacggatt tttattcggg gacggcgtg 29
<210> 47
   <211> 29
   <212> DNA
   <213> Artificial sequence
<220>
   <223> Mutagenic Primer
<400> 47
   atcacggatt tttagacggg gacggcgtg 29
<210> 48
   <211> 26
   <212> DNA
   <213> Artificial sequence
<220>
   <223> Mutagenic Primer
<400> 48
   ggacggcgtg tatgaaggga tcaggg 26
<210> 49
   <211> 32
   <212> DNA
   <213> Artificial sequence
<220>
   <223> Mutagenic Primer
<400> 49
   tgtttgaagg gatcaaggta tacgacggca ac 32
<210> 50
   <211> 34
   <212> DNA
   <213> Artificial sequence
<220>
   <223> Mutagenic Primer
<400> 50
   gacggcgtgt atgaagggat caaggtatac gacg 34
<210> 51
   <211> 29
   <212> DNA
   <213> Artificial sequence
<220>
   <223> Mutagenic Primer
<400> 51
   gctgaaagac gctttcatcc gcttggtcg 29
<210> 52
   <211> 28
   <212> DNA
   <213> Artificial sequence
<220>
   <223> Mutagenic Primer
<400> 52
   gctgaaagac gctcacatcc gcttggtc 28
<210> 53
   <211> 38
   <212> DNA
   <213> Artificial sequence
<220>
   <223> Mutagenic Primer
<400> 53
   ctgaaagacg cttacatcta cttggtcgtt tcaagagg 38
<210> 54
   <211> 40
   <212> DNA
   <213> Artificial sequence
<220>
   <223> Mutagenic Primer
<400> 54
   ggctgaaaga cgctttcatc tacttggtcg tttcaagagg 40
<210> 55
   <211> 39
   <212> DNA
   <213> Artificial sequence
<220>
   <223> Mutagenic Primer
<400> 55
   gctgaaagac gctcacatct acttggtcgt ttcaagagg 39
<210> 56
   <211> 27
   <212> DNA
   <213> Artificial sequence
<220>
   <223> Mutagenic Primer
<400> 56
   gcaggtgacc gcggactcga tccaaac 27
<210> 57
   <211> 27
   <212> DNA
   <213> Artificial sequence
<220>
   <223> Mutagenic Primer
<400> 57
   gcaggtgacc tcggacacga tccaaac 27
<210> 58
   <211> 32
   <212> DNA
   <213> Artificial sequence
<220>
   <223> Mutagenic Primer
<400> 58
   gcaggtgacc gcggacacga tccaaacaat tg 32
<210> 59
   <211> 40
   <212> DNA
   <213> Artificial sequence
<220>
   <223> Mutagenic Primer
<400> 59
   gtcatcataa ttgtcgaacc atacgcaata ttcccgaaac 40
<210> 60
   <211> 40
   <212> DNA
   <213> Artificial sequence
<220>
   <223> Mutagenic Primer
<400> 60
   gtcatcataa ttgtcgaacc aaaggcaata ttcccgaaac 40
<210> 61
   <211> 40
   <212> DNA
   <213> Artificial sequence
<220>
   <223> Mutagenic Primer
<400> 61
   gtcatcataa ttgtcgaacc attggcagaa ttcccgaaac 40
<210> 62
   <211> 45
   <212> DNA
   <213> Artificial sequence
<220>
   <223> Mutagenic Primer
<400> 62
   cgagtgtcat cataattgtc gaaccatacg cagaattccc gaaac 45
<210> 63
   <211> 46
   <212> DNA
   <213> Artificial sequence
<220>
   <223> Mutagenic Primer
<400> 63
   ccgagtgtca tcataattgt cgaaccaaag gcagaattcc cgaaac 46
<210> 64
   <211> 42
   <212> DNA
   <213> Artificial sequence
<220>
   <223> Mutagenic Primer
<400> 64
   aatcgctgaa cttgttaaac aatattcttg tccggatcga gg 42
<210> 65
   <211> 21
   <212> DNA
   <213> Artificial sequence
<220>
   <223> Oligo Primer
<400> 65
   gaagaccgtg gttatcaatt t 21
<210> 66
   <211> 21
   <212> DNA
   <213> Artificial sequence
<220>
   <223> Oligo Primer
<400> 66
   gatggtattt acgaagtaat c 21
<210> 67
   <211> 22
   <212> DNA
   <213> Artificial sequence
<220>
   <223> Oligo Primer
<400> 67
   agatttaata tcacaacgta ac 22
<210> 68
   <211> 22
   <212> DNA
   <213> Artificial sequence
<220>
   <223> Oligo Primer
<400> 68
   gccaagtaaa atttaagatt ta 22
<210> 69
   <211> 21
   <212> DNA
   <213> Artificial sequence
<220>
   <223> Oligo Primer
<400> 69
   atttgctggg tgcgtataaa g 21
<210> 70
   <211> 30
   <212> DNA
   <213> Artificial sequence
<220>
   <223> 4978 DAT GSP1 Upstrm Primer
<400> 70
   gacatgctcc tccgctgtaa ataattcacc 30
<210> 71
   <211> 30
   <212> DNA
   <213> Artificial sequence
<220>
   <223> 4978 DAT GSP1 Dwnstr Primer
<400> 71
   ccctggtgat gaagtgaagc cagtattaac 30
<210> 72
   <211> 27
   <212> DNA
   <213> Artificial sequence
<220>
   <223> 4978 DAT GSP2 Upstrm Primer
<400> 72
   atcgccaaat tgataaccac ggtcttc. 27
<210> 73
   <211> 30
   <212> DNA
   <213> Artificial sequence
<220>
   <223> 4978 DAT GSP2 Dwnstr Primer
<400> 73
   acgtcccgta gcaaactttg aaaaaggtgt 30
<210> 74
   <211> 30
   <212> DNA
   <213> Artificial sequence
<220>
   <223> 7063 DAT GSP1 Upstrm Primer
<400> 74
   tgcatagaat cggtcgatat gttcagtagc 30
<210> 75
   <211> 30
   <212> DNA
   <213> Artificial sequence
<220>
   <223> 7063 DAT GSP1 Dwnstr Primer
<400> 75
   gcggagaaac gattacagaa ggttcttcaa 30
<210> 76
   <211> 27
   <212> DNA
   <213> Artificial sequence
<220>
   <223> 7063 DAT GSP2 Upstrm Primer
<400> 76
   gtcaccaaat tgataaccac ggtcttc 27
<210> 77
   <211> 27
   <212> DNA
   <213> Artificial sequence
<220>
   <223> 7063 DAT GSP2 Dwnstr Primer
<400> 77
   ggtgtacttt atacgcaccc agcaaat 27
<210> 78
   <211> 22
   <212> DNA
   <213> Artificial sequence
<220>
   <223> Adaptor Oligo Primer 1
<400> 78
   gtaatacgac tcactatagg gc 22
<210> 79
   <211> 19
   <212> DNA
   <213> Artificial sequence
<220>
   <223> Adaptor Oligo Primer 2
<400> 79
   actatagggc acgcgtggt 19
<210> 80
   <211> 36
   <212> DNA
   <213> Artificial sequence
<220>
   <223> ATCC4978DAATNde1F Oligo Primer
<400> 80
   ggccttggca tatgagttat agcttatgga atgacc 36
<210> 81
   <211> 36
   <212> DNA
   <213> Artificial sequence
<220>
   <223> ATCC4978DAATBamH1R Oligo Primer
<400> 81
   ggccttaagg atccttatgc gcgaatacct tttggg 36
<210> 82
   <211> 34
   <212> DNA
   <213> Artificial sequence
<220>
   <223> ATCC7063DAATNdelF Oligo Primer
<400> 82
   ggccttggca tatgagctac actttatgga atga 34
<210> 83
   <211> 32
   <212> DNA
   <213> Artificial sequence
<220>
   <223> ATCC7063DAATBamH1R2a Oligo Primer
<400> 83
   ggccaaggat ccgctaccca ctaatcatta ga 32
<210> 84
   <211> 852
   <212> DNA
   <213> Bacillus rotans
<400> 84
   atgagttata gcttatggaa tgaccaaatt gtgaatgatg aagaagtagt agttgataag 60
   gaggaccgtg gctatcaatt tggcgatggt gtttatgaag ttgtaaaagt atataacggt 120
   gaattattta cagcggagga gcatgtcgat cgtttttacg cgagtgctga aaaaattcgc 180
   gttacgatcc cttatacaaa agacaaattg catcaattat tgcatcagtt agttgaaatg 240
   aataaagttc aaacaggaca tatttatttc caaattacgc gtggtgcagg ccctcgtaat 300
   catattttcc ctggtgatga agtgaagcca gtattaacag gtaataccaa ggaaaatcca 360
   cgtcccgtag caaactttga aaaaggtgtg aaagcaacat ttgtagaaga cattcgttgg 420
   ttacgctgtg acattaaatc attaaattta cttggtgcgg tacttgctaa acaagaagca 480
   catgaaaaag gatgctatga agcggtttta catcgtgatg aaatcgtaac agaaggctct 540
   tcttcaaata tttatggaat taaagatggc gtattataca cacatccagc gaataacttc 600
   atcttaaatg gtattacacg tcaagtaatc attaaatgtg ctgctgaaat tggcttacca 660
   gtgaaggaag aagcaatgac aaaaactcag cttcttgcaa tggatgaagt gattgtttca 720
   tcaacgactt cagaagtaac gccaattatc gacatagatg gaacagtaat tggtgcgggt 780
   aaaccgggtg actggacacg taaattacaa gcacaatttg atacgaaaat cccaaaaggt 840
   attcgcgcat aa 852
<210> 85
   <211> 870
   <212> DNA
   <213> Bacillus serositidis
<400> 85
   atgagctaca ctttatggaa tgacaaaatt gtggatgata accaagtatt cattaataaa 60
   gaagacagag gctatcaatt tggtgacggc gtttatgaag ttataaaagt atatgatggg 120
   gaaatgttta ctgctactga acatatcgac cgattctatg caagtgcaga gaagattaaa 180
   ttgactgtac cctatacaaa gcataaactg catcaattac tgcatgaatt agtggaagca 240
   aacgagctga aaactggaaa cttatacttt cagataacac gcggtgcgtc tccgcgtaat 300
   catctatttc caggagacga tgtacttccg gtattaacgg gaaatgtaaa agaagcacca 360
   cgctccatcg aaaatgcgca aaaaggtgta aaagccacat ttgcagaaga tattcgctgg 420
   ctgcgctgcg atattaaatc gctaaatctt ttaggggctg ttttagcgaa acaagaggca 480
   catgaaaagg gctgctacga agcaatttta catcgcggag aaacgattac agaaggttct 540
   tcaacaaacg ttttcggtat aaaaaatggt gtactctata cacatccagc tgataatttc 600
   atcttaagcg gcattacacg cggtgttgtt ttggcatgtg cgaacgaaat tggacttcct 660
   gtaaaacaag aagcatttac aaaagacaaa gcactgcaaa tggacgaaat gttcgtatcc 720
   tctactactt ctgaaataac accagttatc gatcttgatg gagtggccat taatggtgga 780
   gaaattggcg aatggacgcg caagctgcaa aagcaatttg caacgaagct tccgggaagc 840
   ccagcatata atttaacaga atataaatag 870
<210> 86
   <211> 283
   <212> PRT
   <213> Bacillus rotans
<400> 86
   Met Ser Tyr Ser Leu Trp Asn Asp Gln Ile Val Asn Asp Glu Glu Val
   1               5                   10                  15
   Val Val Asp Lys Glu Asp Arg Gly Tyr Gln Phe Gly Asp Gly Val Tyr
               20                  25                  30
   Glu Val Val Lys Val Tyr Asn Gly Glu Leu Phe Thr Ala Glu Glu His
           35                  40                  45
   Val Asp Arg Phe Tyr Ala Ser Ala Glu Lys Ile Arg Val Thr Ile Pro
       50                  55                  60
   Tyr Thr Lys Asp Lys Leu His Gln Leu Leu His Gln Leu Val Glu Met
   65                  70                  75                  80
   Asn Lys Val Gln Thr Gly His Ile Tyr Phe Gln Ile Thr Arg Gly Ala
                   85                  90                  95
   Gly Pro Arg Asn His Ile Phe Pro Gly Asp Glu Val Lys Pro Val Leu
               100                 105                 110
   Thr Gly Asn Thr Lys Glu Asn Pro Arg Pro Val Ala Asn Phe Glu Lys
           115                 120                 125
   Gly Val Lys Ala Thr Phe Val Glu Asp Ile Arg Trp Leu Arg Cys Asp
       130                 135                 140
   Ile Lys Ser Leu Asn Leu Leu Gly Ala Val Leu Ala Lys Gln Glu Ala
   145                 150                 155                 160
   His Glu Lys Gly Cys Tyr Glu Ala Val Leu His Arg Asp Glu Ile Val
                   165                 170                 175
   Thr Glu Gly Ser Ser Ser Asn Ile Tyr Gly Ile Lys Asp Gly Val Leu
               180                 185                 190
   Tyr Thr His Pro Ala Asn Asn Phe Ile Leu Asn Gly Ile Thr Arg Gln
   195 200 205
   Val Ile Ile Lys Cys Ala Ala Glu Ile Gly Leu Pro Val Lys Glu Glu
   210 215 220
   Ala Met Thr Lys Thr Gln Leu Leu Ala Met Asp Glu Val Ile Val Ser
   225 230 235 240
   Ser Thr Thr Ser Glu Val Thr Pro Ile Ile Asp Ile Asp Gly Thr Val
   245 250 255
   Ile Gly Ala Gly Lys Pro Gly Asp Trp Thr Arg Lys Leu Gln Ala Gln
   260 265 270
   Phe Asp Thr Lys Ile Pro Lys Gly Ile Arg Ala
   275 280
<210> 87
   <211> 289
   <212> PRT
   <213> Bacillus serositidis
<400> 87
   Met Ser Tyr Thr Leu Trp Asn Asp Lys Ile Val Asp Asp Asn Gln Val
   1 5 10 15
   Phe Ile Asn Lys Glu Asp Arg Gly Tyr Gln Phe Gly Asp Gly Val Tyr
   20 25 30
   Glu Val Ile Lys Val Tyr Asp Gly Glu Met Phe Thr Ala Thr Glu His
   35 40 45
   Ile Asp Arg Phe Tyr Ala Ser Ala Glu Lys Ile Lys Leu Thr Val Pro
   50 55 60
   Tyr Thr Lys His Lys Leu His Gln Leu Leu His Glu Leu Val Glu Ala
   65 70 75 80
   Asn Glu Leu Lys Thr Gly Asn Leu Tyr Phe Gln Ile Thr Arg Gly Ala
   85 90 95
   Ser Pro Arg Asn His Leu Phe Pro Gly Asp Asp Val Leu Pro Val Leu
   100 105 110
   Thr Gly Asn Val Lys Glu Ala Pro Arg Ser Ile Glu Asn Ala Gln Lys
   115 120 125
   Gly Val Lys Ala Thr Phe Ala Glu Asp Ile Arg Trp Leu Arg Cys Asp
       130                 135                 140
   Ile Lys Ser Leu Asn Leu Leu Gly Ala Val Leu Ala Lys Gln Glu Ala
   145                 150                 155                 160
   His Glu Lys Gly Cys Tyr Glu Ala Ile Leu His Arg Gly Glu Thr Ile
                   165                 170                 175
   Thr Glu Gly Ser Ser Thr Asn Val Phe Gly Ile Lys Asn Gly Val Leu
               180                 185                 190
   Tyr Thr His Pro Ala Asp Asn Phe Ile Leu Ser Gly Ile Thr Arg Gly
           195                 200                 205
   Val Val Leu Ala Cys Ala Asn Glu Ile Gly Leu Pro Val Lys Gln Glu
       210                 215                 220
   Ala Phe Thr Lys Asp Lys Ala Leu Gln Met Asp Glu Met Phe Val Ser
   225                 230                 235                 240
   Ser Thr Thr Ser Glu Ile Thr Pro Val Ile Asp Leu Asp Gly Val Ala
                   245                 250                 255
   Ile Asn Gly Gly Glu Ile Gly Glu Trp Thr Arg Lys Leu Gln Lys Gln
               260                 265                 270
   Phe Ala Thr Lys Leu Pro Gly Ser Pro Ala Tyr Asn Leu Thr Glu Tyr
           275                 280                 285
   Lys
<210> 88
   <211> 29
   <212> DNA
   <213> Artificial sequence
<220>
   <223> B. sphaericus DAT Primer 1
<400> 88
   gatataccat ggatactcat tatggaatg 29
<210> 89
   <211> 32
   <212> DNA
   <213> Artificial sequence
<220>
   <223> B. sphaericus DAT Primer 2
<400> 89
   gttatcggat ccttaggcat taattgaaat tg 32
<210> 90
   <211> 32
   <212> DNA
   <213> Artificial sequence
<220>
   <223> B. licheniformis DAT Primer 1
<400> 90
   ggccggttca tatgaaagtt ctttttaacg gc 32
<210> 91
   <211> 32
   <212> DNA
   <213> Artificial sequence
<220>
   <223> B. licheniformis DAT Primer 2
<400> 91
   ccttccggat ccttaaaccg ttttggctgt ct 32
<210> 92
   <211> 30
   <212> DNA
   <213> Artificial sequence
<220>
   <223> B. halodurans Primer 1
<400> 92
   gatatacata tggattattg cctttaccaa 30
<210> 93
   <211> 33
   <212> DNA
   <213> Artificial sequence
<220>
   <223> B. halodurans Primer 2
<400> 93
   gaatccggat cctcactgct tcatcgctgt ttg 33
<210> 94
   <211> 33
   <212> DNA
   <213> Artificial sequence
<220>
   <223> B. cereus Primer 1
<400> 94
   taagaggaat aacatatggc atacgaaaga ttt 33
<210> 95
   <211> 32
   <212> DNA
   <213> Artificial sequence
<220>
   <223> B. cereus Primer 2
<400> 95
   gaattcggat ccttaagaag atgacatatt gg 32
<210> 96
   <211> 33
   <212> DNA
   <213> Artificial sequence
<220>
   <223> B. cereus Primer 3
<400> 96
   taagaggaat aacatatggg atcgaaattg gca 33
<210> 97
   <211> 36
   <212> DNA
   <213> Artificial sequence
<220>
   <223> NdeI Primer
<400> 97
   ggccttggca tatgggatac actttatgga atgacc 36
<210> 98
   <211> 35
   <212> DNA
   <213> Artificial sequence
<220>
   <223> BamHI Primer
<400> 98
   ttggaaccgg atccttatat atgaagcggt tttgg 35
<210> 99
   <211> 283
   <212> PRT
   <213> Artificial sequence
<220>
   <223> Hybrid DAT
<400> 99
   Met Gly Tyr Thr Leu Trp Asn Asp Gln Ile Val Glu Asp Gly Ser Val
   1               5                   10                  15
   Ser Ile Ser Pro Glu Asp Arg Gly Tyr Gln Phe Gly Asp Gly Val Tyr
               20                  25                  30
   Glu Val Val Lys Val Tyr Asn Gly Asn Met Phe Thr Val Asn Glu His
           35                  40                  45
   Ile Asp Arg Leu Tyr Ala Ser Ala Glu Lys Ile Arg Ile Val Ile Pro
       50                  55                  60
   Tyr Thr Lys Asp Val Phe His Lys Leu Leu His Glu Leu Val Glu Lys
   65                  70                  75                  80
   Asn Asn Leu Asn Thr Gly His Ile Tyr Phe Gln Val Thr Arg Gly Thr
                   85                  90                  95
   Ser Ser Arg Ala His Val Phe Pro Glu Ala Thr Val Pro Ala Val Ile
               100                 105                 110
   Thr Gly Asn Val Lys Ser Gly Glu Arg Ala Leu Glu Asn Leu Glu Lys
           115                 120                 125
   Gly Val Lys Ala Thr Phe Val Glu Asp Ile Arg Trp Leu Arg Cys Asp
       130                 135                 140
   Ile Lys Ser Leu Asn Leu Leu Gly Ala Val Leu Ala Lys Gln Glu Ala
   145                 150                 155                 160
   Ser Glu Lys Gly Cys Tyr Glu Ala Ile Leu His Arg Gly Asp Ile Val
                   165                 170                 175
   Thr Glu Cys Ser Ser Ser Asn Val Phe Gly Ile Lys Asp Gly Lys Leu
               180                 185                 190
   Tyr Thr His Pro Ala Asn Asn Leu Ile Leu Asn Gly Ile Thr Arg Gln
           195                 200                 205
   Val Val Ile Lys Cys Ala Glu Glu Ile Asn Ile Pro Val Val Glu Glu
       210                 215                 220
   Pro Phe Thr Lys Gly Glu Ile Leu Thr Met Asp Glu Leu Phe Val Thr
   225                 230                 235                 240
   Ser Val Thr Ser Glu Ile Thr Pro Val Ile Glu Ile Asp Gly Asn Gln
                   245                 250                 255
   Ile Gly Ala Gly Val Pro Gly Glu Trp Thr Arg Lys Leu Gln Lys Ala
   260 265 270
   Phe Glu Ala Lys Ile Pro Leu Ser Leu Asn Ser
   275 280
<210> 100
   <211> 852
   <212> DNA
   <213> Artificial sequence
<220>
   <223> Hybrid DAT
<400> 100
   atgggataca ctttatggaa tgaccaaatc gtggaagatg gtagcgtcag tattagcccg     60
   gaagatcgcg gttatcaatt cggtgatggc gtatatgaag ttgtgaaagt atataacggt    120
   aacatgttta ctgtaaatga acatattgac cgcttatatg catcagctga aaaaatccgt    180
   attgttattc catatacaaa ggatgtgttt cataagttgc tgcatgaatt agtggaaaaa    240
   aataacttaa acactgggca tatttatttt caagttactc gcggaacttc gagtcgtgcg    300
   catgttttcc ctgaggccac tgtaccagcg gtaatcaccg gtaacgtgaa aagcggcgag    360
   cgtgcgttag aaaatcttga aaaaggtgta aaagctacct ttgtggaaga tatccgttgg    420
   ttacgctgtg atattaaatc tttgaacttg cttggtgcag tattagcaaa acaagaagct    480
   agcgaaaaag gctgctatga agcgattctg catcgaggcg acatcgtaac agaatgctct    540
   tcttcaaatg tatttggaat caaagatggt aagttgtata cccatcctgc aaacaatctg    600
   attttaaatg gaatcactcg ccaggttgtc attaaatgtg cagaggaaat taatatccct    660
   gtagtggaag agccatttac aaaaggcgaa atcttgacga tggatgaatt atttgtaaca    720
   agtgttactt ctgaaattac gccggttatt gaaatcgatg gtaatcagat tggtgcagga    780
   gtgccaggag aatggactcg taaattacaa aaagcttttg aagccaaaat tccactgagc    840
   cttaacagct aa                                                        852
<210> 101
   <211> 37
   <212> DNA
   <213> Artificial sequence
<220>
   <223> NdeI Primer
<400> 101
   ggccttggca tatgggatac actttatgga atgacca 37
<210> 102
   <211> 37
   <212> DNA
   <213> Artificial sequence
<220>
   <223> BamHI Primer
<400> 102
   ttggaaccgg atccttagct gttaaggctc agtggaa 37